(19) Europäisches Patentamt
European Patent Office
Office européen des brevets

(11) EP 2 636 749 B1

(12) **EUROPEAN PATENT SPECIFICATION**

(45) Date of publication and mention of the grant of the patent:
**03.08.2016 Bulletin 2016/31**

(21) Application number: **11837765.4**

(22) Date of filing: **07.11.2011**

(51) Int Cl.:
***C12P 19/26*** (2006.01) ***A61K 47/36*** (2006.01)
***C08B 37/00*** (2006.01)

(86) International application number:
**PCT/JP2011/006224**

(87) International publication number:
**WO 2012/060111 (10.05.2012 Gazette 2012/19)**

(54) **NON-REDUCING END-MODIFIED GLUCAN, METHOD FOR PRODUCING SAME, AND USE THEREOF**

NICHTREDUZIERENDES ENDMODIZIFIERTES GLUCAN, VERFAHREN ZU SEINER HERSTELLUNG UND VERWENDUNG DAVON

GLUCANE À EXTRÉMITÉ MODIFIÉE NON RÉDUCTRICE, SON PROCÉDÉ DE PRODUCTION ET SES APPLICATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.11.2010 JP 2010249087**

(43) Date of publication of application:
**11.09.2013 Bulletin 2013/37**

(73) Proprietor: **Ezaki Glico Co., Ltd.**
**Osaka-shi**
**Osaka 555-8502 (JP)**

(72) Inventors:
- **TAKAHA, Takeshi**
  **Osaka-shi**
  **Osaka 555-8502 (JP)**
- **YANASE, Michiyo**
  **Osaka-shi**
  **Osaka 555-8502 (JP)**
- **KUBO, Akiko**
  **Osaka-shi**
  **Osaka 555-8502 (JP)**
- **SAMBE, Haruyo**
  **Osaka-shi**
  **Osaka 555-8502 (JP)**
- **KAKUTANI, Ryo**
  **Osaka-shi**
  **Osaka 555-8502 (JP)**

(74) Representative: **Uexküll & Stolberg**
**Partnerschaft von**
**Patent- und Rechtsanwälten mbB**
**Beselerstraße 4**
**22607 Hamburg (DE)**

(56) References cited:
**EP-A1- 2 428 578 EP-A1- 2 636 748**

- **SHAKHAWAT HOSSAIN BHUIYAN ET AL: "Characterization of a hyperthermostable glycogen phosphorylase from Aquifex aeolicus expressed in Escherichia coli", JOURNAL OF MOLECULAR CATALYSIS B: ENZYMATIC, vol. 22, no. 3-4, 1 June 2003 (2003-06-01) , pages 173-180, XP055103587, ISSN: 1381-1177, DOI: 10.1016/S1381-1177(03)00029-8**
- **MICHIYO YANASE ET AL: "[alpha]-Glucan phosphorylase and its use in carbohydrate engineering", JOURNAL OF THE SCIENCE OF FOOD AND AGRICULTURE, vol. 86, no. 11, 1 January 2006 (2006-01-01), pages 1631-1635, XP055103494, ISSN: 0022-5142, DOI: 10.1002/jsfa.2513**
- **YUTA UMEGAYA ET AL.: 'Phosphorylase Shokubai ni yoru Mattan ni Glucuronic Acid Zanki o Yusuru Bunkigata Tato no Gosei' POLYMER PREPRINTS vol. 59, no. L, 11 May 2010, page 1814**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YUTA UMEGAYA ET AL.: 'Phosphorylase ni yoru Bunkijo Tato eno Glucuronic Acid Zanki no Ten'i Hanno' THE CELLULOSE SOCIETY OF JAPAN NENJI TAIKAI KOEN YOSHISHU vol. 17TH, 01 July 2010, page 44
- JUN'ICHI KADOKAWA ET AL.: 'Kosoteki Glucuronic Acid Zanki no Ten'i Hanno o Riyo shita Anion- sei Tabunki Tato no Gosei' POLYMER PREPRINTS vol. 59, no. 2, 01 September 2010, page 4875
- NAWAJI, M ET AL.: 'Enzymatic alpha-glucosaminylation of maltooligosaccharides catalyzed by phosphorylase' CARBOHYDR.RES. vol. 343, no. 15, 2008, pages 2692 - 2696, XP025408866
- KAWAZOE S. ET AL.: 'Phosphorylase-catalyzed N-formyl-alpha-glucosaminylation of maltooligosaccharides' CARBOHYDR.RES. vol. 345, no. 5, 30 March 2010, pages 631 - 636, XP026944074
- SATOSHI KAWAZOE ET AL.: 'Tainetsusei Phosphorylase ni yoru N-formyl Glucosamine Zanki no Maltooligo eno Ten'i Hanno' CSJ: THE CHEMICAL SOCIETY OF JAPAN KOEN YOKOSHU vol. 90TH, no. 4, 12 March 2010, page 1032, XP002720411

SEQUENCE LISTING

<110> EZAKI GLICO CO., LTD.

<120> NON-REDUCING END MODIFIED GLUCAN, METHOD FOR PRODUCING SAME, AND USE THEREOF

<130> EG038PCT

<150> JP 2010-249087
<151> 2010-11-05

<160> 6

<170> PatentIn version 3.4

<210> 1
<211> 2079
<212> DNA
<213> Aquifex aeolicus

<220>
<221> CDS
<222> (1) .. (2079)

<400> 1

```
atg gaa gaa gaa aaa gta aaa gag gga ttg tgg gag tta gct tac aac        48
Met Glu Glu Glu Lys Val Lys Glu Gly Leu Trp Glu Leu Ala Tyr Asn
1               5                   10                  15

ctg tgg tgg acg tgg aat ccg ccg gct aag gaa tta ttc aga agc att        96
Leu Trp Trp Thr Trp Asn Pro Pro Ala Lys Glu Leu Phe Arg Ser Ile
            20                  25                  30

gac ccg ctt ttg tgg aag gaa act aag gaa aac ccc att gag tta ttg       144
Asp Pro Leu Leu Trp Lys Glu Thr Lys Glu Asn Pro Ile Glu Leu Leu
        35                  40                  45

agg aaa acc aaa ctc ctt gaa aac aag ctc aaa gac gaa gat ttt ata       192
Arg Lys Thr Lys Leu Leu Glu Asn Lys Leu Lys Asp Glu Asp Phe Ile
    50                  55                  60

tct cac ttc aag tac gtt tat tcc ctt tac aaa acc tac atg aac agg       240
Ser His Phe Lys Tyr Val Tyr Ser Leu Tyr Lys Thr Tyr Met Asn Arg
65                  70                  75                  80

cat tcg aaa tac gag gat acg tat aag aag cct ata gtt ttc ctg tct       288
His Ser Lys Tyr Glu Asp Thr Tyr Lys Lys Pro Ile Val Phe Leu Ser
                85                  90                  95

ccc gag tac gga ctt cac cac aca cta ctt ata tac gcg ggg gga ctg       336
Pro Glu Tyr Gly Leu His His Thr Leu Leu Ile Tyr Ala Gly Gly Leu
            100                 105                 110

ggc ttt tta gca gga gat ata ctc aag gag agc agt gac ttg gga ttt       384
Gly Phe Leu Ala Gly Asp Ile Leu Lys Glu Ser Ser Asp Leu Gly Phe
        115                 120                 125

ccg ctt ata ggt gtc ggg ttt atg tac cct cag ggc tac gta aag cag       432
Pro Leu Ile Gly Val Gly Phe Met Tyr Pro Gln Gly Tyr Val Lys Gln
    130                 135                 140
```

```
agg ata agg gtt gac gga tgg cag gaa gac ctt gac gca caa aat caa      480
Arg Ile Arg Val Asp Gly Trp Gln Glu Asp Leu Asp Ala Gln Asn Gln
145                 150                 155                 160

aag gaa tta atg ccc gtt aaa aaa gtt ctg gac aaa gaa gga aaa tgg      528
Lys Glu Leu Met Pro Val Lys Lys Val Leu Asp Lys Glu Gly Lys Trp
                165                 170                 175

ctc aag tgc tac gtt tac gta agg gat gaa aag gtt tac ttt gga gtc      576
Leu Lys Cys Tyr Val Tyr Val Arg Asp Glu Lys Val Tyr Phe Gly Val
            180                 185                 190

tgg gaa gtt aac gtg gga aag aca aag ctc tac ctt ctt gac acg aac      624
Trp Glu Val Asn Val Gly Lys Thr Lys Leu Tyr Leu Leu Asp Thr Asn
        195                 200                 205

gta gag gaa aat act ccc tgg aac agg gaa ata tcc tca aga ctc tac      672
Val Glu Glu Asn Thr Pro Trp Asn Arg Glu Ile Ser Ser Arg Leu Tyr
    210                 215                 220

gtt ccg gac aaa gac ctg agg tta aga caa cag ata gtt ctt ggt ttt      720
Val Pro Asp Lys Asp Leu Arg Leu Arg Gln Gln Ile Val Leu Gly Phe
225                 230                 235                 240

ggc acc gta ata ctc ctt gaa aag ctg ggc att gat gca gga ggt ttt      768
Gly Thr Val Ile Leu Leu Glu Lys Leu Gly Ile Asp Ala Gly Gly Phe
                245                 250                 255

cac ata aac gaa gat tat ccc tcg ttc gtg ttc ctt gca gaa ata ttt      816
His Ile Asn Glu Asp Tyr Pro Ser Phe Val Phe Leu Ala Glu Ile Phe
            260                 265                 270

aaa ctt cta aaa aaa ggt ctg acc tgg gat aag gcg ata gaa gaa gta      864
Lys Leu Leu Lys Lys Gly Leu Thr Trp Asp Lys Ala Ile Glu Glu Val
        275                 280                 285

aga aag att tct ctc ttt acc acg cac aca cca cta cgg gtt gcc gta      912
Arg Lys Ile Ser Leu Phe Thr Thr His Thr Pro Leu Arg Val Ala Val
    290                 295                 300

aat act tat ccc ttc cac atg ata gag gaa cag ttt cta ttc gtt aag      960
Asn Thr Tyr Pro Phe His Met Ile Glu Glu Gln Phe Leu Phe Val Lys
305                 310                 315                 320

gat gtt tac gga ata gac gta aag aaa gtt ctg gaa ctc gga acg aat     1008
Asp Val Tyr Gly Ile Asp Val Lys Lys Val Leu Glu Leu Gly Thr Asn
                325                 330                 335

cct gaa gac cct tcg gag ggt ttt aac agt acg att atg tcc ctc aga     1056
Pro Glu Asp Pro Ser Glu Gly Phe Asn Ser Thr Ile Met Ser Leu Arg
            340                 345                 350

ctc gca aag tac gta aac gca gtg agt aaa aga cat caa gaa gtt tca     1104
Leu Ala Lys Tyr Val Asn Ala Val Ser Lys Arg His Gln Glu Val Ser
        355                 360                 365

agc aag atg tgg agt ttt tta ttt aaa gaa aag gag aat cca ata gat     1152
Ser Lys Met Trp Ser Phe Leu Phe Lys Glu Lys Glu Asn Pro Ile Asp
    370                 375                 380

tac gta acg aac ggt gtt cac ttt ccc aca tgg att tgt tca gat ttg     1200
Tyr Val Thr Asn Gly Val His Phe Pro Thr Trp Ile Cys Ser Asp Leu
385                 390                 395                 400
```

```
aga aga ctg tac gag gag tat ttg gga gag aac ttt gtg gaa ctt cac      1248
Arg Arg Leu Tyr Glu Glu Tyr Leu Gly Glu Asn Phe Val Glu Leu His
                405                 410                 415

gac cac aag tct ctg tgg gaa tta ata aga gac ata ccc gac gaa gaa      1296
Asp His Lys Ser Leu Trp Glu Leu Ile Arg Asp Ile Pro Asp Glu Glu
            420                 425                 430

ctg tgg gaa tat cac ata aga aat aaa gaa aga ctt att gag cac ata      1344
Leu Trp Glu Tyr His Ile Arg Asn Lys Glu Arg Leu Ile Glu His Ile
        435                 440                 445

aaa gac agg gca agg gaa agg tgg gtc aag gaa aaa gcg gat cct tca      1392
Lys Asp Arg Ala Arg Glu Arg Trp Val Lys Glu Lys Ala Asp Pro Ser
    450                 455                 460

atc ctt atg gcc gaa ggt ctg ttc ctt gat tct gac gtt ctt acg gtc      1440
Ile Leu Met Ala Glu Gly Leu Phe Leu Asp Ser Asp Val Leu Thr Val
465                 470                 475                 480

ggt ttt gcg agg agg atg acc ggt tac aaa aga ccg gat ctt ata ttc      1488
Gly Phe Ala Arg Arg Met Thr Gly Tyr Lys Arg Pro Asp Leu Ile Phe
                485                 490                 495

acg gat gta gaa cgc tta aaa aag ata gtg aat gat tcg gaa aga cct      1536
Thr Asp Val Glu Arg Leu Lys Lys Ile Val Asn Asp Ser Glu Arg Pro
            500                 505                 510

gtt cag ata ata ttc gcg gga aag gct cat ccg gct gat atc gaa ggg      1584
Val Gln Ile Ile Phe Ala Gly Lys Ala His Pro Ala Asp Ile Glu Gly
        515                 520                 525

aaa aag ata atc cag aga ata ttt aac ttt gcg aaa gat ccg gaa ttt      1632
Lys Lys Ile Ile Gln Arg Ile Phe Asn Phe Ala Lys Asp Pro Glu Phe
    530                 535                 540

ggg gga aga ata gct ttc gtt gaa gat tac gac gaa ctc ctt gcc cat      1680
Gly Gly Arg Ile Ala Phe Val Glu Asp Tyr Asp Glu Leu Leu Ala His
545                 550                 555                 560

tac atg gtg agg ggt gtg gac gta tgg ttg aac aac cct ctt cct ccc      1728
Tyr Met Val Arg Gly Val Asp Val Trp Leu Asn Asn Pro Leu Pro Pro
                565                 570                 575

ctt gaa gcc tgc ggg aca agc ggt atg aaa gct tct atg aac gga gtg      1776
Leu Glu Ala Cys Gly Thr Ser Gly Met Lys Ala Ser Met Asn Gly Val
            580                 585                 590

ctt cac ctt tca ata ctt gac ggt tgg tgg att gag ggt tat aac gga      1824
Leu His Leu Ser Ile Leu Asp Gly Trp Trp Ile Glu Gly Tyr Asn Gly
        595                 600                 605

aag aac ggt tgg gct ttc gga gat tac gaa gtt gaa gga gac agg aac      1872
Lys Asn Gly Trp Ala Phe Gly Asp Tyr Glu Val Glu Gly Asp Arg Asn
    610                 615                 620

aga gcg gat gcg gag gcc att tac aac atc ctt gag aat gaa gta atc      1920
Arg Ala Asp Ala Glu Ala Ile Tyr Asn Ile Leu Glu Asn Glu Val Ile
625                 630                 635                 640

ccc ctt tat tac gaa agg gac gag agg gga gtg cca gtt aag tgg ata      1968
Pro Leu Tyr Tyr Glu Arg Asp Glu Arg Gly Val Pro Val Lys Trp Ile
```

```
                645                 650                 655

agt atg atg aag gaa gct ata aaa agc att acc cct aac ttt tgc tcc      2016
Ser Met Met Lys Glu Ala Ile Lys Ser Ile Thr Pro Asn Phe Cys Ser
            660                 665                 670

aga agg atg tta aaa gat tac ata aat aag ttc tat tca aaa att tta      2064
Arg Arg Met Leu Lys Asp Tyr Ile Asn Lys Phe Tyr Ser Lys Ile Leu
        675                 680                 685

aag gag gag gga tga                                                  2079
Lys Glu Glu Gly
    690
```

<210> 2
<211> 692
<212> PRT
<213> Aquifex aeolicus

<400> 2

```
Met Glu Glu Glu Lys Val Lys Glu Gly Leu Trp Glu Leu Ala Tyr Asn
1               5                   10                  15

Leu Trp Trp Thr Trp Asn Pro Pro Ala Lys Glu Leu Phe Arg Ser Ile
            20                  25                  30

Asp Pro Leu Leu Trp Lys Glu Thr Lys Glu Asn Pro Ile Glu Leu Leu
        35                  40                  45

Arg Lys Thr Lys Leu Leu Glu Asn Lys Leu Lys Asp Glu Asp Phe Ile
    50                  55                  60

Ser His Phe Lys Tyr Val Tyr Ser Leu Tyr Lys Thr Tyr Met Asn Arg
65                  70                  75                  80

His Ser Lys Tyr Glu Asp Thr Tyr Lys Lys Pro Ile Val Phe Leu Ser
                85                  90                  95

Pro Glu Tyr Gly Leu His His Thr Leu Leu Ile Tyr Ala Gly Gly Leu
            100                 105                 110

Gly Phe Leu Ala Gly Asp Ile Leu Lys Glu Ser Ser Asp Leu Gly Phe
        115                 120                 125

Pro Leu Ile Gly Val Gly Phe Met Tyr Pro Gln Gly Tyr Val Lys Gln
    130                 135                 140

Arg Ile Arg Val Asp Gly Trp Gln Glu Asp Leu Asp Ala Gln Asn Gln
145                 150                 155                 160

Lys Glu Leu Met Pro Val Lys Lys Val Leu Asp Lys Glu Gly Lys Trp
```

```
                165                 170                 175

Leu Lys Cys Tyr Val Tyr Val Arg Asp Glu Lys Val Tyr Phe Gly Val
            180                 185                 190

Trp Glu Val Asn Val Gly Lys Thr Lys Leu Tyr Leu Leu Asp Thr Asn
        195                 200                 205

Val Glu Glu Asn Thr Pro Trp Asn Arg Glu Ile Ser Ser Arg Leu Tyr
    210                 215                 220

Val Pro Asp Lys Asp Leu Arg Leu Arg Gln Gln Ile Val Leu Gly Phe
225                 230                 235                 240

Gly Thr Val Ile Leu Leu Glu Lys Leu Gly Ile Asp Ala Gly Gly Phe
                245                 250                 255

His Ile Asn Glu Asp Tyr Pro Ser Phe Val Phe Leu Ala Glu Ile Phe
            260                 265                 270

Lys Leu Leu Lys Lys Gly Leu Thr Trp Asp Lys Ala Ile Glu Glu Val
        275                 280                 285

Arg Lys Ile Ser Leu Phe Thr Thr His Thr Pro Leu Arg Val Ala Val
    290                 295                 300

Asn Thr Tyr Pro Phe His Met Ile Glu Glu Gln Phe Leu Phe Val Lys
305                 310                 315                 320

Asp Val Tyr Gly Ile Asp Val Lys Lys Val Leu Glu Leu Gly Thr Asn
                325                 330                 335

Pro Glu Asp Pro Ser Glu Gly Phe Asn Ser Thr Ile Met Ser Leu Arg
            340                 345                 350

Leu Ala Lys Tyr Val Asn Ala Val Ser Lys Arg His Gln Glu Val Ser
        355                 360                 365

Ser Lys Met Trp Ser Phe Leu Phe Lys Glu Lys Glu Asn Pro Ile Asp
    370                 375                 380

Tyr Val Thr Asn Gly Val His Phe Pro Thr Trp Ile Cys Ser Asp Leu
385                 390                 395                 400

Arg Arg Leu Tyr Glu Glu Tyr Leu Gly Glu Asn Phe Val Glu Leu His
                405                 410                 415
```

```
Asp His Lys Ser Leu Trp Glu Leu Ile Arg Asp Ile Pro Asp Glu Glu
            420                 425                 430

Leu Trp Glu Tyr His Ile Arg Asn Lys Glu Arg Leu Ile Glu His Ile
        435                 440                 445

Lys Asp Arg Ala Arg Glu Arg Trp Val Lys Glu Lys Ala Asp Pro Ser
    450                 455                 460

Ile Leu Met Ala Glu Gly Leu Phe Leu Asp Ser Asp Val Leu Thr Val
465                 470                 475                 480

Gly Phe Ala Arg Arg Met Thr Gly Tyr Lys Arg Pro Asp Leu Ile Phe
                485                 490                 495

Thr Asp Val Glu Arg Leu Lys Lys Ile Val Asn Asp Ser Glu Arg Pro
            500                 505                 510

Val Gln Ile Ile Phe Ala Gly Lys Ala His Pro Ala Asp Ile Glu Gly
        515                 520                 525

Lys Lys Ile Ile Gln Arg Ile Phe Asn Phe Ala Lys Asp Pro Glu Phe
    530                 535                 540

Gly Gly Arg Ile Ala Phe Val Glu Asp Tyr Asp Glu Leu Leu Ala His
545                 550                 555                 560

Tyr Met Val Arg Gly Val Asp Val Trp Leu Asn Asn Pro Leu Pro Pro
                565                 570                 575

Leu Glu Ala Cys Gly Thr Ser Gly Met Lys Ala Ser Met Asn Gly Val
            580                 585                 590

Leu His Leu Ser Ile Leu Asp Gly Trp Trp Ile Glu Gly Tyr Asn Gly
        595                 600                 605

Lys Asn Gly Trp Ala Phe Gly Asp Tyr Glu Val Glu Gly Asp Arg Asn
    610                 615                 620

Arg Ala Asp Ala Glu Ala Ile Tyr Asn Ile Leu Glu Asn Glu Val Ile
625                 630                 635                 640

Pro Leu Tyr Tyr Glu Arg Asp Glu Arg Gly Val Pro Val Lys Trp Ile
                645                 650                 655

Ser Met Met Lys Glu Ala Ile Lys Ser Ile Thr Pro Asn Phe Cys Ser
            660                 665                 670
```

```
Arg Arg Met Leu Lys Asp Tyr Ile Asn Lys Phe Tyr Ser Lys Ile Leu
        675                 680                 685

Lys Glu Glu Gly
    690
```

<210> 3
<211> 2793
<212> DNA
<213> Solanum tuberosum

<220>
<221> CDS
<222> (1)..(2790)

<220>
<221> sig_peptide
<222> (1)..(42)

<220>
<221> mat_peptide
<222> (43)..(2790)

<400> 3

```
atg gct agc atg act ggt gga cag caa atg ggt cgg atc ctc acc ttg      48
Met Ala Ser Met Thr Gly Gly Gln Gln Met Gly Arg Ile Leu Thr Leu
                -10                 -5                  -1   1

agt gag aaa att cac cat ccc att act gaa caa ggt ggt gag agc gac      96
Ser Glu Lys Ile His His Pro Ile Thr Glu Gln Gly Gly Glu Ser Asp
        5                   10                  15

ctg agt tct ttt gct cct gat gcc gca tct att acc tca agt atc aaa     144
Leu Ser Ser Phe Ala Pro Asp Ala Ala Ser Ile Thr Ser Ser Ile Lys
    20                  25                  30

tac cat gca gaa ttc aca cct gta ttc tct cct gaa agg ttt gag ctc     192
Tyr His Ala Glu Phe Thr Pro Val Phe Ser Pro Glu Arg Phe Glu Leu
35                  40                  45                  50

cct aag gca ttc ttt gca aca gct caa agt gtt cgt gat tcg ctc ctt     240
Pro Lys Ala Phe Phe Ala Thr Ala Gln Ser Val Arg Asp Ser Leu Leu
                55                  60                  65

att aat tgg aat gct acg tat gat att tat gaa aag ctg aac atg aag     288
Ile Asn Trp Asn Ala Thr Tyr Asp Ile Tyr Glu Lys Leu Asn Met Lys
            70                  75                  80

caa gcg tac tat cta tcc atg gaa ttt ctg cag ggt aga gca ttg tta     336
Gln Ala Tyr Tyr Leu Ser Met Glu Phe Leu Gln Gly Arg Ala Leu Leu
        85                  90                  95

aat gca att ggt aat ctg gag ctt act ggt gca ttt gcg gaa gct ttg     384
Asn Ala Ile Gly Asn Leu Glu Leu Thr Gly Ala Phe Ala Glu Ala Leu
    100                 105                 110

aaa aac ctt ggc cac aat cta gaa aat gtg gct tct cag gaa cca gat     432
Lys Asn Leu Gly His Asn Leu Glu Asn Val Ala Ser Gln Glu Pro Asp
115                 120                 125                 130
```

```
gct gct ctt gga aat ggg ggt ttg gga cgg ctt gct tcc tgt ttt ctg      480
Ala Ala Leu Gly Asn Gly Gly Leu Gly Arg Leu Ala Ser Cys Phe Leu
                135                 140                 145

gac tct ttg gca aca cta aac tac cca gca tgg ggc tat gga ctt agg      528
Asp Ser Leu Ala Thr Leu Asn Tyr Pro Ala Trp Gly Tyr Gly Leu Arg
            150                 155                 160

tac aag tat ggt tta ttt aag caa cgg att aca aaa gat ggt cag gag      576
Tyr Lys Tyr Gly Leu Phe Lys Gln Arg Ile Thr Lys Asp Gly Gln Glu
        165                 170                 175

gag gtg gct gaa gat tgg ctt gaa att ggc agt cca tgg gaa gtt gtg      624
Glu Val Ala Glu Asp Trp Leu Glu Ile Gly Ser Pro Trp Glu Val Val
    180                 185                 190

agg aat gat gtt tca tat cct atc aaa ttc tat gga aaa gtc tct aca      672
Arg Asn Asp Val Ser Tyr Pro Ile Lys Phe Tyr Gly Lys Val Ser Thr
195                 200                 205                 210

gga tca gat gga aag agg tat tgg att ggt gga gag gat ata aag gca      720
Gly Ser Asp Gly Lys Arg Tyr Trp Ile Gly Gly Glu Asp Ile Lys Ala
                215                 220                 225

gtt gcg tat gat gtt ccc ata cca ggg tat aag acc aga acc aca atc      768
Val Ala Tyr Asp Val Pro Ile Pro Gly Tyr Lys Thr Arg Thr Thr Ile
            230                 235                 240

agc ctt cga ctg tgg tct aca cag gtt cca tca gcg gat ttt gat tta      816
Ser Leu Arg Leu Trp Ser Thr Gln Val Pro Ser Ala Asp Phe Asp Leu
        245                 250                 255

tct gct ttc aat gct gga gag cac acc aaa gca tgt gaa gcc caa gca      864
Ser Ala Phe Asn Ala Gly Glu His Thr Lys Ala Cys Glu Ala Gln Ala
    260                 265                 270

aac gct gag aag ata tgt tac ata ctc tac cct ggg gat gaa tca gag      912
Asn Ala Glu Lys Ile Cys Tyr Ile Leu Tyr Pro Gly Asp Glu Ser Glu
275                 280                 285                 290

gag gga aag atc ctt cgg ttg aag caa caa tat acc tta tgc tcg gct      960
Glu Gly Lys Ile Leu Arg Leu Lys Gln Gln Tyr Thr Leu Cys Ser Ala
                295                 300                 305

tct ctc caa gat att att tct cga ttt gag agg aga tca ggt gat cgt     1008
Ser Leu Gln Asp Ile Ile Ser Arg Phe Glu Arg Arg Ser Gly Asp Arg
            310                 315                 320

att aag tgg gaa gag ttt cct gaa aaa gtt gct gtg cag atg aat gac     1056
Ile Lys Trp Glu Glu Phe Pro Glu Lys Val Ala Val Gln Met Asn Asp
        325                 330                 335

act cac cct aca ctt tgt atc cct gag ctg atg aga ata ttg ata gat     1104
Thr His Pro Thr Leu Cys Ile Pro Glu Leu Met Arg Ile Leu Ile Asp
    340                 345                 350

ctg aag ggc ttg aat tgg aat gaa gct tgg aat att act caa aga act     1152
Leu Lys Gly Leu Asn Trp Asn Glu Ala Trp Asn Ile Thr Gln Arg Thr
355                 360                 365                 370

gtg gcc tac aca aac cat act gtt ttg cct gag gca ctg gag aaa tgg     1200
Val Ala Tyr Thr Asn His Thr Val Leu Pro Glu Ala Leu Glu Lys Trp
```

```
                375                 380                 385

agt tat gaa ttg atg cag aaa ctc ctt ccc aga cat gtc gaa atc att      1248
Ser Tyr Glu Leu Met Gln Lys Leu Leu Pro Arg His Val Glu Ile Ile
            390                 395                 400

gag gcg att gac gag gag ctg gta cat gaa att gta tta aaa tat ggt      1296
Glu Ala Ile Asp Glu Glu Leu Val His Glu Ile Val Leu Lys Tyr Gly
        405                 410                 415

tca atg gat ctg aac aaa ttg gag gaa aag ttg act aca atg aga atc      1344
Ser Met Asp Leu Asn Lys Leu Glu Glu Lys Leu Thr Thr Met Arg Ile
    420                 425                 430

tta gaa aat ttt gat ctt ccc agt tct gtt gct gaa tta ttt att aag      1392
Leu Glu Asn Phe Asp Leu Pro Ser Ser Val Ala Glu Leu Phe Ile Lys
435                 440                 445                 450

cct gaa atc tca gtt gat gat gat act gaa aca gta gaa gtc cat gac      1440
Pro Glu Ile Ser Val Asp Asp Asp Thr Glu Thr Val Glu Val His Asp
                455                 460                 465

aaa gtt gaa gct tcc gat aaa gtt gtg act aat gat gaa gat gac act      1488
Lys Val Glu Ala Ser Asp Lys Val Val Thr Asn Asp Glu Asp Asp Thr
            470                 475                 480

ggt aag aaa act agt gtg aag ata gaa gca gct gca gaa aaa gac att      1536
Gly Lys Lys Thr Ser Val Lys Ile Glu Ala Ala Ala Glu Lys Asp Ile
        485                 490                 495

gac aag aaa act ccc gtg agt ccg gaa cca gct gtt ata cca cct aag      1584
Asp Lys Lys Thr Pro Val Ser Pro Glu Pro Ala Val Ile Pro Pro Lys
    500                 505                 510

aag gta cgc atg gcc aac ttg tgt gtt gtg ggc ggc cat gct gtt aat      1632
Lys Val Arg Met Ala Asn Leu Cys Val Val Gly Gly His Ala Val Asn
515                 520                 525                 530

gga gtt gct gag atc cat agt gaa att gtg aag gag gag gtt ttc aat      1680
Gly Val Ala Glu Ile His Ser Glu Ile Val Lys Glu Glu Val Phe Asn
                535                 540                 545

gac ttc tat gag ctc tgg ccg gaa aag ttc caa aac aaa aca aat gga      1728
Asp Phe Tyr Glu Leu Trp Pro Glu Lys Phe Gln Asn Lys Thr Asn Gly
            550                 555                 560

gtg act cca aga aga tgg att cgt ttc tgc aat cct cct ctt agt gcc      1776
Val Thr Pro Arg Arg Trp Ile Arg Phe Cys Asn Pro Pro Leu Ser Ala
        565                 570                 575

atc ata act aag tgg act ggt aca gag gat tgg gtc ctg aaa act gaa      1824
Ile Ile Thr Lys Trp Thr Gly Thr Glu Asp Trp Val Leu Lys Thr Glu
    580                 585                 590

aag ttg gca gaa ttg cag aag ttt gct gat aat gaa gat ctt caa aat      1872
Lys Leu Ala Glu Leu Gln Lys Phe Ala Asp Asn Glu Asp Leu Gln Asn
595                 600                 605                 610

gag tgg agg gaa gca aaa agg agc aac aag att aaa gtt gtc tcc ttt      1920
Glu Trp Arg Glu Ala Lys Arg Ser Asn Lys Ile Lys Val Val Ser Phe
                615                 620                 625

ctc aaa gaa aag aca ggg tat tct gtt gtc cca gat gca atg ttt gat      1968
```

```
Leu Lys Glu Lys Thr Gly Tyr Ser Val Val Pro Asp Ala Met Phe Asp
            630                 635                 640

att cag gta aaa cgc att cat gag tac aag cga caa ctg tta aat atc      2016
Ile Gln Val Lys Arg Ile His Glu Tyr Lys Arg Gln Leu Leu Asn Ile
        645                 650                 655

ttc ggc atc gtt tat cgg tat aag aag atg aaa gaa atg aca gct gca      2064
Phe Gly Ile Val Tyr Arg Tyr Lys Lys Met Lys Glu Met Thr Ala Ala
    660                 665                 670

gaa aga aag act aac ttc gtt cct cga gta tgc ata ttt ggg gga aaa      2112
Glu Arg Lys Thr Asn Phe Val Pro Arg Val Cys Ile Phe Gly Gly Lys
675                 680                 685                 690

gct ttt gcc aca tat gtg caa gcc aag agg att gta aaa ttt atc aca      2160
Ala Phe Ala Thr Tyr Val Gln Ala Lys Arg Ile Val Lys Phe Ile Thr
                695                 700                 705

gat gtt ggt gct act ata aat cat gat cca gaa atc ggt gat ctg ttg      2208
Asp Val Gly Ala Thr Ile Asn His Asp Pro Glu Ile Gly Asp Leu Leu
            710                 715                 720

aag gta gtc ttt gtg cca gat tac aat gtc agt gtt gct gaa ttg cta      2256
Lys Val Val Phe Val Pro Asp Tyr Asn Val Ser Val Ala Glu Leu Leu
        725                 730                 735

att cct gct agc gat cta tca gaa cat atc agt acg gct gga atg gag      2304
Ile Pro Ala Ser Asp Leu Ser Glu His Ile Ser Thr Ala Gly Met Glu
    740                 745                 750

gcc agt gga acc agt aat atg aag ttt gca atg aat ggt tgt atc caa      2352
Ala Ser Gly Thr Ser Asn Met Lys Phe Ala Met Asn Gly Cys Ile Gln
755                 760                 765                 770

att ggt aca ttg gat ggc gct aat gtt gaa ata agg gaa gag gtt gga      2400
Ile Gly Thr Leu Asp Gly Ala Asn Val Glu Ile Arg Glu Glu Val Gly
                775                 780                 785

gaa gaa aac ttc ttt ctc ttt ggt gct caa gct cat gaa att gca ggg      2448
Glu Glu Asn Phe Phe Leu Phe Gly Ala Gln Ala His Glu Ile Ala Gly
            790                 795                 800

ctt aga aaa gaa aga gct gac gga aag ttt gta cct gat gaa cgt ttt      2496
Leu Arg Lys Glu Arg Ala Asp Gly Lys Phe Val Pro Asp Glu Arg Phe
        805                 810                 815

gaa gag gtg aag gaa ttt gtt aga agc ggt gct ttt ggc tct tat aac      2544
Glu Glu Val Lys Glu Phe Val Arg Ser Gly Ala Phe Gly Ser Tyr Asn
    820                 825                 830

tat gat gac cta att gga tcg ttg gaa gga aat gaa ggt ttt ggc cgt      2592
Tyr Asp Asp Leu Ile Gly Ser Leu Glu Gly Asn Glu Gly Phe Gly Arg
835                 840                 845                 850

gct gac tat ttc ctt gtg ggc aag gac ttc ccc agt tac ata gaa tgc      2640
Ala Asp Tyr Phe Leu Val Gly Lys Asp Phe Pro Ser Tyr Ile Glu Cys
                855                 860                 865

caa gag aaa gtt gat gag gca tat cgc gac cag aaa agg tgg aca acg      2688
Gln Glu Lys Val Asp Glu Ala Tyr Arg Asp Gln Lys Arg Trp Thr Thr
            870                 875                 880
```

```
atg tca atc ttg aat aca gcg gga tcg tac aag ttc agc agt gac aga      2736
Met Ser Ile Leu Asn Thr Ala Gly Ser Tyr Lys Phe Ser Ser Asp Arg
        885                 890                 895

aca atc cat gaa tat gcc aaa gac att tgg aac att gaa gct gtg gaa      2784
Thr Ile His Glu Tyr Ala Lys Asp Ile Trp Asn Ile Glu Ala Val Glu
    900                 905                 910

ata gca taa                                                          2793
Ile Ala
915
```

<210> 4
<211> 930
<212> PRT
<213> Solanum tuberosum

<400> 4

```
Met Ala Ser Met Thr Gly Gly Gln Gln Met Gly Arg Ile Leu Thr Leu
                -10                 -5                  -1  1

Ser Glu Lys Ile His His Pro Ile Thr Glu Gln Gly Gly Glu Ser Asp
        5                   10                  15

Leu Ser Ser Phe Ala Pro Asp Ala Ala Ser Ile Thr Ser Ser Ile Lys
    20                  25                  30

Tyr His Ala Glu Phe Thr Pro Val Phe Ser Pro Glu Arg Phe Glu Leu
35                  40                  45                  50

Pro Lys Ala Phe Phe Ala Thr Ala Gln Ser Val Arg Asp Ser Leu Leu
                55                  60                  65

Ile Asn Trp Asn Ala Thr Tyr Asp Ile Tyr Glu Lys Leu Asn Met Lys
            70                  75                  80

Gln Ala Tyr Tyr Leu Ser Met Glu Phe Leu Gln Gly Arg Ala Leu Leu
        85                  90                  95

Asn Ala Ile Gly Asn Leu Glu Leu Thr Gly Ala Phe Ala Glu Ala Leu
    100                 105                 110

Lys Asn Leu Gly His Asn Leu Glu Asn Val Ala Ser Gln Glu Pro Asp
115                 120                 125                 130

Ala Ala Leu Gly Asn Gly Gly Leu Gly Arg Leu Ala Ser Cys Phe Leu
                135                 140                 145

Asp Ser Leu Ala Thr Leu Asn Tyr Pro Ala Trp Gly Tyr Gly Leu Arg
            150                 155                 160
```

```
Tyr Lys Tyr Gly Leu Phe Lys Gln Arg Ile Thr Lys Asp Gly Gln Glu
        165                 170                 175

Glu Val Ala Glu Asp Trp Leu Glu Ile Gly Ser Pro Trp Glu Val Val
    180                 185                 190

Arg Asn Asp Val Ser Tyr Pro Ile Lys Phe Tyr Gly Lys Val Ser Thr
195                 200                 205                 210

Gly Ser Asp Gly Lys Arg Tyr Trp Ile Gly Gly Glu Asp Ile Lys Ala
                215                 220                 225

Val Ala Tyr Asp Val Pro Ile Pro Gly Tyr Lys Thr Arg Thr Thr Ile
            230                 235                 240

Ser Leu Arg Leu Trp Ser Thr Gln Val Pro Ser Ala Asp Phe Asp Leu
        245                 250                 255

Ser Ala Phe Asn Ala Gly Glu His Thr Lys Ala Cys Glu Ala Gln Ala
    260                 265                 270

Asn Ala Glu Lys Ile Cys Tyr Ile Leu Tyr Pro Gly Asp Glu Ser Glu
275                 280                 285                 290

Glu Gly Lys Ile Leu Arg Leu Lys Gln Gln Tyr Thr Leu Cys Ser Ala
                295                 300                 305

Ser Leu Gln Asp Ile Ile Ser Arg Phe Glu Arg Arg Ser Gly Asp Arg
            310                 315                 320

Ile Lys Trp Glu Glu Phe Pro Glu Lys Val Ala Val Gln Met Asn Asp
        325                 330                 335

Thr His Pro Thr Leu Cys Ile Pro Glu Leu Met Arg Ile Leu Ile Asp
    340                 345                 350

Leu Lys Gly Leu Asn Trp Asn Glu Ala Trp Asn Ile Thr Gln Arg Thr
355                 360                 365                 370

Val Ala Tyr Thr Asn His Thr Val Leu Pro Glu Ala Leu Glu Lys Trp
                375                 380                 385

Ser Tyr Glu Leu Met Gln Lys Leu Leu Pro Arg His Val Glu Ile Ile
            390                 395                 400

Glu Ala Ile Asp Glu Glu Leu Val His Glu Ile Val Leu Lys Tyr Gly
        405                 410                 415
```

```
Ser Met Asp Leu Asn Lys Leu Glu Glu Lys Leu Thr Thr Met Arg Ile
    420                 425                 430

Leu Glu Asn Phe Asp Leu Pro Ser Ser Val Ala Glu Leu Phe Ile Lys
435                 440                 445                 450

Pro Glu Ile Ser Val Asp Asp Asp Thr Glu Thr Val Glu Val His Asp
                455                 460                 465

Lys Val Glu Ala Ser Asp Lys Val Val Thr Asn Asp Glu Asp Asp Thr
            470                 475                 480

Gly Lys Lys Thr Ser Val Lys Ile Glu Ala Ala Ala Glu Lys Asp Ile
        485                 490                 495

Asp Lys Lys Thr Pro Val Ser Pro Glu Pro Ala Val Ile Pro Pro Lys
    500                 505                 510

Lys Val Arg Met Ala Asn Leu Cys Val Val Gly Gly His Ala Val Asn
515                 520                 525                 530

Gly Val Ala Glu Ile His Ser Glu Ile Val Lys Glu Glu Val Phe Asn
                535                 540                 545

Asp Phe Tyr Glu Leu Trp Pro Glu Lys Phe Gln Asn Lys Thr Asn Gly
            550                 555                 560

Val Thr Pro Arg Arg Trp Ile Arg Phe Cys Asn Pro Pro Leu Ser Ala
        565                 570                 575

Ile Ile Thr Lys Trp Thr Gly Thr Glu Asp Trp Val Leu Lys Thr Glu
    580                 585                 590

Lys Leu Ala Glu Leu Gln Lys Phe Ala Asp Asn Glu Asp Leu Gln Asn
595                 600                 605                 610

Glu Trp Arg Glu Ala Lys Arg Ser Asn Lys Ile Lys Val Val Ser Phe
                615                 620                 625

Leu Lys Glu Lys Thr Gly Tyr Ser Val Val Pro Asp Ala Met Phe Asp
            630                 635                 640

Ile Gln Val Lys Arg Ile His Glu Tyr Lys Arg Gln Leu Leu Asn Ile
        645                 650                 655

Phe Gly Ile Val Tyr Arg Tyr Lys Lys Met Lys Glu Met Thr Ala Ala
```

```
    660                 665                 670

Glu Arg Lys Thr Asn Phe Val Pro Arg Val Cys Ile Phe Gly Gly Lys
675                 680                 685                 690

Ala Phe Ala Thr Tyr Val Gln Ala Lys Arg Ile Val Lys Phe Ile Thr
                695                 700                 705

Asp Val Gly Ala Thr Ile Asn His Asp Pro Glu Ile Gly Asp Leu Leu
            710                 715                 720

Lys Val Val Phe Val Pro Asp Tyr Asn Val Ser Val Ala Glu Leu Leu
        725                 730                 735

Ile Pro Ala Ser Asp Leu Ser Glu His Ile Ser Thr Ala Gly Met Glu
    740                 745                 750

Ala Ser Gly Thr Ser Asn Met Lys Phe Ala Met Asn Gly Cys Ile Gln
755                 760                 765                 770

Ile Gly Thr Leu Asp Gly Ala Asn Val Glu Ile Arg Glu Glu Val Gly
                775                 780                 785

Glu Glu Asn Phe Phe Leu Phe Gly Ala Gln Ala His Glu Ile Ala Gly
            790                 795                 800

Leu Arg Lys Glu Arg Ala Asp Gly Lys Phe Val Pro Asp Glu Arg Phe
        805                 810                 815

Glu Glu Val Lys Glu Phe Val Arg Ser Gly Ala Phe Gly Ser Tyr Asn
    820                 825                 830

Tyr Asp Asp Leu Ile Gly Ser Leu Glu Gly Asn Glu Gly Phe Gly Arg
835                 840                 845                 850

Ala Asp Tyr Phe Leu Val Gly Lys Asp Phe Pro Ser Tyr Ile Glu Cys
                855                 860                 865

Gln Glu Lys Val Asp Glu Ala Tyr Arg Asp Gln Lys Arg Trp Thr Thr
            870                 875                 880

Met Ser Ile Leu Asn Thr Ala Gly Ser Tyr Lys Phe Ser Ser Asp Arg
        885                 890                 895

Thr Ile His Glu Tyr Ala Lys Asp Ile Trp Asn Ile Glu Ala Val Glu
    900                 905                 910
```

Ile Ala
915

<210> 5
<211> 2151
<212> DNA
<213> Thermococcus zilligii

<220>
<221> CDS
<222> (1)..(2151)

<400> 5

```
atg gcg gac gtt tcc cac aca gtt gag aat tta ata agg gcg aag ctc      48
Met Ala Asp Val Ser His Thr Val Glu Asn Leu Ile Arg Ala Lys Leu
1               5                   10                  15

ccc tac ccg ctg gag aat ctc gca gaa ctg gcc tat aac tac tgg tgg      96
Pro Tyr Pro Leu Glu Asn Leu Ala Glu Leu Ala Tyr Asn Tyr Trp Trp
            20                  25                  30

agc tgg aac aga cgt gcc acc cgg ctc tgg gag tac ata gac tcc gaa      144
Ser Trp Asn Arg Arg Ala Thr Arg Leu Trp Glu Tyr Ile Asp Ser Glu
        35                  40                  45

cac tgg cgt gag tac aag aac ccg gtc aag ctc ctt ctt gac acg ccc      192
His Trp Arg Glu Tyr Lys Asn Pro Val Lys Leu Leu Leu Asp Thr Pro
    50                  55                  60

gaa gag agg ttc tgg gag ctc ctc aag gat gac gac ttc atg aac ctc      240
Glu Glu Arg Phe Trp Glu Leu Leu Lys Asp Asp Asp Phe Met Asn Leu
65                  70                  75                  80

tac gag ctc gtt atg gac cag ttt acc gca tac atg aac ccg aaa tca      288
Tyr Glu Leu Val Met Asp Gln Phe Thr Ala Tyr Met Asn Pro Lys Ser
                85                  90                  95

acg tgg ttc tcg acc aac tac ccc aaa tgg gac aaa ccg ata gtc tat      336
Thr Trp Phe Ser Thr Asn Tyr Pro Lys Trp Asp Lys Pro Ile Val Tyr
            100                 105                 110

ctc tgc atg gag tac ggc ata agc aag agc ctc ccg att tac tcc ggt      384
Leu Cys Met Glu Tyr Gly Ile Ser Lys Ser Leu Pro Ile Tyr Ser Gly
        115                 120                 125

ggc ctc gga atc ctc gcc ggg gat cac ctc aaa acc gcg agt gac ctc      432
Gly Leu Gly Ile Leu Ala Gly Asp His Leu Lys Thr Ala Ser Asp Leu
    130                 135                 140

ggc ctt ccc ttc ata gct ata ggc ctc ctc tac aag cac ggc tac ttc      480
Gly Leu Pro Phe Ile Ala Ile Gly Leu Leu Tyr Lys His Gly Tyr Phe
145                 150                 155                 160

agg cag gag ata gac agg gac gga agg cag agg gag ata ttc ccg gaa      528
Arg Gln Glu Ile Asp Arg Asp Gly Arg Gln Arg Glu Ile Phe Pro Glu
                165                 170                 175

tac agg ccc gag gaa atg ccg ata aag cct gtt cta agc aaa gac gga      576
Tyr Arg Pro Glu Glu Met Pro Ile Lys Pro Val Leu Ser Lys Asp Gly
            180                 185                 190
```

```
aaa ccc cta ctc gtt gag gtc ccc att gaa aac agg atc gtc tac gcg      624
Lys Pro Leu Leu Val Glu Val Pro Ile Glu Asn Arg Ile Val Tyr Ala
        195                 200                 205

agg gcc ttt gag gtc agc gtc ggc atg gcc aag ctc tac ctc ctt gac      672
Arg Ala Phe Glu Val Ser Val Gly Met Ala Lys Leu Tyr Leu Leu Asp
    210                 215                 220

aca gat gtg ccc gag aac agc ccg gag gat agg gcg atc tgc gac tac      720
Thr Asp Val Pro Glu Asn Ser Pro Glu Asp Arg Ala Ile Cys Asp Tyr
225                 230                 235                 240

ctc tac aac gcc gag atg gac aag aga ata aaa cag gag atc ctc ctc      768
Leu Tyr Asn Ala Glu Met Asp Lys Arg Ile Lys Gln Glu Ile Leu Leu
                245                 250                 255

gga ata ggc gga atg aga ctg ctc cag gcc ctc ggc atc gag ccc ggt      816
Gly Ile Gly Gly Met Arg Leu Leu Gln Ala Leu Gly Ile Glu Pro Gly
            260                 265                 270

gtg gtt cac ctc aac gag ggg cat ccc gcc ttt gcc aac ctc cag aga      864
Val Val His Leu Asn Glu Gly His Pro Ala Phe Ala Asn Leu Gln Arg
        275                 280                 285

ata gcc tgg tac atg gaa aaa ggc ctc aca ttc acg gaa gct ttg agc      912
Ile Ala Trp Tyr Met Glu Lys Gly Leu Thr Phe Thr Glu Ala Leu Ser
    290                 295                 300

atc gtc agg gga acc acg gtt ttc acc acc cac acg ccg gtt cct gca      960
Ile Val Arg Gly Thr Thr Val Phe Thr Thr His Thr Pro Val Pro Ala
305                 310                 315                 320

ggc cac gac agg ttc ccg atc gcg gaa gtt agg aag agg ctt tca aaa     1008
Gly His Asp Arg Phe Pro Ile Ala Glu Val Arg Lys Arg Leu Ser Lys
                325                 330                 335

ttc ctc gag gga agg gag gag cta ctc gaa ctc ggc cgc gag ggc gac     1056
Phe Leu Glu Gly Arg Glu Glu Leu Leu Glu Leu Gly Arg Glu Gly Asp
            340                 345                 350

cag atc aac atg acc ctg ctt gca ata aga act tcg agc tac gtc aac     1104
Gln Ile Asn Met Thr Leu Leu Ala Ile Arg Thr Ser Ser Tyr Val Asn
        355                 360                 365

ggc gta agc cag ctc cac gcg gag gta agc aag cgc atg tgg aaa gac     1152
Gly Val Ser Gln Leu His Ala Glu Val Ser Lys Arg Met Trp Lys Asp
    370                 375                 380

ctc tgg ccc gga gtt ccc ctc aac gag atc cct ata gag ggc atc aca     1200
Leu Trp Pro Gly Val Pro Leu Asn Glu Ile Pro Ile Glu Gly Ile Thr
385                 390                 395                 400

aac ggc gtc cac aca atg acg tgg gtc cac gac gag atg agg aag ctc     1248
Asn Gly Val His Thr Met Thr Trp Val His Asp Glu Met Arg Lys Leu
                405                 410                 415

ttc gac cgc tac atc ggc aag gtc tgg agg gag cac acc aac ata gag     1296
Phe Asp Arg Tyr Ile Gly Lys Val Trp Arg Glu His Thr Asn Ile Glu
            420                 425                 430

ggc atc tgg tac gcc gtg gaa agg atc ccg gat gaa gag ctc tgg gga     1344
Gly Ile Trp Tyr Ala Val Glu Arg Ile Pro Asp Glu Glu Leu Trp Gly
        435                 440                 445
```

gcc cac ctc gag gcc aag aga cag ctc ata gag ttc ctc agg gag aag 1392
Ala His Leu Glu Ala Lys Arg Gln Leu Ile Glu Phe Leu Arg Glu Lys
450 455 460

aca atg gag agg aac agg agg ctc gga acc gat gac ccg ata ccg gag 1440
Thr Met Glu Arg Asn Arg Arg Leu Gly Thr Asp Asp Pro Ile Pro Glu
465 470 475 480

ata gac gag aac gcc ctc ata atc ggc ttt gcc aga cgc ttt gcg acc 1488
Ile Asp Glu Asn Ala Leu Ile Ile Gly Phe Ala Arg Arg Phe Ala Thr
485 490 495

tat aag agg gca act ctc atc ctg agc gac ctt gag agg ctg aag aaa 1536
Tyr Lys Arg Ala Thr Leu Ile Leu Ser Asp Leu Glu Arg Leu Lys Lys
500 505 510

atc ctc aac aac cca gaa agg ccg gtt tac ata gtc ttc ggc ggg aag 1584
Ile Leu Asn Asn Pro Glu Arg Pro Val Tyr Ile Val Phe Gly Gly Lys
515 520 525

gcc cat ccg cgc gac gag gct ggg aag gag ttc ctg agg agg gtt tac 1632
Ala His Pro Arg Asp Glu Ala Gly Lys Glu Phe Leu Arg Arg Val Tyr
530 535 540

gag gtc agc cag atg ccg gag ttc agg ggc aag ata ttc gtc ctc gag 1680
Glu Val Ser Gln Met Pro Glu Phe Arg Gly Lys Ile Phe Val Leu Glu
545 550 555 560

aac tac gac atg ggg agc gcg agg ctc atg gtc gct gga gtc gac gtc 1728
Asn Tyr Asp Met Gly Ser Ala Arg Leu Met Val Ala Gly Val Asp Val
565 570 575

tgg ctc aac aac ccg cgc aga ccg ctt gaa gca agc ggg acg agc ggc 1776
Trp Leu Asn Asn Pro Arg Arg Pro Leu Glu Ala Ser Gly Thr Ser Gly
580 585 590

atg aag gcc ggc ctc aac ggt gtc ctc aac gcg agc atc ttc gac gga 1824
Met Lys Ala Gly Leu Asn Gly Val Leu Asn Ala Ser Ile Phe Asp Gly
595 600 605

tgg tgg gtt gaa ggc tac aat ggg aaa aac ggc tgg gtc att ggg gag 1872
Trp Trp Val Glu Gly Tyr Asn Gly Lys Asn Gly Trp Val Ile Gly Glu
610 615 620

gag acc acc gag ccg gag agc gaa gag gac gac gcg aag gac gct gag 1920
Glu Thr Thr Glu Pro Glu Ser Glu Glu Asp Asp Ala Lys Asp Ala Glu
625 630 635 640

agc ctc tac acc ctg ctc gag aag gag ata atc ccc acc tac tac ggg 1968
Ser Leu Tyr Thr Leu Leu Glu Lys Glu Ile Ile Pro Thr Tyr Tyr Gly
645 650 655

aac agg agc cgc tgg ata tac atg atg aag gag agc ata aag agc ata 2016
Asn Arg Ser Arg Trp Ile Tyr Met Met Lys Glu Ser Ile Lys Ser Ile
660 665 670

gcc ccg cgc ttc agc acg cac agg atg gtc aag gag tac atg gac agg 2064
Ala Pro Arg Phe Ser Thr His Arg Met Val Lys Glu Tyr Met Asp Arg
675 680 685

ttc tac tcc aag gcc atg agc aac tac atc tgg ctc acg agg gag aac 2112
Phe Tyr Ser Lys Ala Met Ser Asn Tyr Ile Trp Leu Thr Arg Glu Asn

```
    690                 695                 700

tac aag ggg gcc agg gaa ata gcg gcc tgg aag gag agg                    2151
Tyr Lys Gly Ala Arg Glu Ile Ala Ala Trp Lys Glu Arg
705                 710                 715
```

<210> 6
<211> 717
<212> PRT
<213> Thermococcus zilligii

<400> 6

```
Met Ala Asp Val Ser His Thr Val Glu Asn Leu Ile Arg Ala Lys Leu
1               5                   10                  15

Pro Tyr Pro Leu Glu Asn Leu Ala Glu Leu Ala Tyr Asn Tyr Trp Trp
            20                  25                  30

Ser Trp Asn Arg Arg Ala Thr Arg Leu Trp Glu Tyr Ile Asp Ser Glu
        35                  40                  45

His Trp Arg Glu Tyr Lys Asn Pro Val Lys Leu Leu Leu Asp Thr Pro
    50                  55                  60

Glu Glu Arg Phe Trp Glu Leu Leu Lys Asp Asp Asp Phe Met Asn Leu
65                  70                  75                  80

Tyr Glu Leu Val Met Asp Gln Phe Thr Ala Tyr Met Asn Pro Lys Ser
                85                  90                  95

Thr Trp Phe Ser Thr Asn Tyr Pro Lys Trp Asp Lys Pro Ile Val Tyr
            100                 105                 110

Leu Cys Met Glu Tyr Gly Ile Ser Lys Ser Leu Pro Ile Tyr Ser Gly
        115                 120                 125

Gly Leu Gly Ile Leu Ala Gly Asp His Leu Lys Thr Ala Ser Asp Leu
    130                 135                 140

Gly Leu Pro Phe Ile Ala Ile Gly Leu Leu Tyr Lys His Gly Tyr Phe
145                 150                 155                 160

Arg Gln Glu Ile Asp Arg Asp Gly Arg Gln Arg Glu Ile Phe Pro Glu
                165                 170                 175

Tyr Arg Pro Glu Glu Met Pro Ile Lys Pro Val Leu Ser Lys Asp Gly
            180                 185                 190

Lys Pro Leu Leu Val Glu Val Pro Ile Glu Asn Arg Ile Val Tyr Ala
```

195 200 205

Arg Ala Phe Glu Val Ser Val Gly Met Ala Lys Leu Tyr Leu Leu Asp
210 215 220

Thr Asp Val Pro Glu Asn Ser Pro Glu Asp Arg Ala Ile Cys Asp Tyr
225 230 235 240

Leu Tyr Asn Ala Glu Met Asp Lys Arg Ile Lys Gln Glu Ile Leu Leu
245 250 255

Gly Ile Gly Gly Met Arg Leu Leu Gln Ala Leu Gly Ile Glu Pro Gly
260 265 270

Val Val His Leu Asn Glu Gly His Pro Ala Phe Ala Asn Leu Gln Arg
275 280 285

Ile Ala Trp Tyr Met Glu Lys Gly Leu Thr Phe Thr Glu Ala Leu Ser
290 295 300

Ile Val Arg Gly Thr Thr Val Phe Thr Thr His Thr Pro Val Pro Ala
305 310 315 320

Gly His Asp Arg Phe Pro Ile Ala Glu Val Arg Lys Arg Leu Ser Lys
325 330 335

Phe Leu Glu Gly Arg Glu Glu Leu Leu Glu Leu Gly Arg Glu Gly Asp
340 345 350

Gln Ile Asn Met Thr Leu Leu Ala Ile Arg Thr Ser Ser Tyr Val Asn
355 360 365

Gly Val Ser Gln Leu His Ala Glu Val Ser Lys Arg Met Trp Lys Asp
370 375 380

Leu Trp Pro Gly Val Pro Leu Asn Glu Ile Pro Ile Glu Gly Ile Thr
385 390 395 400

Asn Gly Val His Thr Met Thr Trp Val His Asp Glu Met Arg Lys Leu
405 410 415

Phe Asp Arg Tyr Ile Gly Lys Val Trp Arg Glu His Thr Asn Ile Glu
420 425 430

Gly Ile Trp Tyr Ala Val Glu Arg Ile Pro Asp Glu Glu Leu Trp Gly
435 440 445

```
Ala His Leu Glu Ala Lys Arg Gln Leu Ile Glu Phe Leu Arg Glu Lys
    450                 455                 460

Thr Met Glu Arg Asn Arg Arg Leu Gly Thr Asp Asp Pro Ile Pro Glu
465                 470                 475                 480

Ile Asp Glu Asn Ala Leu Ile Ile Gly Phe Ala Arg Arg Phe Ala Thr
                485                 490                 495

Tyr Lys Arg Ala Thr Leu Ile Leu Ser Asp Leu Glu Arg Leu Lys Lys
            500                 505                 510

Ile Leu Asn Asn Pro Glu Arg Pro Val Tyr Ile Val Phe Gly Gly Lys
        515                 520                 525

Ala His Pro Arg Asp Glu Ala Gly Lys Glu Phe Leu Arg Arg Val Tyr
    530                 535                 540

Glu Val Ser Gln Met Pro Glu Phe Arg Gly Lys Ile Phe Val Leu Glu
545                 550                 555                 560

Asn Tyr Asp Met Gly Ser Ala Arg Leu Met Val Ala Gly Val Asp Val
                565                 570                 575

Trp Leu Asn Asn Pro Arg Arg Pro Leu Glu Ala Ser Gly Thr Ser Gly
            580                 585                 590

Met Lys Ala Gly Leu Asn Gly Val Leu Asn Ala Ser Ile Phe Asp Gly
        595                 600                 605

Trp Trp Val Glu Gly Tyr Asn Gly Lys Asn Gly Trp Val Ile Gly Glu
    610                 615                 620

Glu Thr Thr Glu Pro Glu Ser Glu Glu Asp Asp Ala Lys Asp Ala Glu
625                 630                 635                 640

Ser Leu Tyr Thr Leu Leu Glu Lys Glu Ile Ile Pro Thr Tyr Tyr Gly
                645                 650                 655

Asn Arg Ser Arg Trp Ile Tyr Met Met Lys Glu Ser Ile Lys Ser Ile
            660                 665                 670

Ala Pro Arg Phe Ser Thr His Arg Met Val Lys Glu Tyr Met Asp Arg
        675                 680                 685

Phe Tyr Ser Lys Ala Met Ser Asn Tyr Ile Trp Leu Thr Arg Glu Asn
    690                 695                 700
```

```
Tyr Lys Gly Ala Arg Glu Ile Ala Ala Trp Lys Glu Arg
705                 710                 715
```

## Description

### TECHNICAL FIELD

The present invention relates to a glucan in which at least one (preferably at least two) non-reducing end is modified, a modified product thereof, as well as a method for producing the same, and utilization of the same. More preferably, the present invention relates to a glucan in which at least one residue selected from an N-acetylglucosamine residue and a galactose residue is bound via an α-1,4-bond to each of at least one (preferably at least two) non-reducing end, a modified product thereof, as well as a method for producing the same, and utilization of the same.

### BACKGROUND ART

A medically effective ingredient of medicaments is rapidly changing from a chemically synthesized stable low-molecular weight compound to an unstable substance which is easily degraded in blood, such as a protein, an antibody and a nucleic acid. For this reason, there is a necessity of stabilizing these unstable medically effective ingredients to keep the blood concentration of the medically effective ingredient high for an elongated time. In addition, in order to decrease side effects of drugs, a necessity of delivering drugs to a target tissue efficiently has been increasing. Under such a background, a so-called drug delivery system (DDS) technique has been utilized in earnest (Non-Patent Documents 1 to 4). A DDS technique refers to a technique and a system for allowing a medically effective ingredient to act on "a required site" at "a required amount" for "a required period of time", that is, controlling it with an ideal pharmacokinetics for a drug to maximally exert the effect.

In the DDS technique, a modifying material for a medically effective ingredient is important. The term "modifying material for a medically effective ingredient" in the present specification refers to a material which modifies a medically effective ingredient by covalently binding, or via non-covalent type interaction, with a medically effective ingredient. By utilizing the modifying material, a variety of properties (for example, pharmacokinetics (for example, absorption, distribution, metabolism and excretion), pharmacological effect, stability and the like) of the medically effective ingredient can be modified. As a substance which has been used previously as the modifying material for the medically effective ingredient, there are a variety of substances, most generally it is a macromolecular material. For example, polyethylene glycol (PEG) which is a synthetic macromolecule, and derivatives thereof are widely utilized as a modifying material for medically effective ingredients. Many medically effective ingredient-modifying materials having a functional group for binding the medically effective ingredient on a terminus of a PEG chain have been developed, and such modifying materials are actually utilized in producing a medicament. Specific application examples include pegylated interferon α (product name: PEGASYS). Since interferon α has a small molecular weight and is easily excreted into urine, there was a problem that it has a short half-life in blood. However, the half-life in blood was successfully enhanced dramatically by covalently binding interferon α to a PEG chain having a molecular weight of 40,000 to form an interferon α-conjugate having a high molecular weight. As described above, the remarkable effect is recognized in the modification of the medically effective ingredient or the nanoparticulate carrier for DDSs, with a macromolecular material.

However, on the other hand, a problem has been pointed out. For example, when a high-molecular weight synthetic macromolecule which has no degradability in a living body and will not undergo renal glomerular filtration is administered to blood, there is a risk that the macromolecule is accumulated in a particular organ and a risk that side effects due to the accumulation are generated. The reason is that a molecule having a molecular weight of a few tens thousands or less present in blood, undergoes renal glomerular filtration and is rapidly excreted into urine, but a molecule having a molecular weight of a few tens thousands or more does not undergo renal glomerular filtration and its excretion into urine is limited. For this reason, a modifying material of the medically effective ingredient which can be safely utilized is expected.

Polysaccharides have been used as a food raw material for a long time and, in recent years, have begun to be paid attention as a macromolecular material which is environment friendly, as a safe material having biocompatibility and, further, as a functional material.

An α-1,4 glucan, represented by a starch, glycogen or the like, is a safe natural material which is easily degraded in the environment and also in a body of a human, and is promising as a material in the industry of a DDS medicament, regenerative medicine, intracorporeal imaging or the like, which is expected to grow in the future. When the α-1,4 glucan is used for such a purpose, it is necessary to impart to the α-1,4 glucan a function of interacting with a medically effective ingredient and a function of targeting to an organ and a tissue, and at the same time, it is necessary to control degradability of the α-1,4 glucan.

On the other hand, in the pharmaceutical field, a vaccine for preventing or treating an infectious disease, a cancer or the like is being developed. The vaccine is for administration of an antigen into a body and preventing or treating a disease utilizing an immune response to the antigen. Usually, as the antigen, in the case of a vaccine for an infectious disease, a part of a pathogen, a whole inactivated pathogen, or a surface protein or a peptide of a pathogen

is used. As the antigen, in the case of a cancer vaccine, a protein or a peptide which is specifically expressed on a cancer cell surface is used. Generally, since mere administration of an antigen alone does not lead to effective immunity induction in many cases, a substance which induces an immune response (adjuvant) is administered into a body together with the antigen. For developing an effective vaccine, it is important to develop a safe and effective adjuvant. It is expected an effective and safe adjuvant effectively functions when a purified protein or peptide is administered as an antigen.

In recent years, due to progress of natural science, a mechanism of immunological system has been revealed. It has been revealed that a dendritic cell or a macrophage which is an antigen-presenting cell plays a central role of an immune reaction, and activation of an antigen-presenting cell and induction of production of an inflammatory cytokine (IL-2, IL-6, IL-12, TNF-$\alpha$ and the like) that results from the activation, are necessary for effective immunity induction. It has also been revealed that a substance which has been traditionally utilized as an adjuvant is involved in induction and enhancement of an immune reaction through stimulation and activation of an antigen-presenting cell, and induction of production of an inflammatory cytokine ("Janeway's Immunobiology" (translation under supervision by Takehiko Sasazuki, Nankodo Co., Ltd.) (original book "Janeway's Immunobiology seventh edition Kenneth Murphy, Paul Travers, Mark Walport 2008 Garlnd Science, Taylar & Francis Group, LLC")).

Conventionally, a variety of adjuvants are known, but there are few safe and low cost immune adjuvants which can be used in tumor immunotherapy for the purpose of treating a human tumor, or preventing metastasis and reoccurrence of a human tumor. For example, in tumor immunotherapy utilizing a cultured dendritic cell, keyhole limpet hemocyanin is used as an immune adjuvant (Geiger, J. D., et al., Cancer Res., 61, pp. 8513-8519, 2001), but keyhole limpet hemocyanin is expensive. A method of administering cytokines such as granulocyte-macrophage colony stimulating factor (sometimes abbreviated as "GM-CSF") which directly activates a dendritic cell as an immune adjuvant has been proposed, but cytokines are further expensive.

In the production of a vaccine as a countermeasure for an infectious disease, as a safe and low cost immune adjuvant, immune adjuvants having insufficient immune adjuvant activity such as alum (aluminum hydroxide) and Freund's incomplete adjuvant (since this adjuvant is oily, toxicity is feared) are used. These are low in toxicity, but are weak in the immune adjuvant activity, as compared with Freund's complete adjuvant which is used in an animal experiment.

As described above, the conventional adjuvant (also referred to as an immune adjuvant) has a problem in the ability to stimulate an immune response, safety or both of them. For this reason, it is very important to develop an adjuvant which has a high ability to stimulate an immune response, that is, the ability to stimulate an antigen-presenting cell, and is safe.

[Prior Art Documents]

[Non-Patent Documents]

[Non-Patent Document 1] Hiroaki Okada, "Drug delivery using functional DDS carriers" Chapter 1, General Statement: Kinousei DDS carrier wo mochiita seizai sekkei ni yoru soyaku (Drug Discovery by Preparation Design Using Functional DDS Carrier), CMC Publishing Co., Ltd., 2008, 1-23

[Non-Patent Document 2] Masayuki Yokoyama, "Polymeric materials for drug carriers, Special Topic, DDS ni riyou sareru kobunshi kagaku (Polymer Chemistry Utilized in DDS) ", Drug Delivery System 23-6, 2008: 610-617

[Non-Patent Document 3] Maria Laura Immordino et al., International Journal of Nanomedicine 2006: 1(3) 297-315

[Non-Patent Document 4] J. Milton Harris and Robert B. Chess, NATURE REVIEWS, DRUG DISCOVERY, VOLUME 2, MARCH 2003, 214-221

SUMMARY OF THE INVENTION

PROBLEMS TO BE SOLVED BY THE INVENTION

The present invention is intended to solve the above-mentioned problems.

An ideal modifying material for the medically effective ingredient which can be safely utilized is thought to have the following characteristics:

(1) The modifying material shall be a macromolecular material which can be degraded in a living body;
(2) The modifying material shall have such a stable quality that it can be usable as a medicament. That is, the structure can be specified, and a modifying material having the same quality shall be able to produce every time;
(3) The modifying material shall have a functional group which can bind to or interact with a medically effective ingredient; and

(4) The modifying material shall have tissue targeting functions or cell stimulation functions.

The present inventors thought that an excellent macromolecular substance for modifying material for the medically effective ingredient is a glucan (in the present specification, the glucan refers to an α-1,4-glucan, and an α-1,4-glucan which is branched with an α-1,6-bond(s)). Glycogen or a starch is accumulated in an animal' s and plant' s body as a polysaccharide for storage. Glycogen or a starch is one kind of glucan. Thus, glycogen or a starch which is a component that is always present in a body of a human, and is excellent in biocompatibility. Further, the glucan undergoes hydrolysis by α-amylase in a body, and is converted into glucose or a maltooligosaccharide being a component which is always present in a body. For this reason, the glucan can be said to be the safest macromolecular material.

The glucan has another advantage that design of its structure is relatively easy. Methods for controlling the molecular weight, degree of branching, cyclization and the like of the glucan are known. Completely linear glucans in which glucose residues are bound only with an α-1,4-bond, glucans which are branched at high frequency by some glucose residues bound with an α-1,6-bond, and the like are available. In a branched glucan, every time an α-1,6-bond is increased by one, one new non-reducing end is generated. The number of α-1,6-bonds can be suitably adjusted as desired_upon synthesis of a glucan molecule.

On the other hand, the greatest problem when the glucan is utilized as the modifying material (for example, a carrier, a vaccine adjuvant or the like) as a medically effective ingredient is that the glucan does not have a functional group which can bind to or interact with the medically effective ingredient. It is possible to introduce a cationic or anionic functional group into a large number of hydroxyl groups or aldehyde group on a reducing end present in a glucan using a known chemical method.

Further, it is preferable that a carrier has the tissue targeting function, in terms of utilization of a glucan as a carrier of a medically effective ingredient. As a means for realizing targeting to an organ and a tissue, a method of utilizing a saccharide recognizing mechanism by a receptor or a lectin on a cell surface has been proposed. A receptor or a lectin on a cell surface does not recognize glucose, but recognizes a monosaccharide such as galactose or mannose. Therefore, in order to impart the tissue targeting function to a glucan, it is necessary to bind a monosaccharide having the targeting function, other than glucose, to a non-reducing end of the glucan.

In order to utilize a glucan as a vaccine adjuvant, it is preferable that the glucan has high stimulating activity on an antigen-presenting cell playing a central role in an immune response. The antigen-presenting cell (a dendritic cell, a macrophage, or the like) is not activated by a glucan consisting only of glucose residues, but is activated by a monosaccharide such as galactose or mannose. Therefore, in order to impart the high cell stimulating activity to a glucan, it is necessary to bind a monosaccharide having a cell stimulating function to a non-reducing end of the glucan. However, a method of binding a monosaccharide other than glucose via α-1,4 bond to a non-reducing end of a branched glucan having at least two non-reducing ends has not been previously known. For example, in Nawaj i et al., Carbohydr. Res., 2008, 343, 2692-2696, it is described that α-D-glucosamine-1-phospate (GlcN-1-P) can be transferred to a maltooligosaccharide using potato-derived glucan phosphorylase. However, Nawajietal. describe on page 2694, right column, lines 3-5 that N-acetylglucosamine-1-phosphate (GlcNAc-1-P) is not recognized by phosphorylase. Recognition of a substrate by an enzyme is extremely strict, and it is a common knowledge of those skilled in the art that a normal substrate becomes unrecognizable as a substrate in many cases when it is modified even if only a little. A normal substrate for glucan phosphorylase is glucose-1-phosphate, and analogues of the substrate which were known to be recognized by glucan phosphorylase were only α-D-xylose-1-phosphate, mannose-1-phosphate, glucosamine-1-phosphate, glucuronic acid-1-phosphate (GlcA-1-P) and N-formylglucosamine-1-phosphate (GlcNF-1-P). For this reason, it was thought that phosphorylase cannot be used in order to transfer GlcNAc-1-P or galactose-1-phosphate (Gal-1-P) to a glucan.

MEANS FOR SOLVING THE PROBLEMS

The present inventors intensively studied in order to solve the aforementioned problems and found that *Aquifex aeolicus* VF5-derived α-glucan phosphorylase can utilize as a substrate galactose-1-phosphate (Gal-1-P) and N-acetylglucosamine-1-phosphate (GlcNAc-1-P) which are not its original substrates, can catalyze a reaction that binds a galactose residue (Gal residue) or an N-acetylglucosamine residue (GlcNAc residue) via α-1,4 bond to a non-reducing end of a α-1,4 glucan, and in spite of this, this enzyme can hardly catalyze the reverse reaction thereof, and completed the present invention based on this finding. In addition, the present inventors also found that potato-derived α-glucan phosphorylase can use Gal-1-P as a substrate, and *Thermococcus zilligii* AN1-derived α-glucan phosphorylase can use Gal-1-P and GlcNAc-1-P as a substrate. These α-glucan phosphorylases are enzymes which, when act on glucose-1-phosphate which is the original substrate, catalyze both a reaction wherein a glucose residue is transferred to a glucan receptor (glucan synthesizing reaction) and a reaction wherein the glucose on the non-reducing end of a glucan is phosphorolysed to generate glucose-1-phosphate (glucan degrading reaction). However, surprisingly, when these enzyme use N-acetylglucosamine-1-phosphate or galactose-1-phosphate as a substrate, these enzymes catalyze the reaction wherein the residue of these monosaccharides is transferred to a glucan receptor (glucan synthesizing reaction),

but hardly catalyze the reaction wherein the residue of these monosaccharides bound on the non-reducing end of a glucan is phosphorolysed to generate N-acetylglucosamine-1-phosphate or galactose -1-phosphate (glucan degrading reaction). While glucan degrading reaction again releases the bound monosaccharide residue, the present inventors found that in the method of the present invention, α-glucan phosphorylase hardly catalyzes the glucan degrading reaction. That is, the catalytic activity for the synthesizing reaction is much higher than the catalytic activity for the degradation reaction. Due to this characteristic, if a glucan has a plurality of non-reducing ends, α-glucan phosphorylase can bind at least one residue selected from N-acetylglucosamine residue and galactose residue, one by one, to the plurality of non-reducing ends of the glucan. By utilizing this characteristic of this enzyme, the non-reducing end-modified glucan of the present invention became producible for the first time. Especially, it could be obtained for the first time by utilizing this characteristic of this enzyme that a non-reducing end-modified glucan having high frequency of (for example, 50% or more) binding rate of N-acetylglucosamine residues or galactose residues (for example, glucosamine residues) to the non-reducing ends, starting with a glucan having a large number of (for example, 5 or more) non-reducing ends. In addition, the present inventors found that when a Gal residue or a GlcNAc residue is bound via α-1,4 bond to a non-reducing end of an α-1,4 glucan, glucoamylase resistance can be imparted to the α-1,4 glucan. Further, the present inventors found that the non-reducing end-modified glucan of the present invention can be accumulated into a particular tissue (e.g. liver) when it is administered into blood. The present inventors also found that the non-reducing end-modified glucan of the present invention has the activity of stimulating an antigen-presenting cell (e.g. a dendritic cell or a macrophage) to produce IL-6 (antigen-presenting cell stimulating activity). In addition, the present inventors found that the non-reducing end-modified glucan of the present invention is excellent in biodegradability, and degradation of the non-reducing end-modified glucan in a living body can be controlled by modifying (e.g. acetylating) a hydroxyl group of the non-reducing end-modified glucan of the present invention.

In the non-reducing end-modified branched α-1,4 glucan (e.g. a branched α-1,4 glucan in which at least one residue selected from an N-acetylglucosamine residue and a galactose residue is bound via α-1,4 bond to each of at least one (preferably at least two) non-reducing end(s)), or a hydroxyl group-modified product thereof, a non-reducing end-modified product thereof and a reducing end-modified product thereof of the present invention, when at least one kind of residue selected from a glucuronic acid residue, a mannose residue and a xylose residue is further bound to at least one or more non-reducing ends among the plurality of non-reducing ends, the antigen-presenting cell stimulating activity of these glucans can be further improved.

In the present invention, since a saccharide (e.g. N-acetylglucosamine or galactose) residue is transferred using α-glucan phosphorylase (EC2.4.1.1), linkage of a saccharide residue to a glucan is an α-1,4 bond. That is, a carbon atom at a 4-position of a glucosyl residue at a non-reducing end of a glucan, and a carbon atom at a 1-position of a saccharide (e.g. N-acetylglucosamine or galactose) residue are α-bound via an oxygen atom.

In the present specification, the glucan of the present invention in which at least one residue selected from an N-acetylglucosamine residue and a galactose residue is bound via α-1,4 bond to each of at least one non-reducing end of a glucan is also referred to as a "non-reducing end-modified glucan" of the present invention.

A saccharide residue to be bound to a non-reducing end in the non-reducing end-modified glucan of the present invention may be a residue of a monosaccharide, or may be a residue of an oligosaccharide. That is, the saccharide residue to be bound to a non-reducing end may be an N-acetylglucosamine residue or a galactose residue, or an oligosaccharide residue thereof. In the present specification, an oligosaccharide means a compound wherein 2 or more and 10 or less monosaccharides are bound. The degree of polymerization of saccharide residue bound to one non-reducing end of the non-reducing end-modified glucan of the present invention can be, for example, about 2 or more, about 3 or more, about 4 or more, about 5 or more or the like, and can be, for example, about 10 or less, about 9 or less, about 8 or less, about 7 or less, about 6 or less, about 5 or less, about 4 or less, about 3 or less, about 2 or less or the like. In one embodiment, the saccharide residue bound to one non-reducing end of the non-reducing end-modified glucan of the present invention is residue of 2 sugars (that is, a dimer).

The introduction amount of galactose residues or N-acetylglucosamine residues to a glucan can be controlled by the branching frequency of the glucan used and the frequency of introduction of these residues to a non-reducing end. When one wants to increase an amount of introduction of these residues into a glucan, using a glucan having a high branching frequency and increasing the frequency of introduction of these residues to the non-reducing end. When one wants to decrease an amount of introduction of these residues into a glucan, using a glucan having a low branching frequency or decreasing the frequency of introduction of these residues to the non-reducing end. The lower limit of the introduction amount of these residues to a glucan is the state wherein one of these residues is introduced per glucan molecule, which can be attained by introducing a galactose residue or an N-acetylglucosamine residue to the non-reducing end of the glucan having no branching. In the case of a glucan which is highly branched, since non-reducing ends are distributed in an outermost layer of a glucan molecule, it is considered that introduced either of these residues are distributed in an outermost layer of a glucan molecule after introduction of either of these residues, and this is ideal for interaction and binding with the medically effective ingredient. As described above, a glucan which has either of these residues selectively bound to a non-reducing end has a possibility of being an excellent modifying material for the

medically effective ingredient.

For example, the present invention provides the following glucans and the like:

(Item 1)

A glucan wherein the glucan has non-reducing ends and at least one residue selected from an N-acetylglucosamine residue and a galactose residue is bound via an α-1,4-bond to each of at least one of the non-reducing ends of the glucan (preferably two or more of the two or more non-reducing ends), but neither an N-acetylglucosamine residue nor a galactose residue is present at the position other than the non-reducing ends of the glucan, wherein the glucan is a branched α-1,4 glucan or a linear α-1,4 glucan, and the degree of polymerization of the branched α-1,4 glucan or the linear α-1,4 glucan is preferably 15 or more and 4 x $10^5$ or less. It is noted that the glucan which has an N-acetylglucosamine residue or a galactose residue bound thereto of the present invention is also referred to as the non-reducing end-modified glucan of the present invention.

(Item 2)

The glucan according to item 1, wherein the glucan is a branched α-1,4-glucan, wherein the branched α-1,4-glucan has a plurality of non-reducing ends and at least one residue selected from an N-acetylglucosamine residue and a galactose residue is bound to each of at least one (preferably two or more) non-reducing ends of the branched α-1,4-glucan. That is, this glucan is a branched α-1,4-glucan wherein the branched α-1,4-glucan has a plurality of non-reducing ends and at least one residue selected from an N-acetylglucosamine residue and a galactose residue is bound via an α-1,4-bond to each of at least one (preferably two or more) non-reducing ends of the branched α-1,4-glucan, but neither an N-acetylglucosamine residue nor a galactose residue is present at the position other than the non-reducing ends of the branched α-1,4-glucan, and the degree of polymerization of the branched α-1,4 glucan is preferably 15 or more and 4 x $10^5$ or less.

(Item 3)

The glucan according to item 2, wherein the branched α-1,4-glucan is selected from the group consisting of a branched maltooligosaccharide, starch, amylopectin, glycogen, dextrin, enzymatically synthesized branched glucan and highly branched cyclic glucan.

(Item 4)

Ahydroxyl group-modifiedproduct of the glucan (non-reducing end-modified glucan) according to any one of items 1 to 3, wherein the modification on the hydroxyl group is a modification on some or all of alcoholic hydroxyl groups of the glucan, and the modification on the hydroxyl group is independently selected from the group consisting of hydroxyalkylation, alkylation, acetylation, carboxymethylation, sulfation and phosphorylation.

(Item 5)

A reducing end-modified product of the glucan (non-reducing end-modified glucan) according to any one of items 1 to 3 or a hydroxyl group-modified product thereof.

(Item 6)

A non-reducing end-modified product of the glucan (non-reducing end-modified glucan) according to any one of items 2 to 3 or a hydroxyl group-modified product thereof, or a reducing end-modified product thereof, which is further modified by liking a monosaccharide residue other than an N-acetylglucosamine residue and a galactose residue via an α-1,4-bond to at least one non-reducing end of the plurality of non-reducing ends of the branched α-1,4 glucan.

(Item 6B)

The non-reducing end-modified product of the glucan (non-reducing end-modified glucan) or a hydroxyl group-modified product thereof, or a reducing end-modified product thereof according to item 6, wherein the monosaccharide residue is one kind or two kinds selected from a glucuronic acid residue, a glucosamine residue, a mannose residue, and a xylose residue.

(Item 6C)

The non-reducing end-modified product of the glucan (non-reducing end-modified glucan) or a hydroxyl group-modified product thereof, or a reducing end-modified product thereof according to item 6, wherein the monosaccharide residue is one kind or two kinds selected from a glucuronic acid residue and a mannose residue.

(Item 7)

A method for producing a glucan (that is, a non-reducing end-modified glucan of the present invention) in which at least one residue selected from an N-acetylglucosamine residue and a galactose residue is bound to each of two or more non-reducing ends, characterized by allowing an α-glucan phosphorylase to act on an aqueous solution comprising a glucan (preferably a branched α-1,4-glucan having two or more non-reducing ends) and N-acetylglucosamine-1-phosphate or galactose-1-phosphate, wherein the degree of polymerization of the glucan used as raw material is preferably 15 or more and 4 x $10^5$ or less.

(Item 8)

The method according to item 7, wherein the α-glucan phosphorylase has 95% or more sequence identity with the

amino acid sequence of α-glucan phosphorylase derived from *Aquifex aeolicus* VF5, and has activity of transferring N-acetylglucosamine residue or galactose residue to a non-reducing end of a glucan to form an α-1,4-bond.
(Item 9)
A medicament comprising:

the glucan comprising an N-acetylglucosamine residue or a galactose residue according to any one of items 1 to 3, a hydroxyl group-modified product thereof, or a reducing end-modified product thereof, and
a medically effective ingredient.

(Item 10)
A medicament comprising:

a non-reducing end-modified product of the glucan comprising an N-acetylglucosamine residue or a galactose residue according to any one of items 2 to 3 or a hydroxyl group-modified product thereof, or a reducing end-modified product thereof, which is further modified by liking a monosaccharide residue other than an N-acetylglucosamine residue and a galactose residue via an α-1,4-bond to at least one non-reducing end of the plurality of non-reducing ends of the branched α-1,4 glucan, wherein the monosaccharide residue is one kind or two kinds selected from a glucuronic acid residue, a glucosamine, a mannose residue, and a xylose residue; and
a medically effective ingredient.

(Item 11)
The medicament according to item 9 or 10, wherein the medically effective ingredient is selected from the group consisting of a low-molecular weight organic compound, a protein, a peptide, an antibody, an antibody fragment, a receptor, a receptor fragment, a DNA, an RNA, a siRNA, an miRNA and an RNA aptamer.
(Item 11A)
The medicament according to any one of items 9 to 11, wherein the medically effective ingredient is an antigen protein or a peptide.
(Item 12)
A composition for clinical diagnosis comprising the glucan comprising an N-acetylglucosamine residue or a galactose residue according to any one of items 1 to 3, a hydroxyl group-modified product thereof, or a reducing end-modified product thereof.
(Item 13)
A composition for clinical diagnosis comprising a non-reducing end-modified product of the glucan comprising an N-acetylglucosamine residue or a galactose residue according to any one of items 2 to 3 or a hydroxyl group-modified product thereof, or a reducing end-modified product thereof, which is further modified by liking a monosaccharide residue other than an N-acetylglucosamine residue and a galactose residue via an α-1,4-bond to at least one non-reducing end of the plurality of non-reducing ends of the branched α-1,4 glucan, wherein the monosaccharide residue is one kind or two kinds selected from the group consisting of a glucuronic acid residue, a glucosamine, a mannose residue, and a xylose residue.
(Item 14)
A nanoparticulate carrier for a DDS comprising the glucan comprising an N-acetylglucosamine residue or a galactose residue according to any one of items 1 to 3, a hydroxyl group-modified product thereof, or a reducing end-modified product thereof.
(Item 15)
A nanoparticulate carrier for a DDS comprising a non-reducing end-modified product of the glucan comprising an N-acetylglucosamine residue or a galactose residue according to any one of items 2 to 3 or a hydroxyl group-modified product thereof, or a reducing end-modified product thereof, which is further modified by liking a monosaccharide residue other than an N-acetylglucosamine residue and a galactose residue via an α-1,4-bond to at least one non-reducing end of the plurality of non-reducing ends of the branched α-1,4 glucan, wherein the monosaccharide residue is one kind or two kinds selected from the group consisting of a glucuronic acid residue, a glucosamine, a mannose residue, and a xylose residue.
(Item 16)
The carrier according to item 14 or 15, wherein the nanoparticulate carrier for a DDS is selected from the group consisting of a liposome, a virus particle, a macromolecule micelle and a nanogel composed of macromolecule bearing hydrophobic groups.
(Item 17)
A vaccine adjuvant comprising the glucan comprising an N-acetylglucosamine residue or a galactose residue according to any one of items 1 to 3, a hydroxyl group-modified product thereof, or a reducing end-modified product

thereof.

(Item 18)

The vaccine adjuvant according to item 17, further modified by liking a monosaccharide residue other than an N-acetylglucosamine residue and a galactose residue via an α-1,4-bond to at least one or more non-reducing end of the plurality of non-reducing ends of the branched α-1,4 glucan, wherein the monosaccharide residue is one kind or two kinds selected from a glucuronic acid residue and a mannose residue.

EFFECTS OF THE INVENTION

It is thought that the non-reducing end-modified glucan (a glucan in which at least one residue selected from an N-acetylglucosamine residue and a galactose residue is bound via an α-1,4 bond to each of at least one (preferably at least two) non-reducing end(s)), a hydroxyl group-modified product thereof, a non-reducing end-modified product thereof and a reducing end-modified product thereof of the present invention can be accumulated into a particular tissue when administered into blood. Since the non-reducing end-modified branched α-1,4 glucan (a branched α-1,4 glucan in which at least one residue selected from an N-acetylglucosamine residue and a galactose residue is bound via an α-1,4 bond to each of at least one (preferably at least two) non-reducing end(s)), a hydroxyl group-modified product thereof, a non-reducing end-modified product thereof and a reducing end-modified product thereof of the present invention have the activity of stimulating an antigen-presenting cell such as a dendritic cell or a macrophage to produce IL-6 (antigen-presenting cell stimulating activity), they are thought to be usable as a vaccine adjuvant. In the non-reducing end-modified branched α-1,4 glucan (a branched α-1,4 glucan in which at least one residue selected from an N-acetylglucosamine residue and a galactose residue is bound via an α-1,4 bond to each of at least one (preferably at least two) non-reducing end(s)), a hydroxyl group-modified product thereof, a non-reducing end-modified product thereof and a reducing end-modified product thereof of the present invention, when at least one kind of residue among a glucuronic acid residue, a mannose residue and a xylose residue is further bound to at least one or more non-reducing ends among the plurality of non-reducing ends, the antigen-presenting cell stimulating activity of these glucans can be further improved.

The non-reducing end-modified glucan, a hydroxyl group-modified product thereof, a non-reducing end-modified product thereof and a reducing end-modified product thereof of the present invention can have extended blood half life longer than that of an unmodified glucan, and are very safe since they are finally completely degraded in a living body and excreted. For this reason, these glucans and modified products thereof of the present invention are useful as a modifying material for a medically effective ingredient, a clinical diagnostic, an imaging agent and a nanoparticulate carrier for DDS. Since the non-reducing end-modified glucan, a hydroxyl group-modified product thereof, a non-reducing end-modified product thereof and a reducing end-modified product thereof of the present invention can be controlled in their structure by an enzymatic reaction, they are also excellent in quality stability.

BRIEF DESCRIPTION OF THE DRAWINGS

[Fig. 1] Fig. 1 shows the results of HPAEC-PAD analysis after digestion of a non-reducing end-modified branched glucan (B-GlcNAc) or an unmodified branched glucan (B) with various amylases. See Example 11.

[Fig. 2] Fig. 2 shows the results of HPAEC-PAD analysis after digestion of a non-reducing end-modified branched glucan (B-Gal) or an unmodified branched glucan (B) with various amylases. See Example 12.

[Fig. 3] Fig. 3 shows a schematic view of a structure of products which are thought to be obtained when a non-reducing end-modified branched glucan or an unmodified branched glucan (B) is degraded with various amylases. See Example 12.

[Fig. 4] Fig. 4 is a graph showing a change in weight average molecular weight of a product over time when a branched glucan which has an N-acetylglucosamine residue bound thereto is degraded with α-amylase. See Example 13.

[Fig. 5] Fig. 5 is a graph showing a change in weight average molecular weight over time when non-reducing end N-acetylglucosamine residue-bound branched glucans having different acetylation degrees are degraded with α-amylase. See Example 14.

[Fig. 6] Fig. 6 is a graph showing a change in weight average molecular weight of a product over time when a branched glucan which has a galactose residue bound thereto is degraded with α-amylase. See Example 15.

[Fig. 7] Fig. 7 is a graph showing a change in weight average molecular weight over time when non-reducing end galactose residue-bound branched glucans having different acetylation degrees are degraded with α-amylase. See Example 16.

[Fig. 8] Fig. 8 is a graph showing glucan residual ratios of an acetylated branched glucan which has N-acetylglucosamine residues bound to non-reducing ends and an acetylated non-reducing end-unmodified-branched glucan

(B) in blood. See Example 17.

[Fig. 9] Fig. 9 is a graph showing glucan residual ratios of an acetylated branched glucan which has galactose residues bound to non-reducing ends and an acetylated non-reducing end-unmodified-branched glucan (B) in blood. See Example 18.

[Fig. 10] Fig. 10 is a graph showing concentrations of an acetylated branched glucan which has a galactose residue bound to a non-reducing end and an acetylated non-reducing end unmodified branched glucan (B) in blood and liver. See Example 19.

[Fig. 11] Fig. 11 is a graph showing an IL-6 production amount when various non-reducing end-modified glucans are contacted with a dendritic cell. See Example 20.

[Fig. 12] Fig. 12 is a graph showing an IL-6 production amount when various non-reducing end-modified glucans are contacted with a dendritic cell. See Example 22.

[Fig. 13] Fig. 13 is a graph showing an IL-6 production amount when various non-reducing end-modified glucans are contacted with a dendritic cell. See Example 23.

[Fig. 14] Fig. 14 is a graph showing an IL-6 production amount when various non-reducing end-modified glucans are contacted with a dendritic cell. See Example 24.

[Fig. 15] Fig. 15 is a graph showing an IL-6 production amount when various non-reducing end-modified glucans are contacted with a macrophage. See Example 25.

[Fig. 16] Fig. 16 is a graph showing an IL-6 production amount when various non-reducing end-modified glucans are contacted with a macrophage. See Example 26.

## MODE FOR CARRYING THE INVENTION

The present invention will be explained in detail below.

Throughout the present specification, it should be understood that expression in a singular form includes a concept of a plural form thereof, unless otherwise indicated. In addition, it should be understood that a term used in the present specification is used in a sense which is usually used in the art, unless otherwise indicated.

### (1. Materials)

### (1.1) Glucans and modified products of glucan

"Glucan", when used in the present specification, is a polysaccharide having D-glucose as a constituent unit. In the present invention, it is preferable to use, as the glucan, an α-D-glucan. The bonds which link glucose residues in an α-D-glucan predominantly consist of an α-1,4-glucosidic bond, and can contain an α-1,6-glucosidic bond. An α-D-glucan containing a α-1,6-glucosidic bond has a branched structure. A glucan having at least one α-1,6-glucosidic bond in the molecule is referred to as a branched glucan, and a glucan having no α-1,6-glucosidic bond in the molecule is referred to as a linear glucan. Preferable glucans used in the present invention are a linear glucan and a branched glucan, more preferably a linear α-1,4-glucan and an α-1,4-glucan which is branched with an α-1,6-bond (also referred to as a branched α-1,4 glucan). It is preferable that the glucan used in the present invention does not contain an α-1,3-bond.

The linear α-D-1,4-glucan refers to a polysaccharide in which two or more saccharide units of D-glucose units are bound only with an α-1,4-glucosidic bond(s). In the present specification, unless otherwise indicated, the linear α-D-1,4-glucan is referred to as a linear glucan or a linear α-1,4-glucan. The linear glucan has one non-reducing end. Examples of the linear glucan suitably utilized in the present invention include maltooligosaccharide and amylose.

In the present specification, the term "maltooligosaccharide" refers to a substance which is produced by dehydration condensation of about 2 to about 10 D-glucoses, wherein D-glucose units are bound by α-1,4 bond(s). The degree of polymerization of a maltooligosaccharide is preferably about 3 or more, more preferably about 4 or more, and further preferably about 5 or more. The degree of polymerization of a maltooligosaccharide may be, for example, about 10 or less, about 9 or less, about 8 or less, about 7 or less, or the like. Examples of maltooligosaccharides include maltooligosaccharides such as maltose, maltotriose, maltotetraose, maltopentaose, maltohexaose, maltoheptaose, maltooctaose, maltononaose, and maltodecaose.

In the present specification, the term "amylose" refers to a linear molecule constructed of glucose units connected via α-1,4-bonds. An amylose is contained in natural starch. Amylose may be a natural amylose extracted from a natural starch, or may be an amylose synthesized by an enzymatic reaction (also referred to as "enzymatically synthesized amylose" in the present specification). Natural amylose may contain a branched part in some cases, but enzymatically synthesized amylose does not contain a branch. Further, natural amyloses have a large polydispersity and has a variation in the molecular weight, but an enzymatically synthesized amylose (particularly, an enzymatically synthesized amylose synthesized by the SP-GP method described in International Publication WO 02/097107 pamphlet) has a small polydispersity and has an extremely uniform molecular weight. For this reason, in the present invention, it is preferable to

use an enzymatically synthesized amylose. The degree of polymerization of the amylose used in the present invention is preferably about 2 or more, more preferably about 3 or more, still more preferably about 10 or more, and most preferably about 30 or more. The degree of polymerization of the amylose used in the present invention is preferably about 2 x $10^3$ or less, more preferably about 1 x $10^3$ or less, still more preferably about 700 or less and most preferably about 500 or less.

In the present specification, the term "branched α-D-glucan" refers to a glucan in which a linear glucan, in which D-glucose units are connected with an α-1, 4-glucosidic bond(s), is branched with a bond other than an α-1,4-glucosidic bond. In the present specification, unless otherwise indicated, the branched α-D-glucan is referred to as a branched glucan. A branching bond is either an α-1,6-glucosidic bond, an α-1,3-glucosidic bond, or an α-1,2-glucosidic bond, and most preferably is an α-1,6-glucosidic bond. It is preferable that a branched α-D-glucan used in the present invention does not contain an α-1,3-glucosidic bond and an α-1,2-glucosidic bond. The branched glucan usually has the same number of non-reducing ends as the number of branching bonds. When the branched glucan is treated with an enzyme which selectively breaks only an α-1,6-glucosidic bond (for example, isoamylase, pullulanase, or the like), the branched glucan can be degraded into a mixture of linear α-1,4-glucans. These are referred to as a unit chain of the branched glucan, and the degree of polymerization thereof is referred to as a unit chain length.

Examples of the branched glucan suitably utilized in the present invention include branched α-1,4-glucans having 2 or more non-reducing ends, branched maltooligosaccharide, starches, amylopectin, glycogen, dextrin, enzymatically synthesized branched glucan and highly branched cyclic glucan. Branched CD having one non-reducing end is not preferable.

In the present specification, the term "branched maltooligosaccharide" refers to a substance generated by dehydration condensation of about 3 to about 10 D-glucoses, in which D-glucose units are bound mainly with an α-1,4 bond (s), and which contains one or more branching bonds, i.e., 2 or more non-reducing ends. The degree of polymerization of the branched maltooligosaccharide is preferably about 4 or more, more preferably about 5 or more, further preferably about 6 or more. The degree of polymerization of the branched maltooligosaccharide may be, for example, about 10 or less, about 9 or less, about 8 or less, about 7 or less, or the like.

In the present specification, the term "starch" is a mixture of amylose and amylopectin. As a starch, any starch can be used as long as it is commonly commercially available. The ratio of the amylose and amylopectin contained in a starch is different depending on the kind of plant producing the starch. Almost all starches possessed by glutinous rice, glutinous corn and the like are an amylopectin. On the other hand, a starch consisting only of amyloses, containing no amylopectin, can not be obtained from a common plant. Starch is classified into natural starch, a degraded starch and modified starch.

Natural starch is classified into tuber starch and cereal starch depending on the raw material. Examples of tuber starches include potato starch, tapioca starch, sweet potato starch, kudzu starch, bracken starch and the like. Examples of cereal starches include corn starch, wheat starch, rice starch and the like. Examples of natural starches are high amylose starches (for example, high amylose corn starch) or waxy starches. The starch can be a soluble starch. A soluble starch refers to a water-soluble starch obtained by subjecting a variety of treatment on natural starch. The starch may be selected from the group consisting of soluble starch, waxy starch and high amylose starch. The starch may be a modified starch.

The degree of polymerization of the starch used in the present invention is preferably about 1 x $10^3$ or more, more preferably about 5 x $10^3$ or more, still more preferably about 1 x $10^4$ or more, and most preferably about 2 x $10^4$ or more. The degree of polymerization of the starch used in the present invention is preferably about 1 x $10^7$ or less, more preferably about 3 x $10^6$ or less, still more preferably about 1 x $10^6$ or less and most preferably about 3 x $10^5$ or less.

An amylopectin is a branched molecule in which a glucose unit (s) is bound via an α-1,6 bond to glucose units which are bound via an α-1,4 bond(s). An amylopectin is contained in natural starch. As an amylopectin, for example, waxy corn starch, which consists of 100% amylopectin, can be used. The degree of polymerization of the amylopectin used in the present invention is preferably about 1 x $10^3$ or more, more preferably about 5 x $10^3$ or more, still more preferably about 1 x $10^4$ or more, and most preferably about 2 x $10^4$ or more. The degree of polymerization of the amylopectin used in the present invention is preferably about 1 x $10^7$ or less, more preferably about 3 x $10^6$ or less, still more preferably about 1 x $10^6$ or less and most preferably about 3 x $10^5$ or less.

A glycogen is one kind of glucan constructed of glucose, and is a glucan having a high frequency of branching. A glycogen is widely distributed as a storage polysaccharide for animals in almost all cells in the granule state. In a plant, glycogen is present, for example, in the seed of sweet corn species of corn. In a glycogen, typically, sugar chains consisting of glucoses bound via an α-1,4-bond(s) which have an average degree of polymerization of 12 to 18 are bound by an α-1,6-bond(s) at a ratio of around one chain every about 3 units of glucose, to a sugar chain consisting of glucoses bound via an α-1,4-bond(s). In addition, similarly, a sugar chain consisting of glucoses bound by an α-1,4-bond (s) is bound by an α-1,6-bond to a branch chain bound by an α-1,6-bond(s). For this reason, glycogen forms a network structure. It is also possible to enzymatically synthesize a glycogen. The degree of polymerization of the glycogen used in the present invention is preferably about 500 or more, more preferably about 1 x $10^3$ or more, still more preferably about 2 x $10^3$ or more, and most preferably about 3 x $10^3$ or more. The degree of polymerization of the glycogen used

in the present invention is preferably about 1 x $10^7$ or less, more preferably about 3 x $10^6$ or less, still more preferably about 1 x $10^6$ or less and most preferably about 3 x $10^5$ or less.

Dextrin is one kind of glucan constructed of glucose, and is a glucan having a medium complexity between those of starch and those of maltose. Dextrin is obtained by partially degrading starch by an acid, an alkyl or an enzyme. Dextrin is also obtained by partially degrading starch with a physical treatment such as sonication. The degree of polymerization of the dextrin used in the present invention is preferably about 10 or more, more preferably about 20 or more, still more preferably about 30 or more, and most preferably about 50 or more. The degree of polymerization of the dextrin used in the present invention is preferably about 1 x $10^4$ or less, more preferably about 9 x $10^3$ or less, still more preferably about 7 x $10^3$ or less and most preferably about 5 x $10^3$ or less.

The enzymatically synthesized branched glucan refers to a branched glucan synthesized using an enzyme. By adding a branching enzyme to the reaction solution upon synthesis of amylose by the SP-GP method, the product can be branched. The extent of branching can be regulated by the added amount of the branching enzyme. Since the enzymatically synthesized branched glucan has a uniform structure as compared with a natural branched glucan, it is very advantageous when used as a pharmaceutical material. For example, the degree of polymerization of the enzymatically synthesized branched glucan used in the present invention is preferably about 15 or more, preferably about 20 or more, more preferably about 50 or more, still more preferably about 100 or more, and most preferably about 200 or more. The degree of polymerization of the enzymatically synthesized branched glucan used in the present invention is preferably about 4 x $10^5$ or less, preferably about 2 x $10^5$ or less, more preferably about 1 x $10^5$ or less, still more preferably about 5 x $10^4$ or less and most preferably about 3 x $10^4$ or less.

In the present specification, the term "highly branched cyclic glucan" refers to a glucan having an internally branched cyclic structural moiety and an externally branched structural moiety and having a degree of polymerization of 50 or more. The highly branched cyclic glucan may have at least two non-reducing ends as a whole molecule. The degree of polymerization of the highly branched cyclic glucan as a whole molecule that can be used in the present invention is preferably about 50 or more, more preferably about 60 or more, and still more preferably about 100 or more. The degree of polymerization of the highly branched cyclic glucan as a whole molecule that can be used in the present invention is preferably about 1 x $10^5$ or less, more preferably about 7 x $10^4$ or less, and still more preferably about 5 x $10^4$ or less. The degree of polymerization of the highly branched cyclic glucan as a whole molecule that can be used in the present invention may be, for example, about 1 x $10^4$ or less, about 7 x $10^3$ or less, or about 5 x $10^3$ or less.

The degree of polymerization of the internally branched cyclic structural moiety present in the highly branched cyclic glucan is preferably about 10 or more, more preferably about 15 or more, and further preferably about 20 or more. The degree of polymerization of the internally branched cyclic structural moiety present in the highly branched cyclic glucan is preferably about 500 or less, more preferably about 300 or less, and further preferably about 100 or less.

The degree of polymerization of the externally branched structural moiety present in the highly branched cyclic glucan is preferably about 40 or more, more preferably about 100 or more, further preferably about 300 or more, and further more preferably about 500 or more. The degree of polymerization of the externally branched structural moiety present in the highly branched cyclic glucan is preferably about 3 x $10^3$ or less, more preferably about 1 x $10^3$ or less, further preferably about 500 or less, andfurther more preferably about 300 or less.

The number of $\alpha$-1,6-glucosidic bonds in the internally branched cyclic structural moiety present in the highly branched cyclic glucan may be at least one, and for example, can be one or more, 5 or more, 10 or more or the like; the number of $\alpha$-1,6-glucosidic bonds in the internally branched cyclic structural moiety can be, for example, about 200 or less, about 50 or less, about 30 or less, about 15 or less, about 10 or less or the like.

It is preferable that the highly branched cyclic glucan used in the present application is a highly branched cyclic glucan in which the number of non-reducing ends in the externally branched structural moiety is 2 or more. The number of non-reducing ends (that is, "the number of $\alpha$-1,6-glucosidic bonds" + 1) in the externally branched structural moiety present in the highly branched cyclic glucan is preferably about 2 or more, more preferably about 3 or more, further preferably about 4 or more, especially preferably about 5 or more, and most preferably about 10 or more. The number of non-reducing ends in the externally branched structural moiety present in the highly branched cyclic glucan is preferably about 5 x $10^3$ or less, more preferably about 4 x $10^3$ or less, and further preferably about 3 x $10^3$ or less.

As the highly branched cyclic glucan, a highly branched cyclic glucan having one kind of a degree of polymerization may be used alone, or a mixture of highly branched cyclic glucans having a variety of degree of polymerization may be used. Preferably, the degrees of polymerization of the highly branched cyclic glucan is such that the ratio of the degrees of polymerization of the maximum degree of polymerization to the minimum degree of polymerization is about 100 or less, more preferably about 50 or less, and further more preferably about 10 or less.

The highly branched cyclic glucan is preferably a glucan having an internally branched cyclic structural moiety and an externally branched structural moiety and having a degree of polymerization in a range of 50 to 5 x $10^4$, wherein the internally branched cyclic structural moiety is a cyclic structural moiety formed with an $\alpha$-1,4-glucosidic bond and an $\alpha$-1,6-glucosidic bond, and the externally branched structural moiety is a non-cyclic structural moiety bound to the internally branched cyclic structural moiety. The degree of polymerization of each unit chain of this externally branched

structural moiety is, on average, preferably about 10 or more and preferably about 20 or less. The highly branched cyclic glucan and a method for producing the same are described in detail in Japanese Laid-Open Publication No. 8-134104 (Japanese Patent No. 3107358), and this glucan can be produced according to the description of it. The highly branched cyclic glucan is commercially available, for example, as "Cluster Dextrin" from Ezaki Glico Co., Ltd. The degree of polymerization of the highly branched cyclic glucan used in the present invention is preferably about 50 or more, more preferably about 70 or more, further preferably about 100 or more, most preferably about 150 or more. The degree of polymerization of the highly branched cyclic glucan used in the present invention is preferably about 1 x $10^4$ or less, more preferably about 7 x $10^3$ or less, further preferably about 5 x $10^3$ or less, and most preferably about 4 x $10^3$ or less.

In a specific embodiment, the branched glucan can be particulate. It is known that particles having a diameter of about 4 nm or less are excreted from kidney, particles having a diameter of about 4 nm to about 200 nm are circulated in blood for a long time, particles having a diameter of about 200 nmto about 7 μm are captured by a reticuloendothelial system, and particles having a diameter of about 7 μm or more obstruct capillary blood vessels. A reticuloendothelial system is distributed in liver and spleen. For this reason, by controlling the particle size of the branched glucan, the pharmacokinetics of the non-reducing end-modified glucan and a modified product thereof of the present invention *in vivo* can be controlled. When one intends to circulate the particles in blood for a long time, the particle size of a particulate branched glucan is, as the diameter, preferably about 4 nm or more, more preferably about 10 nm or more, preferably about 200 nm or less, and more preferably about 100 nm or less. The molecular weight of the particulate branched glucan having such a particle size is preferably about 5 x $10^5$ or more, more preferably about 1 x $10^6$ or more, preferably about 5 x $10^7$ or less, and more preferably about 2 x $10^7$ or less. For example, since it is known that particles having a diameter of 20 to 50 nm are accumulated in cancer cells, when it is intended that the particles are accumulated in cancer cells, the particle size of the particulate branched glucan is, as the diameter, preferably about 10 nm or more, more preferably about 15 nmormore, preferably about 100 nmor less, and more preferably about 50 nm or less. The molecular weight of the particulate branched glucan having such a particle size is preferably about 5 x $10^5$ or more, more preferably about 1 x $10^6$ or more, preferably about 2 x $10^7$ or less, and more preferably about 5 x $10^6$ or less.

The number of branches in the α-glucan (i.e. the number of α-1,6-glucosidic bonds) is preferably about 1 or more, more preferably about 2 or more, further preferably about 5 or more, and most preferably about 10 or more. The number of branches in the α-glucan may be about 30 or more. The number of branches of the α-glucan (i.e. the number of α-1,6-glucosidic bonds) is preferably about 5 x $10^3$ or less, more preferably about 3 x $10^3$ or less, further preferably about 2 x $10^3$ or less. The number of branches in the α-glucan may be about 1 x $10^3$ or less.

In the branched α-glucan used in the present invention, the ratio of the number of α-1,6-glucosidic bonds relative to the number of α-1,4-glucosidic bonds ("number of α-1,6-glucosidic bonds" : "number of α-1,4-glucosidic bonds") is preferably 1 : 1 to 1 : 1 x $10^3$, more preferably 1 : 1.1 to 1 : 500, further preferably 1 : 1.2 to 1 : 100, further more preferably 1 : 1.5 to 1 : 50, and most preferably 1 : 2 to 1 : 20. The ratio may be, in some cases, 1 : 10 to 1 : 5 x $10^3$, 1 : 50 to 1 : 1 x $10^3$, or 1 : 100 to 1 : 500. In the present invention, the lower limit of the branching frequency of the branched glucan is preferably 0.2% or more, preferably 1% or more, more preferably 2% or more, and most preferably 5% or more. While there is no specific upper limit of the branching frequency, it can be, for example, 99% or less, 90% or less, 85% or less, 65% or less, 50% or less, or the like. The branching frequency is calculated by {(number of α-1,6-bonds) / (sum of α-1,4-bonds and α-1,6-bonds in glucan)} x 100.

The α-1,6-glucosidic bonds may be randomly distributed in the α-glucan or may be homogeneously distributed in the α-glucan. A distribution to such an extent that a linear chain part(s) of 5 or more saccharide units can be formed in the α-glucan is preferable.

In a particular embodiment, a branched glucan having a large number of non-reducing ends is preferable. The more the number of non-reducing ends in a branched glucan, the binding amount of a saccharide residue such as an N-acetylglucosamine residue, a galactose residue, a mannose residue, a glucuronic acid residue, a glucosamine residue, or a xylose residue per one molecule can be increased. The number of non-reducing ends of a branched α-1,4 glucan used in the present invention is specifically 2 or more, 3 or more, 5 or more, and is preferably about 10 or more, more preferably about 30 or more, and about 60 or more. The number of non-reducing ends of a branched α-glucan is preferably about 1 x $10^4$ or less, more preferably about 5 x $10^3$ or less. A branched glucan having a larger number of non-reducing ends is preferable. Branched CD is not preferable. Since the branched CD has one non-reducing end per one molecule and has a cyclic backbone consisting of an α-1,4 glucan having 6 to 8 glucose residues, and is amylase resistant, it is not preferable.

In the present invention, a modified product of the glucan may be used in place of the glucan. Examples of the modified product of the glucan include a modified starch and an esterified product of the glucan explained above. Furthermore, the modified product of the glucan may be a hydroxyl group-modified product or a reducing end-modified product. In addition, as described later, after at least one residue selected from N-acetylglucosamine residue and galactose residue is bound to at least one non-reducing end of the glucan, a glucan moiety may be modified.

The modified starch is a starch which was made to have a nature that it is easier to use by subjecting a natural starch to treatment such as hydrolysis, esterification or gelatinization. Wide variety of modified starches having a variety

of combinations of a gelatinization initiation temperature, a viscosity of a starch paste, a degree of transparency of a starch paste, stability against retrogradation and the like are available. There are various types of modified starches. An example of such a starch is a starch obtained by immersing starch granules in an acid at a gelatinization temperature or lower of the starch, thereby cutting a starch molecule but not destroying starch granules.

Examples of the modified product of the glucan other than the modified starch include a modified product in which at least one of alcoholic hydroxyl groups of an unmodified glucan is modified (hereinafter, in the present specification, referred to as a "hydroxyl group-modifiedproduct of glucan"), a modified product in which some of non-reducing ends of the glucan is modified (hereinafter, in the present specification, referred to as a " non-reducing end-modified product of glucan") and a modified product in which the reducing end of a glucan is modified (hereinafter, in the present specification, referred to as a "reducing end-modified product of glucan").

Examples of the modification at a hydroxyl group include hydroxyalkylation, alkylation, acylation, carboxymethylation, sulfation and phosphorylation. It is preferable that modification at a hydroxyl group is a modification which can be removed with an enzyme in a body. The hydroxyl group-modified product of the glucan is preferably an acylated glucan, and further preferably an acetylated glucan. The frequency of introduction of the modifying group(s) into alcoholic hydroxyl groups can be arbitrarily set at the time of a modification reaction of the glucan. The frequency of introduction of the modifying group (s) into alcoholic hydroxyl groups is expressed as DS, and DS1 means the state where one modifying group per glucose residue is introduced. DS can be calculated by DS = (number of modifying group)/(number of glucose residue). Since there is an OH group at the 2-position, the 3-position and the 6-position in an unmodified glucose residue, theoretically, maximum 3 modifying groups per glucose residue can be introduced. For this reason, the upper limit of DS is usually 3. The frequency of introduction of the modifying group(s) into alcoholic hydroxyl groups is about DS 0.01 or more, more preferably about DS 0.03 or more, further preferably about DS 0.05 or more, particularly preferably about DS 0.07 or more, and most preferably about DS 0.1 or more. The frequency of introduction of modifying group (s) is preferably about DS 1.5 or less, more preferably about DS 1.3 or less, further preferably about DS 1.1 or less, particularly preferably about DS 1.0 or less, and most preferably about DS 0.9 or less. By modifying the glucan, degradation of the glucan in blood or in a body is suppressed.

Examples of modification at a non-reducing end include binding with a targeting molecule such as a mannose residue, a molecule for drug binding such as a glucuronic acid residue and a glucosamine residue, and an antigen-presenting cell stimulating molecule such as a mannose residue and a xylose residue. Modification at a non-reducing end will be explained in detail in the following 2.6 and 3. Non-reducing end modification is preferably modification by binding of a mannose residue, a glucosamine residue, a glucuronic acid residue, or a xylose residue. Bybinding of a glucosamine residue, an amino group is imparted to a non-reducing end of a glucan. Since an amino group has a positive charge in an aqueous solution, binding by a non-covalent bond with a drug having a negative charge in an aqueous solution becomes possible. In addition, an amino group can also be utilized for binding via a covalent bond with a drug. In addition, by binding of a glucuronic acid residue, a carboxyl group is imparted to a non-reducing end of a glucan. Since a carboxyl group has a negative charge in an aqueous solution, binding by a non-covalent bond with a drug having a positive charge in an aqueous solution becomes possible. In addition, carboxyl group can also be utilized for binding via a covalent bond with a drug. In a particular embodiment, none of a glucosamine residue and a glucuronic acid residue is bound in the non-reducing end-modified product of the present invention.

In modification of a non-reducing end, it is preferable that a saccharide residue such as a mannose residue or a galactose residue, which activates an antigen-presenting cell such as a dendritic cell or a macrophage, is bound. It is preferable that at least two or more of an N-acetylglucosamine residue(s) and a galactose residue(s) are bound to a non-reducing end(s) of a glucan. Further, it is more preferable that two or more kinds of monosaccharide residues that are not only an N-acetylglucosamine residue or a galactose residue, but also include a mannose residue, a glucuronic acid residue, a glucosamine residue, or a xylose residue are bound.

Examples of modification at a reducing end include binding with a substance selected from the group consisting of a monosaccharide, a non-reducing carbohydrate, a biocompatible macromolecule, a liposome constituent component, a glycoside and an amine group-containing low-molecular weight substance. Modification at a non-reducing end will be explained in detail in the following 2.6 and 3 sections.

(1.2) Acetylglucosamine or galactose

Acetylglucosamine has the same meaning as N-acetylglucosamine, and is indicated by GlcNAc. Acetylglucosamine is a substance in which an OH group at a 2-position of glucose is substituted with a $NHCOCH_3$ group.

Galactose is an epimer of D-glucose, and is a substance in which configurations of an OH group and an H group at a 4-position of glucose are interchanged. Since galactose is recognized by an asialoglycoprotein receptor present on a hepatic parenchymal cell surface, it is particularly effective in the present invention.

In the method of the present invention, acetylglucosamine-1-phosphate or galactose-1-phosphate is used. Acetylglucosamine-1-phosphate or galactose-1-phosphate may be commercially available one, or may be synthesized

by a chemical method, an enzymatic method or a biological method such as fermentation. Acetylglucosamine or galactose maybe used for synthesizing acetylglucosamine-1-phosphate or galactose-1-phosphate.

(1.3) Other monosaccharides

In the present invention, monosaccharide-1-phosphates other than acetylglucosamine-1-phosphate or galactose-1-phosphate can be used. Examples of such a monosaccharide-1-phosphate include mannose-1-phosphate, glucuronic acid-1-phosphate, glucosamine-1-phosphate and xylose-1-phosphate . In order to obtain a glucan having the cell stimulating activity, among these monosaccharide-1-phosphates, particularly, mannose-1-phosphate, glucuronic acid-1-phosphate and xylose-1-phosphate are preferable. These monosaccharide-1-phosphates can be used in random combination with acetylglucosamine-1-phosphate and galactose-1-phosphate. As these monosaccharide-1-phosphates, commercially available ones may be used, or chemically synthesized or enzymatically synthesized ones according to the methods known in the art may be used.

(2. Method for producing a glucan in which at least one residue selected from N-acetylglucosamine residue and galactose residue is bound via an α-1,4 bond to non-reducing end)

(2.1) Acetylglucosamine-1-phosphate or galactose-1-phosphate

As acetylglucosamine-1-phosphate or galactose-1-phosphate utilized in the present invention, those synthesized by a chemical method, an enzymatic method, or a biological method such as fermentation can be used. As an example of a method of synthesizing acetylglucosamine-1-phosphate, for example, an enzymatic method is disclosed in Japanese Laid-Open Publication No. 2007-97517. As an example of a method of synthesizing galactose-1-phosphate, for example, an enzymatic method is disclosed in Manu R. M. De Groeve et al., Biotechnol. Lett. 2009, 31, 1873-1877.

As acetylglucosamine-1-phospate, either of acetylglucosamine-1-phospate in a non-salt form and acetylglucosamine-1-phospate in a salt form can be used. For example, a metal salt of acetylglucosamine-1-phospate can be used, and an alkali metal salt of acetylglucosamine-1-phospate (e.g. disodium acetylglucosamine-1-phosphate and dipotassium acetylglucosamine-1-phosphate) can be used.

As galactose-1-phospate, either of galactose-1-phospate in a non-salt form and galactose-1-phospate in a salt form can be used. For example, a metal salt of galactose-1-phospate can be used, and an alkali metal salt of galactose-1-phospate (e.g. disodium galactose-1-phospate and dipotassium galactose-1-phospate) can be used.

(2.2) α-Glucan phosphorylase

In the present specification, the term "α-glucan phosphorylase" means an enzymes having α-glucan phosphorylase activity. α-Glucan phosphorylase is classified in EC 2.4.1.1. α-Glucan phosphorylase activity refers to an activity catalyzing a reaction producing glucose-1-phosphate and partial degraded products of an α-1,4-glucan from inorganic phosphate and the α-1,4-glucan, or the reverse reaction thereof. α-Glucan phosphorylase can also catalyze an α-1,4-glucan synthesizing reaction which is the reverse reaction relative to phosphorolysis. In which direction a reaction proceeds depends on the amount of substrate.

In the present invention, any α-glucan phosphorylase can be used as long as it has a function to transfer a desired residue (e.g. an N-acetylglucosamine residue, when it is intended that an N-acetylglucosamine residue is bound to a glucan; or a galactose residue, when it is intended that a galactose residue is bound to a glucan) to a non-reducing end of glucan. α-glucan phosphorylase used in the present invention can be derived from a bacterium, a yeast, an animal or a plant, α-glucan phosphorylase of the present invention can be derived from, for example, potato, sweet potato, Fava *bean, Arabidopsis thaliana,* spinach, corn, rice, wheat, *Citrus hybrid cultivar, Aquifex aeolicus, Thermotoga maritima, Thermococcus zilligii, Thermoanaerobacter pseudethanolicus,* or the like.

It is preferable that α-glucan phosphorylase used in the present invention is α-glucan phosphorylase derived from *Aquifex aeolicus* VF5, potato-derived α-glucan phosphorylase or *Thermococcus zilligii* AN1-derived α-glucan phosphorylase, and more preferably it is α-glucan phosphorylase derived from *Aquifex aeolicus* VF5. Since α-glucan phosphorylase derived from *Aquifex aeolicus* VF5 have high transferring efficiency, it is quite preferable.

The base sequence of α-glucan phosphorylases derived from *Aquifex aeolicus* VF5 is set forth in SEQ ID NO: 1, and its amino acid sequence is set forth in positions 1-692 of SEQ ID NO: 2. The amino acid sequence of α-glucan phosphorylases derived from *Aquifex aeolicus* VF5 has about 21% to about 24 % sequence identitywith the amino acid sequence of plant α-glucan phosphorylases, about 34% sequence identity with the amino acid sequence of α-glucan phosphorylases derived from *Thermus thermophilus,* and about 38% sequence identity with the amino acid sequence of α-glucan phosphorylases derived from *Thermococcus litoralis.* It has about 38% sequence identity with the amino acid sequence of α-glucan phosphorylases derived from *Thermotoga maritima,* about 38% sequence identity with the

amino acid sequence of maltodextrin phosphorylases derived from *Thermococcus zilligii* AN1, and about 33% sequence identity with those of *Thermoanaerobacter pseudethanolicus.*

The base sequence of type L α-glucan phosphorylases derived from potato is set forth in SEQ ID NO: 3, and its amino acid sequence is set forth in positions 15-930 of SEQ ID NO: 4. The base sequence of α-glucan phosphorylases derived from *Thermococcus zilligii* AN1 is set forth in SEQ ID NO: 5, and its amino acid sequence is set forth in positions 1-717 of SEQ ID NO: 6.

In the present specification, an enzyme "derived from" an organism, means not only that the enzyme is directly isolated from the organism, but also refers to an enzyme obtained by utilizing the organism in any form. For example, when a gene encoding an enzyme obtained from an organism is introduced into *Escherichia coli,* and the enzyme is isolated from that *Escherichia coli,* the enzyme is referred to as being "derived from" the organism.

In the present specification, "identity" of a sequence (for example, an amino acid sequence, a base sequence and the like) refers to the degree of occurrence of the same amino acid (base when base sequences are compared) between two sequences. Identity can be generally determined by comparing two amino acid sequences or two base sequences, and comparing these two sequences which are aligned in an optimal format, which can contain additions or deletions.

In the present specification, the identity of sequences is calculated using maximum matching of GENETYX-WIN Ver.4.0 (Genetics Co., Ltd.). This program aligns sequence data to be analyzed, and sequence data to be compared so that amino acid pairs matched between sequences become greatest while substitution and deletion are considered, and thereupon, gives a score to each of Matches, Mismatches, and Gaps, calculates a sum, outputs alignment at the smallest sum, and calculates identity thereupon (Reference: Takeishi, K., and Gotoh, O. 1984. Sequence Relationships among Various 4.5 S RNA Species J. Biochem. 92:1173-1177).

For example, the amino acid sequence of α-glucan phosphorylases used in the present invention can be same with SEQ ID NO: 2, 4 or 6, i.e., it can have 100% identity. In another embodiment, as long as having activity to transfer a intended residue (i.e., an acetylglucosamine residue, when it is intended that an acetylglucosamine residue is transferred; or a galactose residue, when it is intended that a galactose residue is transferred) to non-reducing end of a glucan to form an α-1,4 bond, this amino acid sequence may be altered in up to a certain number of amino acids compared with a reference amino acid sequence. Such alterations can be selected from the group consisting of a deletion, a substitution (including conservative substitution and non-conservative substitution), or an insertion of at least 1 (for example, 1 or several) amino acids. This alteration may occur at a position of an amino terminus or a carboxyl terminus of the amino acid sequence of SEQ ID NO: 2, 4 or 6, or may occur at any position other than these termini. Alteration of an amino acid residue may be interspersed with one residue, or a few residues may be contiguous. For example, α-glucan phosphorylases used in the present invention may be added with amino acid residues (preferably about 20 or less residues, more preferably about 10 or less residues, and further preferably about 5 or less residues) at either terminus of the amino acid sequence of SEQ ID NO: 2, 4 or 6, for the reasons such as to make ease of purification of the enzyme, to increase stability, or the like.

The α-glucan phosphorylase used in the present invention has an amino acid sequence which has preferably about 50% or more, more preferably about 60% or more, further more preferably about 70% or more, still more preferably about 80% or more, particularly more preferably about 90% or more, and most preferably about 95% or more sequence identity with the amino acid sequence of SEQ ID NO: 2, 4 or 6 and has an activity transferring a intended residue (i.e., an acetylglucosamine residue, when it is intended that an acetylglucosamine residue is transferred; or a galactose residue, when it is intended that a galactose residue is transferred) to a non-reducing end of the glucan to form an α-1,4 bond. The α-glucan phosphorylase used in the present invention can have an amino acid sequence which has about 96 % or more, about 97% or more, about 98 % or more, or about 99 % more sequence identity with amino acid sequence of SEQ ID NO: 2, 4 or 6.

The amount of the α-glucan phosphorylase contained in a solution at the start of the reaction is preferably about 0.01 U/ml or more, more preferably about 0.1 U/ml or more, particularly preferably about 0.5 U/ml or more, and most preferably about 1 U/ml or more. The amount of the α-glucan phosphorylase contained in a solution at the start of the reaction is preferably about 1,000 U/ml or less, more preferably about 100 U/ml or less, particularly preferably about 50 U/ml or less, and most preferably about 20 U/ml or less. If the weight of α-glucan phosphorylase is too large, it may became easy to aggregate the enzyme denatured during the reaction. If the amount used is too small, reaction itself occurred, but the yield of glucan may be lowered. It is noted that unit amount of α-glucan phosphorylase is defined as follows:

Regarding one unit of α-glucan phosphorylase, an α-glucan phosphorylase activity which produces 1 μmol inorganic phosphate (Pi) per one minute shall be one unit (U or Unit) . This measurement of α-glucan phosphorylase activity quantitates free inorganic phosphate (Pi) producedfromG-1-P. After 200 μl of a reaction solution (containing 12.5 mM G-1-P, 1% dextrin and an enzyme solution in a 100 mM acetate buffer (pH 6.0)) is incubated at 50°C for 15 minutes, 800 μl of a molybdenum regent (15 mM ammonium molybdate, 100 mM zinc acetate) is added, and this is stirred to stop the reaction. 200 μl of 568 mM ascorbic acid (pH 5.8) is added, followed by mixing. After incubation at 30°C for 15

minutes, an absorbance is measured at 850 nm using a spectrophotometer. An absorbance is measured similarly using inorganic phosphate having the known concentration, and a standard curve is produced. An absorbance value obtained for a sample is fitted to this standard curve, and the amount of inorganic phosphate in the sample is determined. Inorganic phosphate is quantitated as a phosphoric acid ion. The amount of glucose-1-phosphate is not quantitated.

(2.3 Production of α-glucan phosphorylase)

α-Glucan phosphorylase used in the present invention can be directly isolated from an organism producing α-glucan phosphorylase, such as the aforementioned organisms, present in the natural world. Alternatively, α-glucan phosphorylase used in the present invention may be isolated from a microorganism (for example, bacteria, fungi and the like) which has been genetically modified with a gene encoding α-glucan phosphorylase isolated from the aforementioned organism.

In a preferable embodiment, α-glucan phosphorylase derived from *Aquifex aeolicus* VF5 is produced by chemically synthesizing a gene fragment of SEQ ID NO: 1, constructing an expression vector containing this gene fragment, introducing this expression vector into a microorganism to make a recombinant microorganism, culturing this recombinant microorganism to produce α-glucan phosphorylase, and collecting produced α-glucan phosphorylase. α-glucan phosphorylase derived from other organism can also be produced as well. An enzymatic production method by gene recombination is well-known to those skilled in the art. A host microorganism used in the present invention includes a prokaryote and a eukaryote, and a mesophile is preferable. Examples of a particularly preferable microorganism include, but not limited to, *Escherichia coli.*

α-glucan phosphorylase having the amino acid sequence of positions 1-692 of SEQ ID NO: 2, positions 15-930 of SEQ ID NO: 4, or positions 1-717 of SEQ ID NO: 6, or an amino acid sequence having homology thereto and having an activity transferring an N-acetylglucosamine, galactose, mannose, glucuronic acid, glucosamine or xylose residue, and a polynucleotide encoding the α-glucan phosphorylase which are used in the present invention can be produced using conventional genetic engineering techniques.

(2.4) Production of a glucan containing acetylglucosamine residue(s) or galactose residue(s)

The glucan containing an acetylglucosamine residue(s) or galactose residue(s) of the present invention can be produced by a method including a step of allowing a reaction of a solution containing acetylglucosamine-1-phosphate or galactose-1-phosphate, aglucan, and α-glucan phosphorylase (for example, α-glucan phosphorylase derived from *Aquifex aeolicus* VF5, α-glucan phosphorylase derived from potato, or α-glucan phosphorylase derived from *Thermococcus zilligii* AN1) which can catalyze a reaction of transferring an acetylglucosamine residue or a galactose residue to a non-reducing end of a glucan. By using a glucan modified product in place of a glucan in this method, a modified product of the glucan containing acetylglucosamine residue(s) or galactoseresidue(s) canbeproduced. As an example, a method using a glucan will be explained below.

First, a reaction solution is prepared. The reaction solution can be prepared, for example, by adding acetylglucosamine-1-phosphate or galactose-1-phosphate, a glucan, and α-glucan phosphorylase to a suitable solvent. Either one of acetylglucosamine-1-phosphate and galactose-1-phosphate may be used, or both of acetylglucosamine-1-phosphateor galactose-1-phosphate may be used simultaneously. If necessary, any buffer and inorganic salts for the purpose of adjusting the pH, as far as an enzymatic reaction is not inhibited, may be added to this reaction solution. If necessary, glucose-1-phosphate which is an original substrate of α-glucan phosphorylase may be added to this reaction solution. In the case of a reaction where acetylglucosamine-1-phosphate or galactose-1-phosphate and glucose-1-phosphate are coexistent, a reaction of binding a glucose residue to a non-reducing end of a receptor glucan and a reaction of binding acetylglucosamine residue or galactose residue are simultaneously performed. If an acetylglucosamine residue or a galactose residue is bound to a non-reducing end of the glucan, α-glucan phosphorylase can further transfer a molecule to the non-reducing end of the acetylglucosamine residue or the galactose residue, however, the efficiency of the transfer is much lower than the efficiency of transferring to a glucose residue. However, when a glucose residue is bound to a non-reducing end of a glucan, α-glucan phosphorylase can further transfer a glucose residue or an acetylglucosamine residue or galactose residue to a non-reducing end of a resulting molecule. For this reason, when glucose-1-phosphate is coexistent, the chain length of the glucan can be extended efficiently. Therefore, the structure of the finally obtained non-reducing end-modified glucan is controlled by a ratio between added acetylglucosamine-1-phosphate or galactose-1-phosphate and added glucose-1-phosphate. If necessary, an enzyme selected from the group consisting of a debranching enzyme, a branching enzyme, 4-α-glucanotransferase and a glycogen debranching enzyme may be added to this reaction solution.

By changing the ratio, in a reaction solution, of the amount of acetylglucosamine-1-phosphate or galactose-1-phosphate and the number as a population of non-reducing ends of a glucan, frequency of introduction of acetylglucosamine residue or galactose residue can be controlled. That is, as increasing (number of molecule of glucan) x (amount

of acetylglucosamine-1-phosphate or galactose-1-phosphate relative to number of non-reducing ends in the molecule), the frequency of introduction of acetylglucosamine residue or galactose residue can be increased. In addition, also by regulating the amount of enzyme added or enzyme reaction time, the frequency of introduction of acetylglucosamine residue or galactose residue can be regulated.

When non-reducing end modification of a glucan is performed with two or more kinds of saccharide residues, a reaction may be performed by adding two or more kinds of saccharide-1-phospates, in one kind of them or mixed in the same reaction liquid. For example, desired saccharide-1-phosphate selected from N-acetylglucosamine-1-phosphate, galactose-1-phosphate, glucuronic acid-1-phosphate, glucosamine-1-phosphate, mannose-1-phosphate, and xylose-1-phosphate may be reacted one kind by one kind. Alternatively, two or more kinds of them may be reacted simultaneously. For example, when it is desired that a saccharide residue selected from a glucuronic acid residue, a glucosamine residue, a mannose residue and a xylose residue is further bound, glucuronic acid-1-phosphate, glucosamine-1-phosphate, mannose-1-phosphate, and xylose-1-phosphate are reacted in one kind of them or a combination of them, with a non-reducing end of a branched glucan.

Then, the reaction solution is reacted, if necessary, by heating by a method known in the art. The reaction temperature can be any temperature, as far as the effect of the present invention is obtained. The reaction temperature can be representatively a temperature of about 30°C to about 90°C. It is preferable that the temperature of a solution in this reaction step is such a temperature that, after a predetermined reaction time, about 50% or more, more preferably about 80% or more activity of the activity of the α-glucan phosphorylase contained in this solution before the reaction remains. The reaction temperature is preferably about 35°C to about 80°C, more preferably about 35°C to about 70°C, and further preferably about 35°C to about 65°C. α-Glucan phosphorylase derived from *Aquifex aeolicus* VF5 is a thermostable enzyme, and its optimal reaction temperature is about 80°C to 90°C. From the viewpoint of the reaction speed, it is preferable that the reaction temperature is high to some extent. On the other hand, from the viewpoint of the optimal reaction temperature of α-glucan phosphorylase derived from *Aquifex aeolicus* VF5, a reaction at about 80°C to 90°C is possible. However, from the viewpoint of stability of the resulting product, stability of acetylglucosamine-1-phosphate or galactose-1-phosphate and glucose-1-phosphate, and the like, it is preferable that the reaction temperature is slightly lower than the optimal reaction temperature of the α-glucan phosphorylase derived from *Aquifex aeolicus* VF5. The reaction temperature is preferably about 30°C or higher, more preferably about 35°C or higher, further preferably about 40°C or higher. In a particular embodiment, the reaction temperature may be about 45°C or higher or about 50°C or higher. The reaction temperature is preferably about 90°C or lower, more preferably about 80°C or lower, further preferably about 70°C or lower. In a particular embodiment, the reaction temperature may be about 65°C or lower or about 60°C or lower. In the case where an enzyme for which an optimum reaction temperature is lower such as potato-derived α-glucan phosphorylase is used, it is preferable that the reaction temperature is set lower than the case where *Aquifex aeolicus* VF5-derived α-glucan phosphorylase is used.

The reaction time can be set in any time period, in view of the reaction temperature and remaining activity of an enzyme. The reaction time is representatively about 1 hour to about 100 hours, more preferably about 1 hour to about 72 hours, further more preferably about 2 hours to about 36 hours, and most preferably about 2 hours to about 24 hours. In a particular embodiment, the reaction time may be, for example, about 1 hour or longer, about 2 hours or longer, about 5 hours or longer, about 10 hours or longer, about 12 hours or longer, or about 24 hours or longer. In a particular embodiment, the reaction time may be, for example, about 100 hours or shorter, about 72 hours or shorter, about 60 hours or shorter, about 48 hours or shorter, about 36 hours or shorter, or about 24 hours or shorter.

Heating may be performed using any means, but it is preferable that heating is performed with stirring so as to homogeneously transmit the heat to the whole solution. The solution is stirred by placing it into, for example, a reaction tank made of stainless steel, provided with a warm water jacket and a stirring device.

Furthermore, in the method of the present invention, at least one of acetylglucosamine-1-phosphate or galactose-1-phosphate,a glucan, and α-glucan phosphorylase may be further added to a reaction solution at a stage where the reaction has proceeded to some extent.

In this way, a solution containing a non-reducing end-modified glucan is produced.

After completion of the reaction, in the reaction solution, if necessary, an enzyme in the reaction solution can be inactivated by, for example, heating at 100°C for 10 minutes. After completion of the reaction, enzyme may be removed by making pH of the reaction solution to be acidic to denature the enzyme protein. Alternatively, a post step maybe performed without performing treatment of inactivating an enzyme. The reaction solution may be stored as it is, or maybe treated in order to isolate the produced non-reducing end-modified glucan.

After completion of the reaction, after the non-reducing end-modified glucan is purified, or before the non-reducing end-modified glucan is purified, a hydroxyl group-modified product of the non-reducing end-modified glucan may be produced by modifying at least one of alcoholic hydroxyl groups of a glucan moiety of the resulting non-reducing end-modified glucan. It is preferable that modification is performed after purification of the non-reducing end-modified glucan. Modification can be performed according to a method known in the art. Examples of modification include hydroxyalkylation, alkylation, acylation, carboxymethylation, sulfation, and phosphorylation. Acylation is preferable, and

acetylation is more preferable. By modifying a reducing end of the glucan after producing the glucan containing N-acetylglucosamine residue(s) or galactose residue(s) or a hydroxyl group-modified product of the glucan containing N-acetylglucosamine residue(s) or galactose residue(s), a reducing end-modified product of the glucan containing N-acetylglucosamine residue(s) or galactose residue(s) or a hydroxyl group-modified product of the glucan containing N-acetylglucosamine residue(s) or galactose residue(s) may be produced. Further, a non-reducing end of these glucan moiety to which neither N-acetylglucosamine residue nor galactose residue is bound, may be modified. Binding of an acetylglucosamine residue or galactose residue to the glucan, modification of a hydroxyl group, modification of a reducing end, and modification of some of non-reducing ends with a modifying group other than an N-acetylglucosamine residue or galactose residue may be performed in any order.

(2.5 Purification of a glucan in which at least one residue selected from an N-acetylglucosamine residue or an galactose residue is liked via α-1,4 bond to the non-reducing end(s))

<Purification method>

The produced non-reducing end-modified glucan (or a modified product thereof) can be purified as necessary. Examples of the impurities removed by purification include inorganic phosphate, N-acetylglucosamine-1-phosphate or galactose-1-phosphate, inorganic salts and the like. Examples of a method of purifying a glucan include a method using an organic solvent (T. J. Schoch et al., J. American Chemical Society, 64, 2957 (1942)) and a method not using an organic solvent.

Examples of the organic solvent which can be used in purification using the organic solvent include acetone, n-amylalcohol, pentazole, n-propylalcohol, n-hexylalcohol, 2-ethyl-1-butanol, 2-ethyl-1-hexanol, lauryl alcohol, cyclohexanol, n-butyl alcohol, 3-pentanol, 4-methyl-2-pentanol, d,1-borneol, α-terpineol, isobutyl alcohol, sec-butyl alcohol, 2-methyl-1-butanol, isoamyl alcohol, tert-amyl alcohol, menthol, methanol, ethanol and ether.

As an example of the purification method not using an organic solvent, there is a method of removing inorganic phosphate, acetylglucosamine-1-phosphate or galactose-1-phosphate, and inorganic salts by subjecting a non-reducing end-modified glucan to membrane fractionation using an ultrafiltration membrane or chromatography, without precipitating the non-reducing end-modified glucan dissolved in water, after the non-reducing end-modified glucan production reaction.

Examples of the ultrafiltration membrane which can be used in purification include an ultrafiltration membrane of a molecular weight cut off of about $1 \times 10^3$ to about $1 \times 10^4$, preferably about $5 \times 10^3$ to about $5 \times 10^4$, more preferably about $1 \times 10^4$ to about $3 \times 10^4$ (UF membrane unit manufactured by DAICEL).

Examples of a support which can be used in chromatography include a support for gel filtration chromatography, a support for ligand exchange chromatography, a support for ion-exchange chromatography and a support for hydrophobic chromatography.

(2.6) Glucan in which at least one residue selected from N-acetylglucosamine residue and galactose residue is bound via α-1,4 bond to non-reducing end(s), and modified product thereof.

The glucan of the present invention is a glucan in which at least one residue selected from an N-acetylglucosamine residue and a galactose residue is bound via α-1,4 bond to each of at least one non-reducing end of a glucan. The glucan of the present invention is also referred to as the non-reducing end-modified glucan of the present invention. The glucan-modified product of the present invention can be a hydroxyl group-modified product of the non-reducing end-modified glucan, or a further non-reducing end-modified product or reducing end-modified product of the present invention.

The non-reducing end-modified glucan of the present invention is a glucan in which at least one residue selected from an N-acetylglucosamine residue and a galactose residue is bound via α-1,4 bond to each of at least one non-reducing end of a glucan. To an end of an N-acetylglucosamine residue or a galactose residue bound to a non-reducing end of this glucan may be further bound a saccharide or the like, if necessary. When two or more of an N-acetylglucosamine residue or a galactose residue, or a combination thereof are bound to one non-reducing end of the non-reducing end-modified glucan of the present invention, a structure is that these monosaccharides are bound and bind to one end. For example, the structure is that wherein one monosaccharide residue is bound to one non-reducing end of a glucan, and the next monosaccharide residue is further bound to the non-reducing end of the monosaccharide residue. In one embodiment, only one monosaccharide residue is bound to each end of non-reducing ends of a glucan.

In the non-reducing end-modified glucan of the present invention, N-acetylglucosamine residue(s) or galactose residue(s) or combination thereof may be bound to all of the non-reducing ends of glucan, or these monosaccharide residue(s) may be bound to only a part of the non-reducing ends. That is, some of the non-reducing ends can remain unbound by these monosaccharide residues.

Conversion ratio based on the number of non-reducing end(s), that is, the ratio of the number of bond (s) between monosaccharide(s) selected from N-acetylglucosamine residue(s) or galactose residue(s) or combination thereof and non-reducing end(s) relative to the number of non-reducing end (s) present before these monosaccharide (s) is/are bound, can be selected as appropriate depending on the application wherein the glucan containing either of these monosaccharide residues is used or the like, from the ratio close proximity of 0% (for example, about 1%) to 100%. For example, when it is desired that a small amount of N-acetylglucosamine residue(s) or galactose residue(s) be present, a low conversion ratio can be selected, and when it is desired that a large amount of N-acetylglucosamine residue(s) or galactose residue(s) be present, a high conversion ratio can be selected.

By appropriately adjusting the condition of the enzymatic reaction in producing a non-reducing end-modified glucan, for example, by adjusting the reaction time, the concentration of the substrate or the like of the enzymatic reaction, a non-reducing end-modified glucan having a selected conversion ratio can easily be obtained.

Further, the ratio of the total number of N-acetylglucosamine residue(s) or galactose residue (s) bound to non-reducing end(s) relative to the number of non-reducing end(s) present in a glucan before these monosaccharide residues(s) is/are bound can be used as an indicator for the amount of these monosaccharides. In the present specification, this ratio is described as binding ratio. In general, in analyzing a non-reducing end-modified glucan of the present invention, it is easier to measure the binding ratio than to measure the above-mentioned conversion ratio, therefore, it is advantageous from the practical viewpoint to design a non-reducing end-modified glucan based on binding ratio.

It is noted that depending on the condition of enzymatic reaction in synthesizing a non-reducing end-modified glucan, to the end of either of these monosaccharide residues bound to one non-reducing end, either of these monosaccharide residues may be further bound in some cases. That is, in some cases, the structure may be that wherein two these monosaccharides are serially bound to one non-reducing end (that is, a structure wherein a disaccharide is bound to a non-reducing end). A compound having such a structure can also be used as the non-reducing end-modified glucan of the present invention. The above-mentioned binding ratio has an advantage that the amount of N-acetylglucosamine residue(s) or galactose residue(s) can be evaluated even if the amount of N-acetylglucosamine residue(s) or galactose residue(s) is increased by binding a plurality of these monosaccharide residues to one non-reducing end as described above. For this reason, in contrast to the above-mentioned conversion ratio, the binding ratio may have a value greater than 100% in some cases. The binding ratio can be selected as appropriate depending on the application wherein the non-reducing end-modified glucan is used or the like. For example, when it is desired that a small amount of N-acetylglucosamine residue(s) or galactose residue(s) be present, a low binding ratio (for example, about 1% to about 2%) can be selected, and when it is desired that a large amount of N-acetylglucosamine residue(s) or galactose residue(s) be present, a high binding ratio (for example, bout 80% to about 120%) can be selected.

When the application of the non-reducing end-modified branched glucan is a nanoparticulate carrier for a DDS, a total of the binding ratios of the N-acetylglucosamine residues and galactose residues is preferably about 2% or more, more preferably about 5% or more, further preferably about 10% or more, and most preferably about 15% or more; the total of the binding ratios of the N-acetylglucosamine residues and the galactose residues is preferably about 200% or less, more preferably about 150% or less, further preferably about 120% or less, and most preferably about 100% or less.

When the application of the non-reducing end-modified branched glucan is an antigen-presenting cell stimulating molecule, the binding ratio of the N-acetylglucosamine residues or galactose residues is, in a total of them, preferably about 5% or more, more preferably about 10% or more, further preferably about 15% or more, and most preferably about 20% or more; the binding ratio of the N-acetylglucosamine residues or galactose residues is, in a total of them, preferably about 200% or less, more preferably about 150% or less, further preferably about 120% or less, and most preferably about 100% or less.

When the application of the non-reducing end-modified branched glucan is a DDS carrier, the number of bonds of the N-acetylglucosamine residues or the galactose residues per glucan molecule is, in a total of them, preferably about 2 or more, more preferably about 5 or more, further preferably about 10 or more, particularly preferably about 20 or more, and most preferably about 50 or more. The number of bonds of the N-acetylglucosamine residues or the galactose residues per glucan molecule is, in a total of them, preferably about 5000 or less, more preferably about 4000 or less, further preferably about 3000 or less, and most preferably about 2000 or less.

When the application of the non-reducing end-modified branched glucan is an antigen-presenting cell stimulating molecule, the number of bonds of the N-acetylglucosamine residues or the galactose residues per glucan molecule is, in a total of them, preferably about 2 or more, more preferably about 4 or more, further preferably about 8 or more, particularly preferably about 10 or more, and most preferably about 15 or more. The number of bonds of the N-acetylglucosamine residues or the galactose residues per glucan molecule is, in a total of them, preferably about 2000 or less, more preferably about 1000 or less, further preferably about 500 or less, and most preferably about 200 or less.

In the non-reducing end-modified glucan of the present invention, a sugar residue other than N-acetylglucosamine residue or galactose residue maybe bound to the non-reducing end to which neither N-acetylglucosamine residue nor galactose residue is bound. That is, some of non-reducing ends may bind with an N-acetylglucosamine residue or galactose residue, and the remaining non-reducing end(s) may bind with a sugar residue other than these

monosaccharides.

Alternatively, it is also possible tomake a structure wherein some of non-reducing ends bind to N-acetylglucosamine residue(s) or galactose residue(s), some bind to a sugar residue other than these monosaccharide, and the remaining non-reducing end(s) bind to nothing. In this regard, it is possible that among a plurality of non-reducing ends, the number of end (s) binding to N-acetylglucosamine residue (s) or galactose residue(s), the number of end(s) binding to a sugar residue other than these monosaccharides, and the number of end (s) not binding to a sugar be selected freely.

In a particular embodiment of the present invention, a saccharide residue bound to a non-reducing end is an N-acetylglucosamine residue, a galactose residue or a residue of an oligomer (e.g. a dimer) of one kind of them or a combination of them.

The hydroxyl group-modified product is as described above.

The non-reducing end-modified product will be explained. When a glucan moiety of the glucan in which at least one residue selected from N-acetylglucosamine residue and galactose residue is bound via α-1,4 bond to the non-reducing end, or a modified product thereof is a branched α-1,4-glucan, and there is a non-reducing end to which neither N-acetylglucosamine residue nor galactose residue is bound, other substances can be bound to a non-reducing end to which neither N-acetylglucosamine residue nor galactose residue is bound. In a preferable embodiment,"atargeting molecule", "a molecule for drug binding", or "a cell stimulating molecule" is bound to a non-reducing end to which no amino sugar residue is bound. In the present specification, the term "targeting molecule" refers to a molecule having tissue targeting function. Examples of a targeting molecule include mannose, xylose, fucose, galactosamine, an antibody, an antibody fragment, a receptor, a receptor fragment and a receptor ligand. Since mannose is recognized by a mannose receptor expressed on a variety of macrophages including a Kupffer cell and a sinusoid vascular endothelial cell of liver, it is effective. Mannose can be bound to a non-reducing end of the glucan containing acetylglucosamine residue(s) or galactose residue(s) or a modified product thereof, for example, by allowing an α-glucan phosphorylase to act to mannose-1-phosphate as a substrate. When bound by an enzymatic reaction, mannose is bound at the position 4 of a non-reducing terminal glucose residue to which neither N-acetylglucosamine residue nor galactose residue is bound. For example, it is also possible to combine mannose and galactose at any proportion and bind to a plurality of non-reducing ends of the branched glucan.

In the present specification, the term "molecule for drug binding" refers to a molecule having an anionic or cationic functional group having a function of binding with a medically effective ingredient. Examples of the molecule for drug binding include glucosamine, glucuronic acid, or a derivative thereof. In the case of a conjugate with a glucosamine residue, an amino group is imparted to a non-reducing end. Since an amino group has a positive charge in an aqueous solution, binding with a drug having a negative charge in an aqueous solution by a non-covalent bond becomes possible. In addition, an amino group can also be utilized for binding with a drug via a covalent bond. In addition, it becomes possible to chemically modify an amino group imparted to a non-reducing end of a glucan and introduce a hydrophobic group into a non-reducing end of a glucan. A glucan in which a hydrophobic group is thus introduced into a non-reducing end can be utilized for binding with a protein or a peptide by hydrophobic interaction. In addition, in the case of a conjugate with a glucuronic acid residue, a carboxyl group is imparted to a non-reducing end. Since a carboxyl group has a negative charge in an aqueous solution, binding with a drug having a positive charge in an aqueous solution by a non-covalent bond becomes possible. In addition, a carboxyl group can also be utilized for binding with a drug via a covalent bond. In addition, it also becomes possible to introduce a hydrophobic group into a non-reducing end of a glucan, by chemically modifying a carboxyl group imparted to a non-reducing end of a glucan. A glucan in which a hydrophobic group is thus introduced into a non-reducing end can be utilized for binding with a protein or a peptide by hydrophobic interaction.

In the present specification, the term "antigen-presenting cell stimulating molecule" refers to a molecule having the activity of stimulating a dendritic cell or a macrophage which is an antigen-presenting cell in an innate immune response. The antigen-presenting cell stimulating activity can be measured by a variety of methods. In the present invention, it is preferable that the antigen-presenting cell stimulating activity is measured by a production amount of interleukin-6 (IL-6). Examples of the antigen-presenting cell stimulating molecule include N-acetylglucosamine, galactose, mannose, glucuronic acid, and xylose. By binding of these saccharide residues to a plurality of non-reducing ends, the cell stimulating activity is further enhanced.

In modification of a non-reducing end, it is preferable that a saccharide residue such as a mannose residue or a galactose residue, which activates an antigen-presenting cell such as a dendritic cell or a macrophage, is bound. It is preferable that at least two or more residues selected from the group consisting of an N-acetylglucosamine residue and a galactose residue are bound to a non-reducing end of a glucan. Further, it is more preferable that at least two or more kinds of monosaccharide residues selected from the group consisting of not only an N-acetylglucosamine residue and a galactose residue, but also a mannose residue, a glucuronic acid residue, a glucosamine residue, or a xylose residue are bound.

In the present specification, the term "adjuvant" refers to a substance which is administered with an antigen, and is used for potentiating the immune response. In "Janeway's Immunobiology" (translated under supervision of Takehiko Sasazuki, Nankodo Co., Ltd.) (original book "Janeway's Immunobiology seventh edition Kenneth Murphy, Paul

Travers, Mark Walport 2008 Garlnd Science, Taylar & Francis Group, LLC"), it is described that a dendritic cell or a macrophage produces IL-6 in response to cell infection, and that produced IL-6 activates a lymphocyte and IL-6 potentiates production of an antibody which works in protection against infection. Therefore, those skilled in the art can understand that the immunological function can be potentiated if activation of a dendritic cell or a macrophage can be enhanced and production of IL-6 can be potentiated. As shown in Examples 20 and 22 to 26 of the present specification, the non-reducing end-modified glucan of the present invention could increase IL-6 production of a dendritic cell or a macrophage by contacting with a dendritic cell or a macrophage. For this reason, it is clear that the non-reducing end-modified glucan of the present invention can act as an adjuvant.

Immunostimulating action can be measured by a known method. The immunostimulating action has approximately the same meaning as the cell stimulating activity. As described above, IL-6 production is an indicator of the cell stimulating activity. For example, it can be measured using an action of inducing a cytokine having the immunostimulating action as an indicator. In Examples of the present specification, in order to investigate the immunostimulating action, an indicator of cell stimulation activation of a dendritic cell and a macrophage, which are involved in an immune response, was measured by production of IL-6 which is one of cytokines.

The reducing end-modified product will be explained. The "reducing end-modified product of the N-acetylglucosamine residue-containing glucan" refers to a substance in which another substance is bound to a reducing end present in the N-acetylglucosamine residue-containing glucan of the present invention. The "reducing end-modified product of the galactose residue-containing glucan" refers to a substance in which another substance is bound to a reducing end present in the galactose residue-containing glucan of the present invention. In a preferable embodiment, as a method of binding with a different substance at a reducing end, there are the following two methods. The first method is a method of binding a reducing end of a maltooligosaccharide having a degree of polymerization of 2or more, more preferably a degree of polymerization of 3 or more, further preferably a degree of polymerization of 4 or more to another substance by a known enzymatic procedure or a known chemical procedure and, thereafter, binding acetylglucosamine residue or galactose residue to a non-reducing end of the maltooligosaccharide using the method of the present invention. The second method is a method of binding a reducing end of the glucan containing acetylglucosamine residue (s) or galactose residue (s) of the present invention to another substance by a known enzymatic procedure.

A method of enzymatically binding a maltooligosaccharide to a different substance in the first method is disclosed, for example, in Japanese Laid-Open Publication No. 5-276883, Japanese Laid-Open Publication No. 07-241181 and International Publication No. WO01/073106.

The method of enzymatically binding a maltooligosaccharide to a different substance in the first method can be used for substances having an amine group. For example, as a method of chemically binding maltopentaose and a substance having an amine group, there are the following three kinds of methods:

(A) A method of binding reducing terminal aldehyde of maltopentaose and a substance having an amine group by reductive amination;

(B) A method of oxidizing reducing terminal aldehyde of maltopentaose into maltotetraosyl gluconic acid and, thereafter, dehydration-condensing this with a substance having an amine group with a condensing agent; and

(C) A method of oxidizing reducing terminal aldehyde of maltopentaose into maltotetraosyl gluconic acid and, thereafter, dehydrating this to prepare maltotetraosyl gluconolactone, and heating this and a substance having an amine group under an anhydrous solvent condition to allow binding. The three kinds of methods (A), (B) and (C) are described in detail in Japanese Patent Application No. 2008-121693. Furthermore, a glucan having high-molecular weight can be bound to a different substance similarly to these methods.

An enzyme utilizable in the second method that is a method of binding a reducing end of the glucan containing acetylglucosamine residue (s) or galactose residue (s) of the present invention to a different substance by an enzymatic procedure can be applied only to carbohydrates and glycosides. As the enzyme, a so-called glucan chain transferring enzyme such as a branching enzyme, CGTase, a D enzyme, amylomaltase or the like is used. These enzymes cut an $\alpha$-1,4 bond in the glucan containing acetylglucosamine residue (s) or galactose residue(s), and transfer a fragment on its non-reducing end side (fragment containing acetylglucosamine residue(s) or galactose residue(s)) to a receptor molecule (herein, a carbohydrate or a glycoside).

Examples of a substance to be bound include monosaccharides, non-reducing carbohydrates, biocompatible macromolecules, liposome constituent components, glycosides, and amine group-containing low-molecular weight substances.

Examples of the monosaccharides include monosaccharides having a functional group, such as glucosamine, glucuronic acid and gluconic acid. Particularly, glucosamine, glucuronic acid, or gluconic acid is preferable. In the case of a conjugate with a glucosamine residue, an amino group is imparted to a reducing end. Since an amino group has a positive charge in an aqueous solution, binding with a drug having a negative charge in an aqueous solution by a non-covalent bond becomes possible. In addition, an amino group can also be utilized for binding with a drug via a covalent

bond. In addition, in the case of a conjugate with a glucuronic acid residue or a gluconic acid residue, a carboxyl group is imparted to a reducing end. Since a carboxyl group has a negative charge in an aqueous solution, binding with a drug having a positive charge in an aqueous solution by a non-covalent bond becomes possible. In addition, a carboxyl group can also be utilized for binding with a drug via a covalent bond. In addition, since a carboxyl group imparted to a reducing end of a glucan can be chemically modified to introduce a cationic group or a hydrophobic group, for example, a carboxyl group can also be utilized for binding with a protein via a non-covalent bond by hydrophobic interaction. Examples of reducing carbohydrates include sorbitol, sucrose, trehalose, dextrin, and amylose. Examples of biocompatible macromolecules include starches, cellulose, chitin, chitosan, dextran, proteins and peptides. Examples of liposome constituent components include phospholipids, fatty acids, ceramides and surfactants. Examples of glycosides include ascorbic acid glucoside, hydroquinone glucoside, hesperidin glucoside, rutin glucoside, para-nitrophenylmaltopentaose, dodecylmaltose, flavonoid glycosides, terpene glycosides, phenol glycosides, chalcone glycosides and steroid glycosides. Examples of the amine group-containing low-molecular weight substances include various amino acids, dodecylamine, ethylenediamine, and diethylaminoethylamine, and the like.

In another embodiment, a reducing end-modified product of a glucan in which at least one residue selected from an N-acetylglucosamine residue and a galactose residue is bound via α-1,4 bond to a non-reducing end (non-reducing end-modified glucan) refers to a product obtained by oxidizing a reducing end aldehyde of a glucan. Oxidation of the reducing end aldehyde is performed by a known chemical procedure such as periodate oxidation. By the oxidation, an aldehyde group is converted into a carboxyl group. An example of oxidation of a reducing end of a molecule in which an acetylglucosamine residue or a galactose residue is bound to a non-reducing end of a linear glucan is shown in the following formula:

[Chemical formula 1]

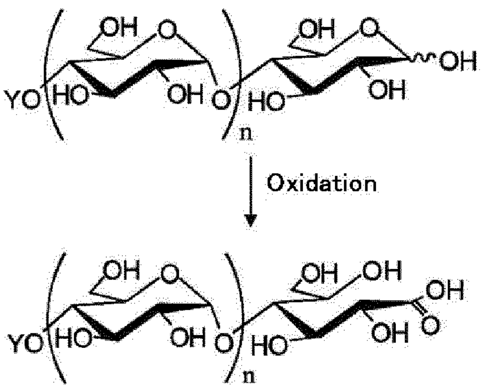


Y=

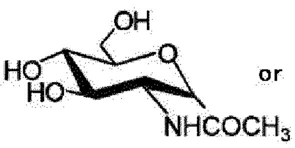

or

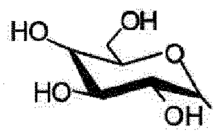

wherein n is any integer of 1 or more.

(3. Utilization of glucan in which at least one residue selected from N-acetylglucosamine residue and galactose residue is bound via α-1,4 bond to non-reducing end, and modified product thereof)

In the glucan in which at least one residue selected from an N-acetylglucosamine residue and a galactose residue is bound via α-1,4 bond to a non-reducing end of the present invention and a modified product thereof, at least one residue selected from an N-acetylglucosamine residue and a galactose residue is bound to a non-reducing end, and therefore they can be targeted a particular tissue.

When the non-reducing end-modified glucan of the present invention is utilized as the modifying material of the medically effective ingredient of medicaments, it is necessary to control degradation of the modifying material in a body. When the glucan is a linear α-1,4-glucan, it undergoes rapid degradation with α-amylase, and the time of existence in a body may be too short. In such a case, there is a possibility that an object as the modifying material cannot be attained. Then, by modifying some or all of hydroxyl groups of glucose constituting a glucan, the necessary time for degradation can be controlled. A hydroxyl group-modified product of such a glucan containing acetylglucosamine residue(s) or galactose residue (s) is preferable in the present invention. Modification is etherification or esterification. Etherification is preferably etherification with alkyl halide or an alcohol. The number of carbon atoms of alkyl halide or an alcohol used in etherification is preferably 1 to 10, more preferably 1 to 5, further preferably 1 to 3. The halogen group can be preferably fluoro, chloro, bromooriodo. Esterification is preferably esterification with carboxylic acid or acyl halide.

The number of carbon atoms of carboxylic acid or acyl halide used in esterification is preferably 1 to 10, more preferably 1 to 5. Modification is desirably esterification. Esterification is more preferably acylation, further preferably acetylation.

As described above, the non-reducing end-modified glucan and a modified product thereof of the present invention can be freely designed for their structure of the glucan and the structure of its non-reducing end and, by utilizing this, the pharmacokinetics of the medically effective ingredient of medicaments can be arbitrary controlled. This is the modifying material of the medically effective ingredient of medicaments, which has a structure completely degradable in a body, and can be safely utilized without anxiety for toxicity due to accumulation.

Therefore, according to the present invention, a medicament containing the non-reducing end-modified glucan or a modified product thereof of the present invention and the medically effective ingredient is provided. In the medicament of the present invention, the medically effective ingredient is preferably selected from the group consisting of a low-molecular weight organic compound, a protein, a peptide, an antibody, an antibody fragment, a receptor, a receptor fragment, a DNA, an RNA, a siRNA, an miRNA and an RNA aptamer.

According to a particular embodiment, the present invention provides a composition for clinical diagnosis, containing the non-reducing end-modified glucan, a hydroxyl group-modified product thereof, a reducing end-modified product thereof, or a carboxylic acid group-modified product of the present invention.

According to a particular embodiment, the present invention provides a nanoparticulate carrier for a DDS, containing the non-reducing end-modified glucan, a hydroxyl group-modified product thereof, or a reducing end-modified product thereof of the present invention is provided. This nanoparticulate carrier for a DDS is preferably selected from the group consisting of a liposome, a virus particle, a macromolecule micelle and a nanogel composed of macromolecule bearing hydrophobic groups.

(4. The structure possessed by glucan in which at least one residue selected from N-acetylglucosamine residue and galactose residue is bound via α-1,4-bound to non-reducing end and modified product thereof of the present invention)

In the present specification, a glucan in which at least one N-acetylglucosamine residue is bound via α-1,4-bound to each of at least one non-reducing end of a glucan, but no N-acetylglucosamine residue is present at the positions other than the non-reducing end of the glucan is referred to as an "N-acetylglucosamine-containing glucan". In the present specification, a glucan in which at least one galactose residue is bound to each of at least one non-reducing end of a glucan, but no galactose residue is present at the positions other than the non-reducing end of the glucan is referred to as a "galactose-containing glucan".

It is noted that when two or more N-acetylglucosamine residues are bound to one non-reducing end of a glucan, the structure is that wherein one N-acetylglucosamine residue is bound to one non-reducing end of the glucan and a next N-acetylglucosamine residue is bound to the end of the N-acetylglucosamine residue. In the present specification, a glucan in which at least one N-acetylglucosamine residue is bound via α-1,4 bond to each of at least one non-reducing end of a glucan, but no N-acetylglucosamine residue is present at a position other than a non-reducing end is referred to as "N-acetylglucosamine residue-containing glucan". These are similarly applied to a galactose residue.

In the present specification, even when a sugar chain consisting of two or more N-acetylglucosamine residues each bound via an α-1,4-bond is bound to a specific non-reducing end of a glucan and no N-acetylglucosamine residue is present at the position other than the non-reducing end of the glucan, it is considered that an N-acetylglucosamine residue is bound only to a non-reducing end and no N-acetylglucosamine residue is present at the position other than the non-reducing end.

In the present specification, also in the case where a saccharide chain consisting of two or more galactose residues which are respectively bound via an α-1,4 bond is bound to a particular non-reducing end of a glucan and no galactose residue is present at a position other than a non-reducing end in the glucan, it is deemed that a galactose residue is bound to only a non-reducing end, and no galactose residue is present at a position other than a non-reducing end.

The non-reducing end-modified glucan of the present invention is a non-reducing end-modified glucan in which at least one residue selected from an N-acetylglucosamine residue and a galactose residue is bound via α-1,4 bond to each of at least one non-reducing end of a glucan, but neither an N-acetylglucosamine residue nor a galactose residue is present at a position other than a non-reducing end of the glucan, wherein the glucan is a branched α-1,4 glucan or a linear α-1,4 glucan. The residue bound to each of the non-reducing ends can be independently selected from an acetylglucosamine residue and a galactose residue. For this reason, as a whole molecule, only an acetylglucosamine residue, a galactose residue, or an acetylglucosamine residue and a galactose residue may be bound to a non-reducing end. As described in another part in the present specification, a residue selected from a glucuronic acid residue, a glucosamine residue, a mannose residue, and a xylose residue may be further bound to a non-reducing end which has no acetylglucosamine residue or a galactose residue bound thereto, among non-reducing ends, and an acetylglucosamine residue or a galactose residue bound to a non-reducing end.

When a glucan moiety of a non-reducing end-modified glucan is a linear glucan, whereby the number of non-reducing ends is one, one N-acetylglucosamine residue or galactose residue is bound to the non-reducing end. When a glucan moiety of a non-reducing end-modified glucan is a branched glucan, whereby there are two or more non-reducing ends, at least one residue selected from an N-acetylglucosamine residue and a galactose residue is bound to one or more non-reducing ends among these. In the non-reducing end-modified glucan of the present invention, an N-acetylglucosamine residue or a galactose residue is not present at a position other than a non-reducing end. It is thought that absence of an N-acetylglucosamine residue or a galactose residue at a position other than a non-reducing end can be confirmed by, for example, treating a non-reducing end-modified glucan with an excessive amount of α-amylase and glucoamylase, or isoamylase. Glucoamylase is an enzyme which cuts α-1,4 bonds and α-1,6 bonds from a non-reducing end side, and when it acts on an unmodified branched glucan, the glucan is all degraded into glucose. A glucan in which at least one residue selected from an N-acetylglucosamine residue and a galactose residue is bound via α-1,4 bond to a non-reducing end exhibits resistance to glucoamylase. On the other hand, α-amylase acts on an α-1,4 glucan to produce maltose and glucose, but an α-1,4 glucan chain in which at least one residue selected from an N-acetylglucosamine residue and a galactose residue is α-1,4 bound exhibits resistance to α-amylase. A glucan containing an acetylglucosamine residue or a galactose residue only at a non-reducing end, produces N-acetylglucosaminylmaltose or galactosylmaltose and glucose, by treatment with an excessive amount of glucoamylase and α-amylase, and isoamylase. On the other hand, a glucan in which at least one residue selected from an N-acetylglucosamine residue and a galactose residue is bound via α-1,4 bond also to a place other than a non-reducing end produces a saccharide other than N-acetylglucosaminylmaltose or galactosylmaltose, and glucose, by treatment with an excessive amount of α-amylase and isoamylase. By this method, it can be confirmed that an N-acetylglucosamine residue or a galactose residue is not bound to a position other than a non-reducing end.

The glucan of the present invention, a modified product thereof and the like are described in the above 2.6. Herein, a structure which is thought to be possessed by the glucan which has an N-acetylglucosamine residue or a galactose residue bound thereto in accordance with the present invention will be described in the following.

(4.1) Branched glucan in which at least one residue selected from N-acetylglucosamine residue and galactose residue is bound via α-1,4 bond to non-reducing end, and modified product thereof

When a glucan moiety contained in the non-reducing end-modified glucan of the present invention is a branched α-1,4 glucan, in the non-reducing end-modified glucan, at least one residue selected from an N-acetylglucosamine residue and a galactose residue is bound via α-1,4 bond to at least one of a plurality of non-reducing ends possessed by the branched α-1,4 glucan. According to the present invention, a modified product of the non-reducing end-modified branched glucan is also provided. Modification of the modified product is as detailed above. A substance which is bound in a conjugate is also as detailed above.

A branched glucan moiety contained in the non-reducing end-modified branched glucan, and a modified product thereof of the present invention is a branched α-1,4-glucan having at least 2 or more non-reducing end, and is preferably selected from the group consisting of a branched maltooligosaccharide, a starch, amylopectin, glycogen, dextrin, an enzymatically synthesized branched glucan and highly branched cyclic glucan. A preferable range of these branched glucan moieties is as explained in "(1.1) Glucans and modified products of glucan".

The molecular weight of the branched α-1,4-glucan in which at least one residue selected from N-acetylglucosamine residue and galactose residue is bound via α-1,4-bound to at least 2 or more non-reducing ends of the present invention is preferably about 1 x $10^3$ or more, more preferably about 3 x $10^3$ or more, and further preferably about 5 x $10^3$ or more. The molecular weight of the branched glucan containing N-acetylglucosamine residue(s) or galactose residue (s) of the present invention is preferably about 1 x $10^9$ or less, more preferably about 3 x $10^8$ or less, and further preferably about 1 x $10^8$ or less.

The molecular weight of the modified product of the non-reducing end-modified branched α-1,4-glucan of the present invention is preferably about 1 x $10^3$ or more, more preferably about 3 x $10^3$ or more, and further preferably about 5 x $10^3$ or more. The molecular weight of the modified product of the non-reducing end-modified branched glucan of the present invention is preferably about 1 x $10^9$ or less, more preferably about 3 x $10^8$ or less, and further preferably about 1 x $10^8$ or less.

In the present invention, a modified product of the branched glucan in which at least one residue selected from N-acetylglucosamine residue and galactose residue bound via α-1,4-bond to non-reducing end is preferable. Modification is preferably acylation or etherification, and more preferably acetylation. The acylation degree is preferably about 0.1 or more, further preferably about 0.2 or more, and particularly preferably about 0.3 or more. The acylation degree is calculated by quantification of acetic acid released by heating under an alkali condition. The etherification degree is preferably about 0.1 or more, further preferably about 0.2 or more, and particularly preferably about 0.3 or more. The etherification degree is calculated by NMR.

The number of the acetylglucosamine residues or the galactose residues bound to the branched glucan and a modified product thereof of the present invention, in which at least one residue selected from N-acetylglucosamine

residue and galactose residue bound via α-1,4-bond to non-reducing end, is, respectively independently, preferably 1 or more, more preferably 2 or more, and further preferably 3 or more, per one molecule. The number of N-acetylglucosamine residue (s) or galactose residue(s) bound to the branched glucan or a modified product thereof of the present invention, in which at least one residue selected from N-acetylglucosamine residue and galactose residue bound via α-1,4-bond to non-reducing end is not limited to them, and for example, may be 5 or more, 10 or more, 15 or more, 20 or more, 50 or more, or 100 or more, or the like, per one molecule. The number can be suitably adjusted according to the purpose. The total of the upper limits of the number of acetylglucosamine residue or galactose residue bound to the branched glucan or a modified product thereof of the present invention, in which at least one residue selected from N-acetylglucosamine residue and galactose residue bound via α-1,4-bond to non-reducing end is the number of non-reducing ends of the branched glucan moiety. The number of N-acetylglucosamine residue or galactose residue bound to the branched glucan or a modified product thereof of the present invention, in which at least one residue selected from N-acetylglucosamine residue and galactose residue bound via α-1,4-bond to non-reducing end can be, respectively independently, for example, about 1,000 or less, about 800 or less, about 700 or less, about 600 or less, about 500 or less, about 400 or less, about 300 or less, about 200 or less, about 100 or less, about 50 or less, or the like, per one molecule.

In a particular embodiment, the total number of N-acetylglucosamine residue or galactose residue bound to the branched glucan in which at least one residue selected from N-acetylglucosamine residue and galactose residue bound via α-1,4-bond to non-reducing end and a modified product thereof of the present invention is preferably about 10% or more, more preferably about 20% or more, particularly preferably about 30% or more and, for example, can be about 40% or more, about 50% or more or about 60% or more of the number of non-reducing ends possessed by the branched glucan moiety. In a particular embodiment, the total number of N-acetylglucosamine residue or galactose residue bound to the branched glucan in which at least one residue selected from N-acetylglucosamine residue and galactose residue bound via α-1,4-bond to non-reducing end and a modified product thereof of the present invention is preferably about 100% or less, more preferably about 90% or less, particularly preferably about 80% or less and, for example, can be about 70% or less, about 60% or less, or about 50% or less of the number of non-reducing ends possessed by the branched glucan moiety.

(4.2) Linear glucan in which at least one residue selected from N-acetylglucosamine residue and galactose residue bound via α-1,4-bond to non-reducing end, and modified product thereof

In the non-reducing end-modified linear glucan of the present invention, at least one residue selected from an N-acetylglucosamine residue and a galactose residue is bound via α-1,4 bond to a non-reducing end of a linear glucan moiety. Since the linear glucan has only one non-reducing end, the non-reducing end-modified linear glucan of the present invention contains only one N-acetylglucosamine residue or galactose residue. According to the present invention, a modified product of the non-reducing end-modified linear glucan is also provided. Modification of the modified product is as detailed above.

In the non-reducing end-modified linear glucan of the present invention, at least one residue selected from an N-acetylglucosamine residue and a galactose residue is bound via α-1,4 bond to a non-reducing end of a linear glucan moiety. Since the linear glucan has only one non-reducing end, if an amount of N-acetylglucosamine-1-phosphate or galactose-1-phosphate to be added to a reaction liquid in a reaction of N-acetylglucosamine-1-phosphate or galactose-1-phosphate with a glucan is small, the non-reducing end-modified linear glucan of the present invention contains only one N-acetylglucosamine residue or galactose residue. When an amount of N-acetylglucosamine-1-phosphate or galactose-1-phosphate to be added to a reaction liquid in the reaction of N-acetylglucosamine-1-phosphate or galactose-1-phosphate with a glucan is large, two or more N-acetylglucosamine residues or galactose residues which are bound via α-1,4 bond may be bound to the non-reducing end of the linear glucan. According to the present invention, a modified product of the linear glucan in which at least one residue selected from an N-acetylglucosamine residue and a galactose residue is bound via α-1,4 bond is also provided. According to the present invention, a conjugate of the linear glucan in which at least one residue selected from an N-acetylglucosamine residue and a galactose residue is bound via α-1,4 bond, or a modified product thereof is also provided. Modification of the modified product is as detailed above. A substance bound to an amino sugar-containing glucan or a modified product thereof in a conjugate is also as detailed above.

The linear glucan moiety contained in the linear glucan or a modified product thereof of the present invention, in which at least one residue selected from N-acetylglucosamine residue and galactose residue bound via α-1,4-bond is preferably selected from the group consisting of a maltooligosaccharide, amylose (for example, natural amylose or enzymatically synthesized amylose) and a derivative thereof. A preferable range of these linear glucan moieties is as explained in " (1.1) Glucan and modified product of glucan".

The molecular weight of the non-reducing end-modified linear glucan of the present invention is preferably about 450 or more, more preferably about 600 or more, and further preferably about $1 \times 10^3$ or more. The molecular weight of the non-reducing end-modified linear glucan of the present invention is preferably about $2 \times 10^5$ or less, more

preferably about 1.5 x $10^5$ or less, and further preferably about 1 x $10^5$ or less.

The molecular weight of the modified product of the non-reducing end-modified linear glucan of the present invention is preferably about 450 or more, more preferably about 600 or more, further preferably about 1 x $10^3$ or more. The molecular weight of the modified product of the non-reducing end-modified linear glucan of the present invention is preferably about 2 x $10^5$ or less, more preferably about 1.5 x $10^5$ or less, further preferably about 1 x $10^5$ or less.

In the present invention, a modified product of the non-reducing end-modified linear glucan is preferable. Modificationispreferably acylationor etherification,more preferably acetylation. The acylation degree is preferably about 0.1 or more, further preferably about 0.2 or more, and particularly preferably about 0.3 or more. The acylation degree is calculated by quantification of acetic acid released by heating under an alkali condition. The etherification degree is preferably about 0.1 or more, further preferably about 0.2 or more, and particularly preferably about 0.3 or more. The etherification degree is calculated by NMR.

Examples of the non-reducing end-modified linear glucan of the present invention can be represented, for example, by the structure of the following formula 1:

[Chemical formula 2]

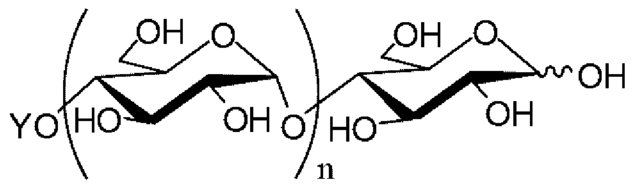

Formula 1

Y = 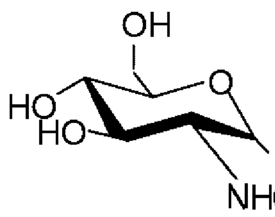 or 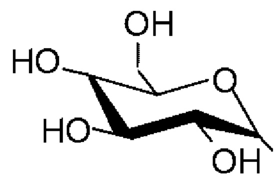

wherein n is an integer of 1 or more. n is preferably about 1 or more, more preferably about 2 or more, further preferably about 3 or more, still further preferably about 5 or more, particularly preferably about 8 or more, and most preferably about 10 or more. n is preferably about 1200 or less, more preferably about 900 or less, and further preferably about 600 or less.

Examples of the non-reducing end-modified linear glucan and the reducing end-modified product thereof of the present invention can be represented, for example, by the structure of the following formula 2:

[Chemical formula 3]

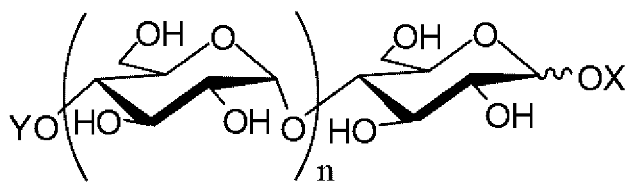

Formula 2

Y = 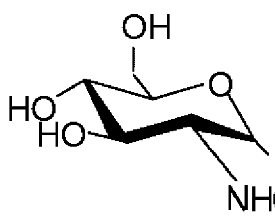 or 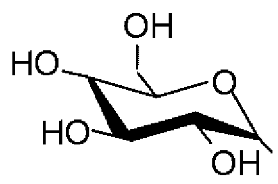

wherein n is an integer of 1 or more. n is preferably about 1 or more, more preferably about 2 or more, further preferably about 3 or more, still further preferably about 5 or more, particularly preferably about 8 or more, and most preferably about 10 or more. n is preferably about 1200 or less, more preferably about 900 or less, and further preferably about 600 or less. X is selected from the group consisting of hydrogen, monosaccharides, non-reducing carbohydrates, biocompatible macromolecules, liposome constituent components, glycosides, and amine group-containing low-molecular

weight substances. X is preferably selected from the group consisting of hydrogen, glucosamine, glucuronic acid, gluconic acid, sorbitol, sucrose, trehalose, dextrin, amylose, starches, cellulose, chitin, chitosan, dextran, proteins, peptides, phospholipids,fatty acids, ceramide, surfactants, ascorbic acid glucosides, hydroquinone glucosides, hesperidin glucosides, rutin glucosides, ceramide, para-nitrophenyl maltopentaose, dodecylmaltose, flavonoid glycosides, terpene glycosides, phenol glycosides, chalcone glycosides, steroid glycosides, amino acids, dodecylamine, ethylenediamine and dimethylaminoethylamine. X is more preferably selected from the group consisting of hydrogen, glucosamine, glucuronic acid, gluconic acid, dextrin and amylose. Particularly, hydrogen, glucosamine, glucuronic acid, gluconic acid and dextrin are preferable. Provided that when X is amine or the like, since the reducing end moiety of the glucan is cleaved, the reducing end-modified product of the present invention can be represented by the following:

[Chemical formula 4]

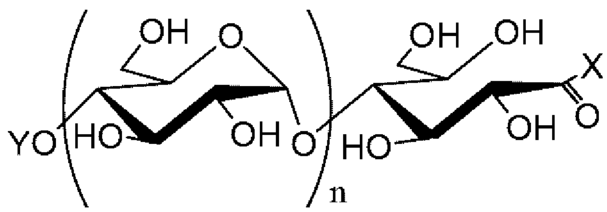

When X is hydrogen and Y is an N-acetylglucosamine residue, then this molecule is an N-acetylglucosamine-containing linear glucan, and when X is a substance other than hydrogen and Y is an N-acetylglucosamine residue, then this molecule is a reducing end-modified product of an N-acetylglucosamine-containing linear glucan. When X is hydrogen and Y is a galactose residue, then this molecule is a galactose-containing linear glucan, and when X is a substance other than hydrogen and Y is a galactose residue, then this molecule is a reducing end-modified product of a galactose-containing linear glucan.

An example of the non-reducing end-modified linear glucan, a hydroxyl group-modified product thereof and a reducing end-modified product thereof of the present invention can be represented by, for example, the structure of the following formula 3.

[Chemical formula 5]

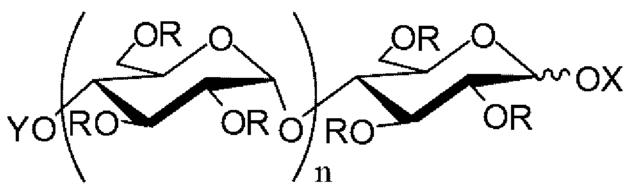

**Formula 3**

Y = 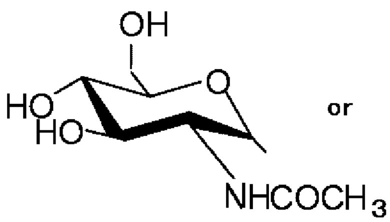 or 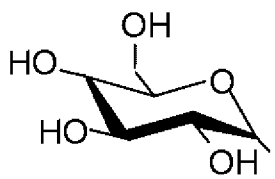

wherein n is an integer of 1 or more. n is preferably about 1 or more, more preferably about 2 or more, further preferably about 3 or more, still further preferably about 5 or more, particularly preferably about 8 or more, and most preferably about 10 or more. n is preferably about 1200 or less, more preferably about 900 or less, and further preferably about 600 or less. X is preferably selected from the group consisting of hydrogen, monosaccharides, non-reducing carbohydrates, biocompatible macromolecules, liposome constituent components, glycosides, and amine group-containing low-molecular weight substances. X is preferably selected from the group consisting of hydrogen, glucosamine, glucuronic acid, gluconic acid, sorbitol, sucrose, trehalose, dextrin, amylose, starches, cellulose, chitin, chitosan, dextran, proteins, peptides, phospholipids, fatty acids, ceramide, surfactants, ascorbic acid glucosides, hydroquinone glucosides, hesperidin glucosides, rutin glucosides, ceramide, para-nitrophenyl maltopentaose, dodecylmaltose, flavonoid glycosides, terpene glycosides, phenol glycosides, chalcone glycosides, steroid glycosides, amino acids and dodecylamine, ethylenediamine and dimethylaminoethylamine. X is more preferably selected from the group consisting of hydrogen, glucosamine, glucuronic acid, gluconic acid, dextrin and amylose. Particularly, hydrogen, glucosamine, glucuronic acid, gluconic acid and dextrin are preferable. Wherein R is preferably independently selected from the group consisting of hydrogen, hydroxyalkyl group, an alkyl group, an acetyl group, a carboxymethyl group, a sulfuric acid group and a phosphoric acid group. Provided that when X is amine or the like, since the reducing end moiety of the glucan is cleaved, the reducing end-modified product of the present invention can be represented by the following:

[Chemical formula 6]

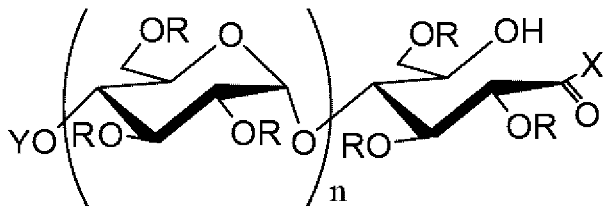

When X and all of R are hydrogen and Y is an N-acetylglucosamine residue, then this molecule is a N-acetylglucosamine-containing linear glucan; when X is hydrogen, each R is independently hydrogen or another group, provided that at least one of R is a group other than hydrogen and Y is an acetylglucosamine residue, then this molecule is a hydroxyl group-modified product of a N-acetylglucosamine-containing linear glucan; when X is a substance other than hydrogen, all of R is hydrogen, and Y is an N-acetylglucosamine residue, then this molecule is a reducing end-modified product of a N-acetylglucosamine-containing linear glucan; when X is a substance other than hydrogen, each R is independently hydrogen or another group, provided that at least one of R is a group other than hydrogen and Y is an N-acetylglucosamine residue, then this molecule is a reducing end-modified product of a hydroxyl group-modified product of a N-acetylglucosamine-containing linear glucan. When X and all of R are hydrogen and Y is a galactose residue, then this molecule is a galactose-containing linear glucan; when X is hydrogen, each R is independently hydrogen or another group, provided that at least one of R is a group other than hydrogen and Y is a galactose residue, then this molecule is a hydroxyl group-modified product of a galactose-containing linear glucan; when X is a substance other than hydrogen, all of R is hydrogen, and Y is a galactose residue, then this molecule is a reducing end-modified product of a galactose-containing linear glucan; when X is a substance other than hydrogen, each R is independently hydrogen or another group, provided that at least one of R is a group other than hydrogen and Y is a galactose residue, then this molecule is a reducing end-modified product of a hydroxyl group-modified product of a galactose-containing linear glucan.

Examples of the non-reducing end-modified glucan of the present invention can be represented, for example, by the following formula 5:

[Chemical formula 7]

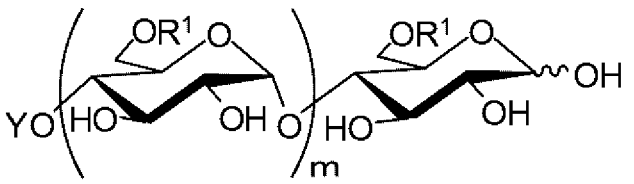 **Formula 5**

Y = 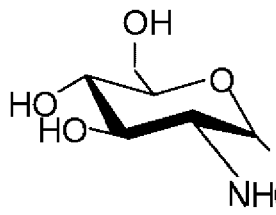 or 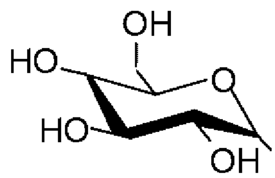

wherein m is an integer of 1 or more. m is preferably 1 or more, more preferably 2 or more, further preferably 3 or more, still further preferably about 5 or more, particularly preferably about 8 or more, and most preferably about 10 or more, and m is preferably about 1200 or less, more preferably about 900 or less, and further preferably about 600 or less. $R^1$ is independently H, a glucan chain having the structure of formula A or a glucan chain having the structure of formula B. When all of $R^1$ is H, the glucan shown by formula 5 is a linear glucan. When at least one of $R^1$ has the structure of formula A or formula B, the glucan shown by formula 5 is a branched glucan.

[Chemical formula 8]

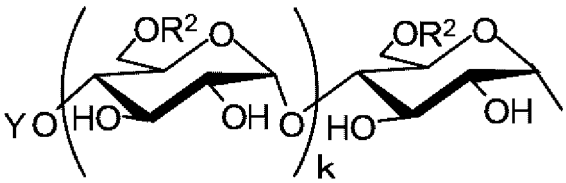

Formula A

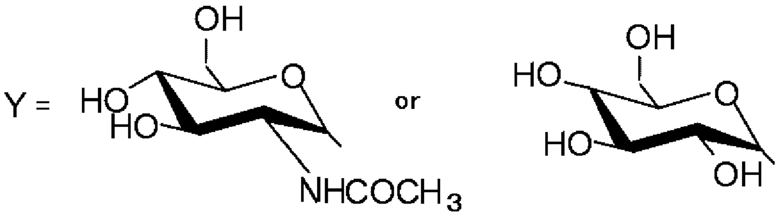

in formula A, k is an integer of 1 or more. k is preferably 1 or more, more preferably 2 or more, further preferably 3 or more, still further preferably about 5 or more, particularly preferably about 8 or more, and most preferably about 10 or more, and k is preferably about 100 or less, more preferably about 25 or less, and further preferably about 20 or less. In formula A, each $R^2$ is independently H, a glucan chain having the structure of formula A or a glucan chain having the structure of formula B.

[Chemical formula 9]

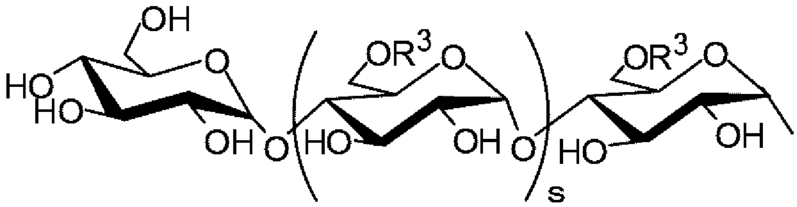

Formula B

In formula B, s is an integer of 1 or more. s is preferably 1 or more, more preferably 2 or more, further preferably 3 or more, still further preferably about 5 or more, particularly preferably about 8 or more, and most preferably about 10 or more, and s is preferably about 100 or less, more preferably about 25 or less, and further preferably about 20 or less. In f ormula B, $R^3$ is independently H, a glucan chain having the structure of formula A or a glucan chain having the structure of formula B.

As explained above, in formula 5, $R^1$ can have a structure in which the position of $R^2$ of a group having the structure of formula A or $R^3$ of a group having the structure of formula B is substituted with a group having the structure of formula A or a group having the structure of formula B several times. The total of times of substitutions with formula A and formula B is equal to the number of unit chains of the branched glucan molecule represented by formula 5. The number of unit chain of branched glucan molecule is preferably about 1 or more, more preferably about 10 or more, and further more preferably about 30 or more. The number of unit chain of branched glucan molecule is preferably about 5, 000 orless, more preferably about 2, 000 orless, and further more preferably about 1,000 or less.

Examples of the non-reducing end-modified glucan or the hydroxyl group-modified product thereof of the present invention can be represented, for example, by the structure of the following formula 6:

[Chemical formula 10]

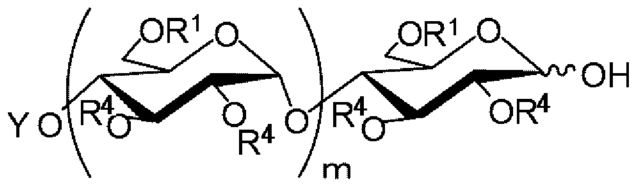

Formula 6

Y = 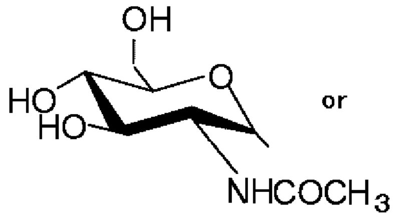 or 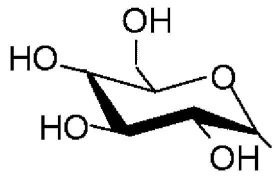

wherein m is an integer of 1 or more. m is preferably about 1 or more, more preferably about 2 or more, further preferably about 3 or more, still further preferably about 5 or more, particularly preferably about 8 or more, and most preferably about 10 or more, and m is preferably about 1200 or less, more preferably about 900 or less, and further preferably about 600 or less. $R^1$ is independently H, a hydroxyalkyl group, an alkyl group, an acetyl group, a carboxymethyl group, a sulfuric acid group, a phosphoric acid group, a glucan chain having the structure of formula 6A or a glucan chain having the structure of formula 6B.

[Chemical formula 11]

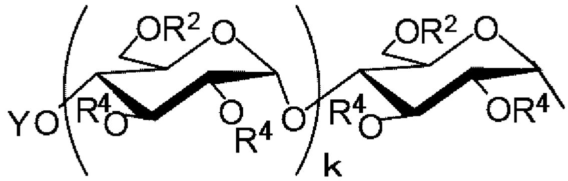

Formula 6A

Y = 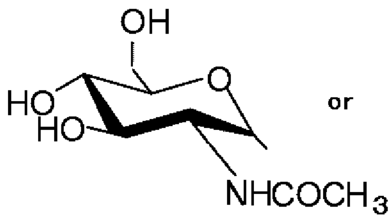 or 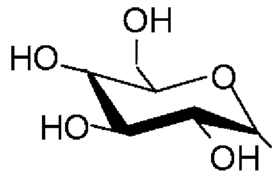

in formula 6A, k is an integer of 1 or more. k is preferably 1 or more, more preferably 2 or more, further preferably 3 or more, still further preferably about 5 or more, particularly preferably about 8 or more, and most preferably about 10 or more, and k is preferably about 100 or less, more preferably about 25 or less, and further preferably about 20 or less. In formula 6A, each $R^2$ is independently H, a hydroxyalkyl group, an alkyl group, an acetyl group, a carboxymethyl group, a sulfuric acid group, a phosphoric acid group, a glucan chain having the structure of formula 6A or a glucan chain having the structure of formula 6B.

[Chemical formula 12]

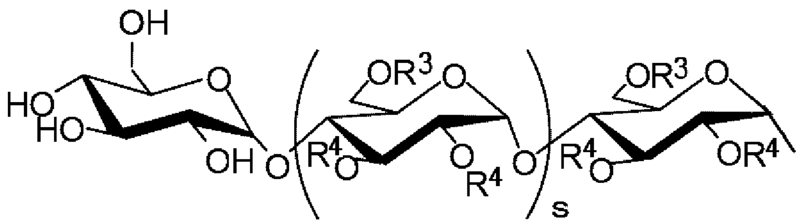

Formula 6B

in formula 6B, s is an integer of 1 or more. s is preferably about 1 or more, more preferably about 2 or more, further preferably about 3 or more, still further preferably about 5 or more, particularly preferably about 8 or more, and most preferably about 10 or more, and s is preferably about 100 or less, more preferably about 25 or less, and further preferably about 20 or less. In formula 6A, each $R^3$ is independently H, a hydroxyalkyl group, an alkyl group, an acetyl group, a carboxymethyl group, a sulfuric acid group and a phosphoric acid group, a glucan chain having the structure of formula 6A or a glucan chain having the structure of formula 6B.

In formula 6, formula 6A and formula 6B, each $R^4$ is independently selected from the group consisting of H, a hydroxyalkyl group, an alkyl group, an acetyl group, a carboxymethyl group, a sulfuric acid group and a phosphoric acid group.

Examples of the non-reducing end-modified glucan, a hydroxyl group-modified product thereof and a reducing end-modified product thereof of the present invention can be represented, for example, by the structure of the following formula 7:

[Chemical formula 13]

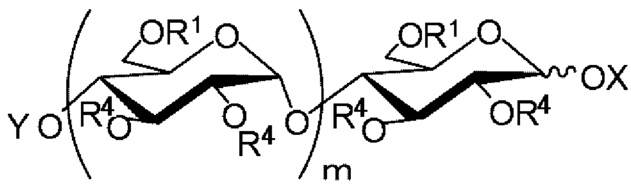

Formula 7

Y =

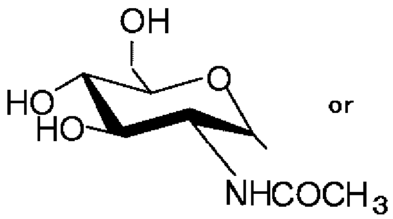

or

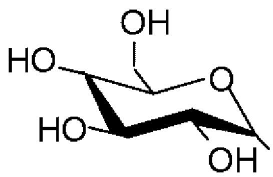

wherein m is an integer of 1 or more. m is preferably about 1 or more, more preferably about 2 or more, further preferably about 3 or more, still further preferably about 5 or more, particularly preferably about 8 or more, and most preferably about 10 or more, and m is preferably about 1200 or less, more preferably about 900 or less, and further preferably about 600 or less. $R^1$ is independently H, a glucan chain having the structure of formula 6A or a glucan chain having the structure of formula 6B. Formula 6A and formula 6B are same with the definition for the aforementioned formula 6.

In formula 7, formula 6A and formula 6B, $R^4$ is independently selected from the group consisting of hydrogen, a hydroxyalkyl group, an alkyl group, an acetyl group, a carboxymethyl group, a sulfuric acid group and a phosphoric acid group,

in formula 7, X is preferably independently selected from the group consisting of monosaccharides, non-reducing carbohydrates, biocompatible macromolecules, liposome constituent components, glycosides, and amine group-containing low-molecular weight substances. X is more preferably selected from the group consisting of hydrogen, glucosamine, glucuronic acid, gluconic acid, sorbitol, sucrose, trehalose, dextrin, amylose, starches, cellulose, chitin, chitosan, dextran, proteins, peptides, phospholipids, fatty acids, surfactants, ascorbic acid glucosides, hydroquinone glucosides, hesperidin glucosides, rutin glucosides, ceramide, para-nitrophenyl maltopentaose, dodecylmaltose, flavonoid glycosides, terpene glycosides, phenolglycosides, chalcone glycosides, steroid glycosides, amino acids and dodecylamine, ethylenediamine, and diethylaminoethylamine. X is more preferably selected from the group consisting of hydrogen, glucosamine, glucuronic acid, gluconic acid, dextrin and amylose. Particularly, hydrogen, glucosamine, glucuronic acid, gluconic acid and dextrin are preferable. Provided that when X is amine or the like, since the reducing end moiety of the glucan is cleaved, the reducing end-modified product of the present invention can be represented as follows:

[Chemical formula 14]

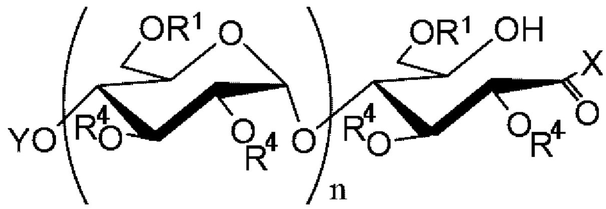

As described in another place in the present specification, in the non-reducing end-modified glucan of the present invention, at least one kind of residue of a glucuronic acid residue, a mannose residue and a xylose residue may be bound to at least one or more non-reducing end among a plurality of non-reducing ends thereof. For example, in one embodiment, in the case of a glucan of the aforementioned formula 5, 6 or 7, a residue denoted by Y is a residue selected from the group consisting of an N-acetylglucosamine residue, a galactose residue, a glucuronic acid residue, a mannose residue and a xylose residue, provided that at least one Y in a molecule is an N-acetylglucosamine residue or a galactose residue.

Examples of reducing end modification of the non-reducing end-modified glucan of the present invention and the purpose of the modification are summarized in the following Table 1. The structures in the table are merely examples.

[Table 1]

Table 1: Examples of modification and application of glucan containing acetylglucosamine residue or galactose residue

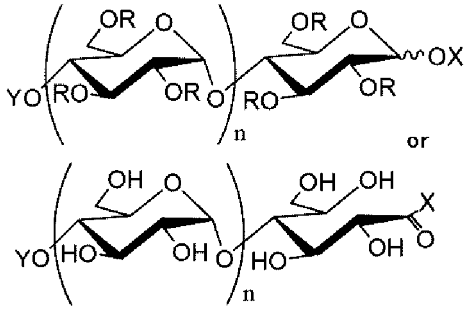

Y= 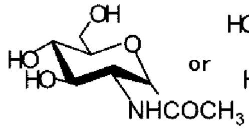 or 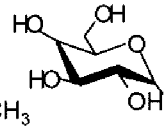

(GlcNAc) (Gal)

| Modification site | Specific example and structure | Application |
|---|---|---|
| Unmodified | (Y=GlcNAc or Gal, R=H) 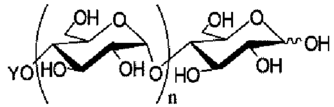 | - |
| Hydroxyl group | Substitution with acetyl group (Y=GlcNAc or Gal, R=Ac) 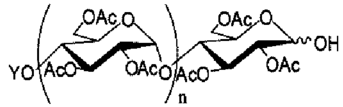 | ▪ Suppressing degradation with α-amylase, and improving blood retention property. |
| Reducing end (cationation) | Binding with glucosamine (GlcN) (Y=GlcNAc or Gal, R=H) 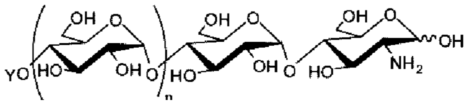 | ▪ Imparting a positive charge. ▪ Can be involved in electrostatic interaction with a medically effective ingredient (for example, nucleic acid, protein). ▪ Can be utilized in covalent bond with a medically effective ingredient. |
| | Binding with ethylenediamine (Y=GlcNAc or Gal, R=H) 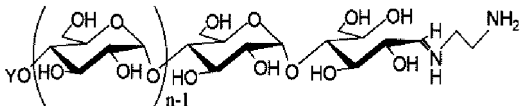 | |

(continued)

| Modification site | Specific example and structure | Application |
|---|---|---|
| Reducing end (anionation) | Binding with glucuronic acid (Y=GlcNAc or Gal, R=H) 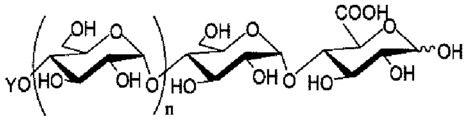 | ▪ Imparting a negative charge. Can be involved in electrostatic interaction with a medically effective ingredient (for example, protein). ▪ Can be utilized in covalent bond with a medically effective ingredient. |
| | Oxidation of terminal aldehyde (Y=GlcNAc or Gal, R=H) 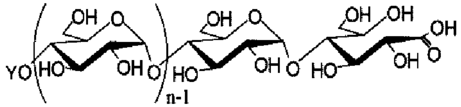 | |
| Reducing end (making to be hydrophobic) | Binding with dodecylamine (Y=GlcNAc or Gal, R=H) 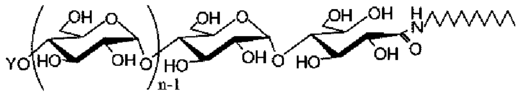 | ▪ Impartion of a hydrophobicity. ▪ Can be involved in hydrophobic interaction with a medically effective ingredient (for example, protein, hydrophobic small molecule). |

Table 1 exemplifies the case where at least one residue selected from an N-acetylglucosamine residue and a galactose residue is bound to a non-reducing end. As explained in another part in the present specification, an oligosaccharide residue in which two or more residues selected from an N-acetylglucosamine residue and a galactose residue are bound via $\alpha$-1, 4 bond may be bound to a non-reducing end. In addition, a glucose residue, a mannose residue, a glucuronic acid residue, a glucosamine residue, a xylose residue or an oligosaccharide residue constructed of them or the like may be bound to a 4-position of an N-acetylglucosamine residue or a galactose residue. In addition, for example, in the case of a branched glucan having a plurality of non-reducing ends, a plurality of N-acetylglucosamine residues or galactose residues may be bound to the position of Y shown in Table 1. In addition, one kind or two or more kinds of a glucose residue, a mannose residue, a glucuronic acid residue, a glucosamine residue, a xylose residue and an oligosaccharide residue constructed of them or the like other than an N-acetylglucosamine residue and a galactose residue may be bound.

Since the non-reducing end-modified glucan, a hydroxyl group-modified product thereof, a non-reducing end-modified product and a reducing end-modified product thereof of the present invention have biodegradability, they can be used safely in a living body. In the present specification, the term "biodegradability" refers to that a substance can be degraded in a body to a molecular weight at which a degradation product can be excreted to the outside of the body. The non-reducing end-modified glucan, a hydroxyl group-modified product thereof, a non-reducing end-modified product and a reducing end-modified product thereof of the present invention can be degraded to an oligosaccharide size by combination of amylases in a body.

The non-reducing end-modified glucan, a hydroxyl group-modified product thereof, a non-reducing end-modified product thereof and a reducing end-modified product thereof of the present invention are also biocompatible. In the present specification, the term "biocompatibility" refers to absence of toxicity and immunological rejection ability to a living body.

(Production of medicament)

In the non-reducing end-modified glucan, a hydroxyl group-modified product thereof, a non-reducing end-modified product thereof and a reducing end-modified product thereof of the present invention, an N-acetylglucosamine residue and a galactose residue at a non-reducing end have tissue targeting property and organ accumulating property. Therefore, they can be effectively utilized as a DDS carrier. By mixing them with a drug, or by binding them to a drug using a known method, they can be suitably utilized.

Particularly, a non-reducing end-modified product and a reducing end-modified product of a glucan in which at least one residue selected from an N-acetylglucosamine residue and a galactose residue is bound via $\alpha$-1,4 bond to a non-reducing end of the present invention (non-reducing end-modified glucan) can be utilized as an effective modifying material for a medically effective ingredient, which has two functions of the function of binding a medically effective ingredient and the tissue targeting function.

When a non-reducing end-modified product of the glucan in which at least one residue selected from an N-acetylglucosamine residue and a galactose residue is bound via $\alpha$-1,4 bond to a non-reducing end of the present invention (non-reducing end-modified glucan) contains, for example, a glucuronic acid residue, a carboxyl group is imparted to a non-reducing end. Since a carboxyl group has a negative charge in an aqueous solution, binding with a drug having a positive charge in an aqueous solution (for example, polypeptide (for example, protein, oligopeptide or the like), cationic polymer, cationic lipid or the like) by a non-covalentbond becomes possible. In addition, a carboxyl group can also be utilized by binding with a drug via a covalent bond. For example, since a carboxyl group imparted to a non-reducing end of a glucan can be chemically modified to introduce a cationic group or a hydrophobic group, for example, a carboxyl group can also be utilized by binding with a protein via a non-covalent bond by hydrophobic interaction. For example, when a glucosamine residue is contained, an amino group is imparted to a non-reducing end. Since an amino group has a positive charge in an aqueous solution, binding with a drug having a negative charge in an aqueous solution (e.g. nucleic acid molecule, anionic polymer, anionic lipid or the like) by a non-covalent bond becomes possible. In addition, an amino group can also be utilized by binding with a drug via a covalent bond.

When a reducing end-modified product of the non-reducing end-modified glucan of the present invention is, for example, a reducing end conjugate with ethylenediamine, an amino group is imparted to a reducing end. Since an amino group has a positive charge in an aqueous solution, binding with a drug having a negative charge in an aqueous solution by a non-covalent bond becomes possible. In addition, an amino group can also be utilized by binding with a drug via a covalent bond.

When a reducing end-modified product of the glucan containing an N-acetylglucosamine residue or a galactose residue of the present invention is, for example, an oxide of a reducing end, a carboxyl group is imparted to a reducing end. Since a carboxyl group has a negative charge in an aqueous solution, binding with a drug having a positive charge in an aqueous solution by a non-covalent bond becomes possible. In addition, a carboxyl group can also be utilized by binding with a drug via a covalent bond. In addition, when a carboxyl group imparted to a non-reducing end of a glucan

is chemically modified to introduce a cationic group or a hydrophobic group, for example, this can also be utilized by binding with a protein via a non-covalent bond by hydrophobic interaction.

In the non-reducing end-modified glucan, a hydroxyl group-modified product thereof, a non-reducing end-modified product thereof and a reducing end-modified product thereof of the present invention, an N-acetylglucosamine residue and a galactose residue at a non-reducing end have the activity of stimulating an antigen-presenting cell such as a dendritic cell or a macrophage. Therefore, they can be utilized as a vaccine adjuvant for a medicament. The glucan containing at least two of one kind or two or more kinds of glucuronic acid, glucosamine, mannose, N-acetylglucosamine, galactose and a xylose residue at a non-reducing end, a hydroxyl group-modified product thereof, a non-reducing end-modified product thereof and a reducing end-modified product thereof of the present invention have an increased activity of stimulating an antigen-presenting cell such as a dendritic cell or a macrophage. Therefore, they can be utilized as a vaccine adjuvant for a medicament.

According to the present invention, a composition for antigen-presenting cell stimulating activity, including the non-reducing end-modified glucan in which at least one residue selected from an N-acetylglucosamine residue and a galactose residue is bound via α-1,4 bond to a non-reducing end, a hydroxyl group-modified product thereof, a non-reducing end-modified product thereof and a reducing end-modified product thereof is also provided.

(Method of therapy)

The non-reducing end-modified glucan in which at least one residue selected from an N-acetylglucosamine residue and a galactose residue is bound via α-1,4 bond to a non-reducing end, a hydroxylgroup-modifiedproduct thereof, a non-reducing end-modified product thereof and a reducing end-modified product thereof of the present invention are used as a carrier of a medically effective ingredient. For this reason, a method of therapy including administering a mixture of the carrier and a medically effective ingredient to a subject is also provided. Combination of an objective disease and a medically effective ingredient, and an administration route, an administration frequency and a dose thereof, or the like can be appropriately determined by those skilled in the art.

According to the present invention, a cancer immunotherapy method, an inflammation therapy method or an immunostimulating method, including administering the non-reducing end-modified glucan, in which at least one residue selected from an N-acetylglucosamine residue and a galactose residue is bound via α-1,4 bond to a non-reducing end, a hydroxyl group-modified product thereof, a non-reducing end-modified product thereof and a reducing end-modified product thereof of the present invention, having the antigen-presenting cell stimulating activity to a subject, is also provided.

When a residue or a group capable of being charged negatively, such as a glucuronic acid residue, is bound to the non-reducing end-modified glucan of the present invention, the resulting glucan can be usefully used in antibody therapy, drug therapy and the like. In a particular embodiment of antibody therapy, the method of therapy of the present invention includes administering a complex of such the non-reducing end-modified glucan, a hydroxylgroup-modified product thereof, a non-reducing end-modified product thereof and a reducing end-modified product thereof of the present invention, and an antibody protein molecule to a subject.

When a residue or a group capable of being charged positively, such as a glucosamine residue, is bound to the non-reducing end-modified glucan of the present invention, the resulting glucan can be usefully used in gene therapy, drug therapy and the like. In a particular embodiment of gene therapy, the method of therapy of the present invention includes administering a complex of such the non-reducing end-modified glucan, a hydroxyl group-modified product thereof, a non-reducing end-modified product thereof and a reducing end-modified product thereof of the present invention, and a nucleic acid molecule to a subject.

In a particular embodiment, when a residue or a group capable of being charged positively such as a glucosamine residue is bound to the non-reducing end-modified glucan of the present invention, a method including administering a complex formed of a carrier complex of a glucan obtained and a nucleic acid molecule, and a cationic polymer or a cationic lipid to a subject, wherein the nucleic acid molecule includes a gene for therapy is also provided. In a particular embodiment of drug therapy, the method of therapy of the present invention includes administering a complex of such the glucan of the present invention with a medically effective ingredient to a subject.

A nucleic acid molecule including a gene encoding a protein for therapy appropriate for the therapy of an objective disease can also be produced using the conventional genetic engineering technology. The nucleic acid molecule may be an expression vector or a plasmid. A protein appropriate for the therapy of an objective disease, a gene encoding the protein, an expression vector or a plasmid used for preparing or delivering the gene, and an element contained in them are known in the art.

## Examples

The present invention will be explained below based on examples, but the present invention is not limited to

the examples. α-Glucan phosphorylase derived from *Aquifex aeolicus* VF5 used in the examples is *Aquifex aeolicus* VF5-derived α-glucan phosphorylase prepared by the following Production Example 1.

(Production Example 1: Preparation of *Aquifex aeolicus* VF5-derived α-glucan phosphorylase)

*Aquifex aeolicus* VF5-derived α-glucan phosphorylase was recombination-produced by the following method.

(A) Making of *Aquifex aeolicus* VF5-derived α-glucan phosphorylase gene

A nucleic acid (also referred to as an "α-glucan phosphorylase gene") having a base sequence (base sequence of 491380$^{th}$ to 493458$^{th}$ of ACCESSION No. AE000657 of GenBank base sequence database) encoding the amino acid sequence for *Aquifexaeolicus* VF5-derived α-glucan phosphorylase gene (the amino acid sequence described in SEQ ID NO: 2 of Sequence Listing; the amino acid sequence obtained by translating the base sequence of 491380$^{th}$ to 493458$^{th}$ (SEQ ID NO: 1) of ACCESSION No. AE000657 of GenBank base sequence database of National Center for Biotechnology Information (NCBI) in the USA) was chemically synthesized by a method well-known to those skilled in the art. An NdeI site was created upstream of a translation initiation codon of this α-glucan phosphorylase gene. In addition, a BamHI site was created downstream of a translation stop codon, and this synthetic gene was cut with NdeI and BamHI, and inserted into plasmid pET11c (manufactured by Novagen) which had been previously cut with NdeI and BamHI to make a plasmid pET-AqGP having an *Aquifexaeolicus* VF5-derived α-glucan phosphorylase gene.

(B) Expression of *Aquifex aeolicus* VF5-derived α-glucan phosphorylase gene in *Escherichia coli*

*Escherichia coli* BL21 (DE3) was transformed with this plasmid pET-AqGP according to a conventional method to obtain a transformant. A liquid containing the transformant was diluted and applied on an ampicillin-containing LB agar medium (100 μg/ml ampicillin, 1% tryptone manufactured by Difco, 0.5% yeast extract manufactured by Difco, 0.5% NaCl, 1.5% agar, pH 7.3) so that independent colonies were obtained, and this was cultured at 37°C overnight. *Escherichia coli* grown on this ampicillin-containing LB agar medium is a transformant which harbors an introduced plasmid, and can express the introduced plasmid. In such a way, *Escherichia coli* expressing an α-glucan phosphorylase gene was successfully made.

(C) Preparation of *Aquifex aeolicus* VF5-derived α-glucan phosphorylase enzyme

*Escherichia coli* expressing the *Aquifex aeolicus* VF5-derived α-glucan phosphorylase gene, made in the aforementioned (B), was inoculated with a LB medium (50 μg/ml ampicillin, 1% tryptone manufactured by Difco, 0.5% yeast extract manufacture by Difco, 0.5% NaCl, pH 7.3), and cultured at 37°C for 5 hours. Then, IPTG (isopropyl-β-D-thiogalactopyranoside) and pyridoxine hydrochloride were added to this culture so that the final concentration became 0.1 mM IPTG and 1 mM pyridoxine hydrochloride and, further, this was cultured at 37°C for 24 hours. Then, bacterial cells were collected by centrifugation of the culture, and washed with 20 mM citrate buffer (pH 6.7) to remove medium components. The bacterial cells after washing were suspended in 20 mM citrate buffer (pH 6.7), crushed with a sonicator, and centrifuged, and the supernatant was used as a bacterial cell extract. The resulting bacterial cell extract was heated at 60°C for 30 minutes. Then, this bacterial cell extract was loaded on a Q-Sepharose FF column which had been previously equilibrated, and this was eluted at a concentration gradient of from 0.1 M to 0.3 M NaCl, in 20 mM citrate buffer (pH 6.7), to collect a GP-purified enzyme-containing active fraction.

Using about 1 μg of the resulting purified enzyme-containing active fraction, native PAGE (Native polyacrylamide gel electrophoresis) was performed. As a result, in a fraction obtained from *Escherichia coli* expressing α-glucan phosphorylase, a single band was recognized at the position of the molecular weight of about 150 kDa and, at other places, no band was found. Since the molecular weight of the α-glucan phosphorylase derived from *Aquifex aeolicus* VF5 is predicted to be about 75 kDa as calculated from their amino acid sequence, as a result of this native PAGE, it is thought that this α-glucan phosphorylase takes a dimer structure. In this manner, it was demonstrated that the α-glucan phosphorylase derived from *Aquifex aeolicus* VF5 was homogeneously purified.

(Production Example 2: Production of branched glucan)

50g of a waxy corn starch (manufactured by SANWA CORNSTARCH CO., LTD) was suspended into 1,000 ml of 10 mM sodium phosphate buffer (pH 7.0), and the suspension was heated to about 100°C to gelatinize. 200, 000 units of a highly thermostable branching enzyme prepared according to the method described in Example 1 of Japanese Laid-Open Publication No. 2000-316581 was added to the starch paste which had been cooled to about 70°C to prepare a reaction solution, and then which was allowed to react at 70°C for 16 hours. After the reaction solution was heated at

100°C for 20 minutes, the supernatant after centrifuging it at 6, 500 rpm for 10 minutes was filtered with a membrane having a pore diameter of 0.8 μm. Then, the filtrate was desalted using a gel filtration chromatography (AKTA purifier) system (column: HiPrep™ 26/10 Desalting manufactured by GE Healthcare) to remove low-molecular weight polysaccharides. 1,000 ml of the filtrate was divided into 7.5 ml of aliquots, and applied to a gel filtration chromatography system, and elution fractions from 2.7 minutes to 3.7 minutes at a flow rate of 10 ml/min were fractionated, respectively. The elution fractions obtained from 1,000 ml of the filtrate were combined, the combined elution fractions was filtered with a membrane having a pore diameter of 0.2 μm, and then lyophilized to obtain about 35 g of a powder of a branched glucan. The weight average molecular weight of the branched glucan was investigated using a high performance liquid chromatography (HPLC) system (column: OHPAK SB-806MHQ, manufactured by SHOWA DENKO K.K.) equipped with a multiangle laser light scattering detector (DAWN DSP, manufactured by Wyatt Technology Corporation) and a differential refractometer (Shodex RI-71, manufactured by SHOWA DENKO K.K.). 20 mg of a powder of the branched glucan was dissolved in 10 ml of 100 mM aqueous sodium nitrate solution, and the solution was filtered with a membrane having a pore diameter of 0.45 μm to obtain a filtrate. 100 μl of the resulting filtrate was injected into the aforementioned HPLC system. It was shown that the weight average molecular weight of the branched glucan is about 140 K.

When isoamylase was allowed to act on the aforementioned branched glucan, and the reducing power was investigated by the modified Park-Johnson method (Hizukuri et al., Starch, Vol., 35, pp. 348-350, (1983)), it was shown that the unit chain length of branching was about 15 on average, and the number of branching was about 60 on average.

(Production Example 3: Recombination production of recombinant potato α-glucan phosphorylase)

Type L potato α-glucan phosphorylase was recombination-produced by the following method shown in Japanese National Phase PCT Laid-Open Publication No. 2004-526463.

BamHI site was created at the upstream and the downstream of a potato glucan phosphorylase gene (Nakano et al., Journal of Biochemistry 106: 691-695 (1989); SEQ ID NO: 3 encoding the amino acid sequence of SEQ ID NO: 4), this gene was cut with BamHI, and was incorporated into pET3d (manufactured by STRATAGENE) that had been previously cut with BamHI to obtain the plasmid pET-PGP113. In this plasmid, a glucan phosphorylase gene was operably linked under control of an isopropyl-β-D-thiogalactopyranoside (IPTG) inducible promoter. This plasmid was introduced into *Escherichia coli* TG-1 (manufactured by STRATAGENE) by the competent cell method. This *Escherichia coli* was plated on a plate containing LB medium (1% tryptone (manufactured by Difco), 0.5% yeast extract (manufactured by Difco), 1% sodium chloride, and 1.5% agar) containing the antibiotic ampicillin, and this was cultured at 37°C overnight. *Escherichia coli* grown on this plate was selected to obtain *Escherichia coli* in which a potato-derived glucan phosphorylase gene had been introduced. It was confirmed by analyzing the sequence of the introduced gene that the resulting *Escherichia coli* contains the glucan phosphorylase gene. In addition, it was confirmed by activity measurement that the resulting *Escherichia coli* expresses the glucan phosphorylase.

This *Escherichia coli* was inoculated in 1 liter of LB medium (1% tryptone (manufactured by Difco), 0.5% yeast extract (manufactured by Difco) and 1% sodium chloride) containing the antibiotic ampicillin, and this was cultured at 37°C for 3 hours with shaking at 120 rpm. Thereafter, IPTG was added to this medium to 0.1mM, and pyridoxine was added to this medium to 1mM, and this was cultured with shaking at 22°C for a further 20 hours. Then, this culture was centrifuged at 5,000 rpm for 5 minutes to collect the *Escherichia coli* cells. The resulting cells were suspended in 50 ml of 20 mM Tris-HCl buffer (pH 7.0) containing 0.05% Triton X-100 and, then, this was crushed by sonication to obtain 50 ml of a crushed cell liquid. This crushed liquid contained 4.7 U/mg glucan phosphorylase.

This crushed cell liquid is heated at 55°C for 30 minutes. After heating, it is centrifuged at 8, 500 rpm for 20 minutes to remove insoluble proteins and the like, to obtain the supernatant. The resulting supernatant was applied to an anion exchange resin Q-Sepharose which had been pre-equilibrated, allowing a glucan phosphorylase to be adsorbed onto the resin. The resin was washed with a buffer containing 200 mM sodium chloride to remove impurities. Subsequently, the protein was eluted with a buffer containing 300 mM sodium chloride, to obtain the recombinant potato glucan phosphorylase enzyme solution.

(Production Example 4: Preparation of microorganism-derived α-glucan phosphorylase)

Using a nucleic acid having a base sequence (base sequence of $1^{st}$ to $2151^{th}$ described in ACCESSION No. AJ318499 of GenBank base sequence database; SEQ ID NO: 5) encoding the amino acid sequence of *Thermococcus zilligii* AN1-derived α-glucan phosphorylase (SEQ ID NO: 6) in place of an *Aquifex aeolicus* VF5-derived α-glucan phosphorylase gene, α-glucan phosphorylase liquid was obtained in the same manner with the Production Example 1.

(Example 1: Confirmation of reaction specificity of each enzyme)

1 ml of a reaction liquid containing 10 mM maltopentaose (G5), 100 mM sodium acetate buffer (pH 5.5), 10

mM glucose-1-phosphate analogue shown in the following Table 2, and an enzyme (10 U) prepared in any of Production Example 1, 3 or 4 was allowed to act at 50 °C for 24 hours. When each enzyme catalyzes sugar transfer reaction from various glucose-1-phosphate analogues to a non-reducing end of G5, inorganic phosphate is produced. Then, the concentration of inorganic phosphate in the reaction liquid was measured, the binding ratio of a saccharide to G5 was calculated using the following equation, and the results are shown in Table 2. It is noted that since various reactions occurred as side reactions in this sugar transfer reaction, disproportionation of the length of a glucan chain occurred, and as a result, when maltopentaose having a degree of polymerization of 5 was used as a starting material, various saccharide residue-containing maltooligosaccharides having a degree of polymerization of a glucose residue of 3 or more were obtained, but for simplifying calculation, a binding ratio of a saccharide to G5 was calculated.

Binding ratio of saccharide to G5 = (concentration of inorganic phosphate produced in reaction liquid (mM) / concentration of G5 in reaction liquid (mM)) x 100 (%)

It is noted that the concentration of inorganic phosphate (mM) was obtained by mixing 800 μl of a molybdenum reagent (15 mM ammonium molybdate, 100 mM zinc acetate) into an aqueous solution (200 μl) containing inorganic phosphate, subsequently, adding 200 μl of a 568 mM aqueous ascorbic acid solution (pH 5.0), stirring the mixture, retaining the mixture at 30°C for 20 minutes, and measuring an absorbance at 850 nm using a spectrophotometer. An absorbance was similarly measured using inorganic phosphate having a known concentration to prepare a standard curve. By fitting an absorbance obtained in a sample to this standard curve, the concentration of inorganic phosphate (mM) in the reaction liquid was obtained.

[Table 2]

Table 2: Binding ratio of glucose-1-phosphate analogue to G5 using each enzyme (%)

| Glucose-1-phosphate analogue | Binding ratio (%) | | |
|---|---|---|---|
| | *Aquifex aeolicus*-derived α-GP obtained in Production Example 1 | Potato-derived α-GP obtained in Production Example 3 | *Thermococcus zilligii-derived* α-GP obtained in Production Example 4 |
| Glucose-1-phosphate | 100 | 71.3 | 74.2 |
| Glucuronic acid-1-phosphate | 64.6 | ND | ND |
| Glucosamine-1-phosphate | 68.8 | 53.5 | 45.5 |
| N-acetylglucosamine-1-phosphate | 53.4 | ND | 8.4 |
| Mannose-1-phosphate | 53.8 | 23.3 | 8.7 |
| Galactose-1-phosphate | 39.3 | 22.3 | 9.4 |
| Galactosamine-1-phosphate | ND | ND | ND |
| Galacturonic acid-1-phosphate | ND | ND | ND |
| Fucose-1-phosphate | ND | ND | NT |

In the table, ND indicates that the value was below the detection range, and NT indicates that the measurement was unimplemented.

As can be understood from Table 2, it was found that *Aquifex aeolicus* VF5-derived α-glucan phosphorylase acts on glucuronic acid-1-phosphate (GlcA-1-P), glucosamine-1-phosphate (GlcN-1-P), N-acetylglucosamine-1-phosphate (GlcNAc-1-P), mannose-1-phosphate (Man-1-P) and galactose-1-phosphate (Gal-1-P) in addition to glucose-1-phosphate which is an original substrate, and can catalyze a reaction of binding various saccharide residues to a non-reducing end of G5 (that is, these analogues can be utilized as a substrate). However, *Aquifex aeolicus* VF5-derived α-glucan phosphorylase could not utilize galactosamine-1-phosphate (GalN-1-P), galacturonic acid-1-phosphate (GalA-1-P) and fucose-1-phosphate (Fuc-1-P) as a substrate.

On the other hand, potato-derived α-glucan phosphorylase acted on GlcN-1-P, Man-1-P and Gal-1-P, and

could catalyze a reaction of binding various saccharides to a non-reducing end of G5, but could not act on GlcA-1-P, GlcNAc-1-P, GalN-1-P, GalA-1-P and Fuc-1-P. *Thermococcus zilligii* AN1-derived α-glucan phosphorylase acted on GlcN-1-P, and could catalyze a reaction of binding various saccharides to a non-reducing end of G5. *Thermococcus zilligii* AN1-derived α-glucan phosphorylase could act slightly on GlcNAc-1-P, GalN-1-P, and Man-1-P, but could not act on GlcA-1-P, GalN-1-P, and GalA-1-P. As described above, it is not the case that α-glucan phosphorylase can utilize any glucose-1-phosphate analogue other than glucose-1-phosphate which is an original substrate. It was revealed that what glucose-1-phosphate analogue can be utilized as a substrate is entirely different depending on an origin of an enzyme to be used.

(Example 2: Production of maltooligosaccharide which has N-acetylglucosamine residue bound thereto)

*Aquifexaeolicus* VF5-derived α-glucan phosphorylase (50 U) obtained in Production Example 1 was allowed to act on 5 ml of a reaction liquid containing 10 mM maltopentaose (G5), 10 mM N-acetylglucosamine-1-phosphate, and 100 mM sodium acetate buffer (pH 5.5) at 50°C for 24 hours. As a result, maltooligosaccharides which have an N-acetylglucosamine residue bound thereto were obtained. Since various reactions occurred as side reactions in this sugar transfer reaction, disproportionation of the length of a glucan chain occurred, and as a result, when maltopentaose having the degree of polymerization of 5 was used as a starting material, various maltooligosaccharides which has an N-acetylglucosamine residue bound thereto, in which the degree of polymerization of a glucose residue was 3 or more, were obtained.

The molar concentration of the produced maltooligosaccharides which have an N-acetylglucosamine residue bound thereto is equal to the molar concentration of inorganic phosphate in the reaction liquid. Then, by quantitating the molar concentration of inorganic phosphate in the reaction liquid, the molar concentration of the maltooligosaccharides which has an N-acetylglucosamine residue bound thereto was calculated. Based on this concentration, the amount of maltooligosaccharides which have an N-acetylglucosamine residue bound thereto was calculated. As a result, it was confirmed that 28.3 mg of maltooligosaccharides which have an N-acetylglucosamine residue bound thereto were produced in the aforementioned reaction liquid.

(Example 3: Production of maltooligosaccharide which has galactose residue bound thereto)

*Aquifexaeolicus* VF5-derived α-glucan phosphorylase (50 U) obtained in Production Example 1 was allowed to act on 5 ml of a reaction liquid containing 10 mM maltopentaose (G5), 10 mM galactose-1-phosphate, and 100 mM sodium acetate buffer (pH 5.5) at 50°C for 24 hours. As a result, maltooligosaccharides which have a galactose residue bound thereto were obtained. Since various reactions occurred as side reactions in this sugar transfer reaction, disproportionation of the length of a glucan chain occurred, and as a result, when maltopentaose having the degree of polymerization of 5 was used as a starting material, various maltooligosaccharides which have a galactose residue bound thereto, in which the degree of polymerization of a glucose residue was 3 or more, were obtained.

The molar concentration of the produced maltooligosaccharides which have a galactose residue bound thereto is equal to the molar concentration of inorganic phosphate in the reaction liquid. Then, by quantitating the molar concentration of inorganic phosphate in the reaction liquid, the molar concentration of the maltooligosaccharides which have a galactose residue bound thereto was calculated. Based on this concentration, an amount of the maltooligosaccharides which have a galactose residue bound thereto was calculated. As a result, it was confirmed that 19.8 mg of the maltooligosaccharides which have a galactose residue bound thereto was produced in the reaction liquid.

(Reference Example 4: Production of glucosamine-bound maltooligosaccharide)

*Aquifexaeolicus* VF5-derived α-glucan phosphorylase (50 U) obtained in Production Example 1 was allowed to act on 5 ml of a reaction liquid containing 10 mM maltopentaose (G5), 10 mM glucosamine-1-phosphate, and 100 mM sodium acetate buffer (pH 5.5) at 50°C for 24 hours. As a result, maltooligosaccharides which have a glucosamine residue bound thereto were obtained. Since various reactions occurred as side reactions in this sugar transfer reaction, disproportionation of the length of a glucan chain occurred, and as a result, when maltopentaose having the degree of polymerization of 5 was used as a starting material, various maltooligosaccharides which have a glucosamine residue bound thereto, in which the degree of polymerization of glucose residue was 3 or more, were obtained.

The molar concentration of the produced glucosamine-bound maltooligosaccharide is equal to the molar concentration of inorganic phosphate in the reaction liquid. Then, by quantitating the molar concentration of inorganic phosphate in the reaction liquid, the molar concentration of the maltooligosaccharides which have a glucosamine residue bound thereto was calculated. Based on this concentration, an amount of the maltooligosaccharides which have a galactose residue bound thereto was calculated. As a result, it was confirmed that 34.2 mg of the maltooligosaccharides which have a glucosamine residue bound thereto was produced in the aforementioned reaction liquid.

(Example 5: Production of branched glucan which has N-acetylglucosamine residue bound thereto)

*Aquifexaeolicus* VF5-derived α-glucan phosphorylase (50 U) obtained in Production Example 1 was allowed to act on 5 ml of a reaction liquid containing 110 mg of the branched glucan obtained in Production Example 2, 10 mM N-acetylglucosamine-1-phosphate, and 100 mM sodium acetate buffer (pH 5.5) at 50°C for 24 hours.

An amount of N-acetylglucosamine transferred to the branched glucan is equal to an amount of inorganic phosphate produced in the reaction liquid. Then, the concentration of inorganic phosphate in the reaction liquid was quantitated, and an amount of N-acetylglucosamine transferred to the branched glucan was obtained from the resulting concentration of inorganic phosphate. Assuming that N-acetylglucosamine was equally bound to the branched glucan in the reaction liquid, an amount of the branched glucan which has an N-acetylglucosamine residue bound thereto was obtained. In the aforementioned reaction liquid, 114.8 mg of the branched glucan which has an N-acetylglucosamine residue bound thereto was produced. It is noted that the binding ratio to a non-reducing end was 46.8%.

(Example 6: Production of branched glucan which has galactose residue bound thereto)

*Aquifexaeolicus* VF5-derived α-glucan phosphorylase (50 U) obtained in Production Example 1 was allowed to act on 5 ml of a reaction liquid containing 110 mg of the branched glucan obtained in Production Example 2, 10 mM galactose-1-phosphate, and 100 mM sodium acetate buffer (pH 5.5) at 50°C for 24 hours.

An amount of galactose transferred to the branched glucan is equal to an amount of inorganic phosphate produced in the reaction liquid. Then, the concentration of inorganic phosphate in the reaction liquid was quantitated, and from the resulting concentration of inorganic phosphate, an amount of galactose transferred to the branched glucan was obtained. Assuming that galactose was equally bound to the branched glucan in the reaction liquid, an amount of the branched glucan which has a galactose residue bound thereto was obtained. In the aforementioned reaction liquid, 113.1 mg of the branched glucan which has a galactose residue bound thereto was produced. It is noted that the binding ratio of non-reducing end was 38.5%.

(Reference Example 7: Production of branched glucan which has glucosamine residue bound thereto)

*Aquifexaeolicus* VF5-derived α-glucan phosphorylase (50 U) obtained in Production Example 1 was allowed to act on 5 ml of a reaction liquid containing 110 mg of the branched glucan obtained in Production Example 2, 10 mM glucosamine-1-phosphate, and 100 mM sodium acetate buffer (pH 5.5) at 50°C for 24 hours.

An amount of glucosamine transferred to the branched glucan is equal to an amount of inorganic phosphate produced in the reaction liquid. Then, the concentration of inorganic phosphate in the reaction liquid was quantitated, and from the resulting concentration of inorganic phosphate, an amount of glucosamine transferred to the branched glucan was obtained. Assuming that glucosamine is equally bound to the branched glucan in the reaction liquid, an amount of the branched glucan which has a glucosamine residue bound thereto was obtained. In the aforementioned reaction liquid, 115.4 mg of the branched glucan which has a glucosamine residue bound thereto was produced. It is noted that the binding ratio to a non-reducing end was 66.8%.

(Example 8: Production of reducing end-modified product of galactose-containing maltooligosaccharide)

To 10 ml of 5% maltopentaose (G5) was added 10 ml of a 0.2 M $I_2$/MeOH solution, and 4 ml of a 4% KOH/MeOH solution was slowly added dropwise in portions while stirring the liquid at 30°C, to perform an iodic acid oxidation reaction. A 4% KOH/MeOH solution was further added dropwise until the color of iodine disappeared, to perform a reaction for 1 hour while stirring the liquid. Methanol in an amount which is 3-hold of the volume of the resulting reaction liquid was added to precipitate G5 oxide, and the precipitate was collected by centrifugation. About 0.4 g of G5-reducing end-modified product in which a reducing end of G5 was carboxylated was obtained.

In this oxidation reaction, the following reaction occurred.

[Chemical formula 15]

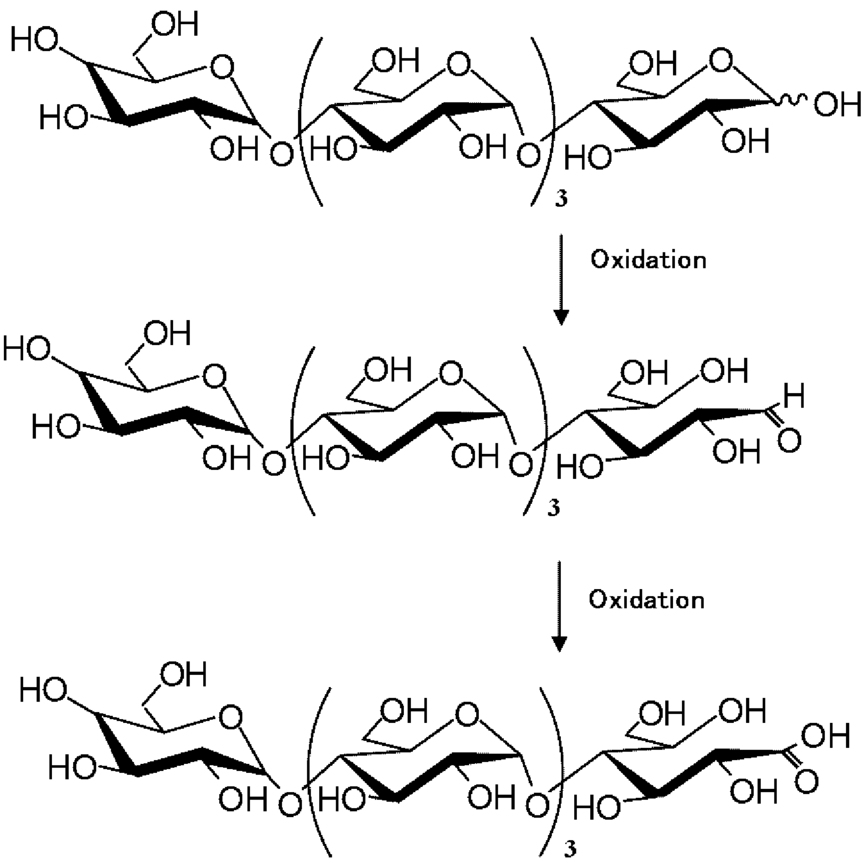


Next, *Aquifex aeolicus* VF5-derived α-glucan phosphorylase (50 U) obtained in Production Example 1 was allowed to act on 5 ml of a reaction liquid containing the 10 mM G5-reducing end-modified product obtained above, 10 mM galactose-1-phosphate, and 100 mM sodium acetate buffer (pH 5.5) at 50°C for 24 hours. As a result, reducing end-modified products of the maltooligosaccharides which have a galactose residue bound thereto were obtained. Since various reactions occurred as side reactions in this sugar transfer reaction, disproportionation of the length of a glucan chain occurred, and as a result, when maltopentaose having the degree of polymerization of 5 was used as a starting material, various reducing end-modified products of the maltooligosaccharides which have a galactose residue bound thereto, in which the degree of polymerization of a glucose residue was 3 or more, were obtained.

The molar concentration of the produced reducing end-modified product of the maltooligosaccharides which have a galactose residue bound thereto is equal to the molar concentration of inorganic phosphate in the reaction liquid. Then, by quantitating the molar concentration of inorganic phosphate in the reaction liquid, the molar concentration of the reducing end-modified products of the maltooligosaccharides which have a galactose residue bound thereto was calculated. Based on this concentration, an amount of the maltooligosaccharides which have a galactose residue bound thereto was calculated. As a result, it was confirmed that 20.1 mg of the reducing end-modified product of the maltooligosaccharide which have a galactose residue bound thereto were produced in the aforementioned reaction liquid.

(Example 9A: Production of galactose-containing branched glucan or N-acetylglucosamine-containing glucan which has gluconic acid bound to reducing end)

10 ml of 0.2 M $I_2$/MeOH solution was added to 10 ml of 5% maltopentaose (G5), and 4 ml of 4% KOH/MeOH solution was added dropwise slowly with stirring at 30 °C to conduct iodate oxidation reaction. A 4% KOH/MeOH solution was further added dropwise until the color of iodine disappeared, to perform a reaction for 1 hour while stirring the liquid. Methanol in an amount which is 3-fold of the volume of the resulting reaction liquid was added to precipitate G5 oxide, and the precipitate (about 0.4 g) was collected by centrifugation. The precipitate was maltohexaosyl gluconic acid in which reducing end glucose of maltopentaose is converted into gluconic acid. To a solution wherein 6 g of glucose-1-phosphate and 0.6 mg of maltohexaosyl gluconic acid were dissolved in 100 ml of 0.2 M maleate buffer (pH 6.0), 150 U of *Aquifex aeolicus* VF5-derived α-glucan phosphorylase produced in Production Example 1 and 2,000 U of highly thermostable branching enzyme prepared according to the method described in Example 1 of Japanese Laid-Open Publication No. 2000-316581 were added to prepare a reaction solution, and thereafter, the reaction was performed for

16 hours with stirring at 50°C. The enzyme was inactivated by heating the solution, and the inactivated enzyme was removed by filtering through a glass filter. Twice the amount of ethanol was added to the filtrate to precipitate the glucan reaction product and then centrifuged. The precipitate was washed twice with 300 milliliters of 1:1 water:ethanol solution to remove the coexisting glucose-1-P, and further washed twice with ethanol, and thereafter lyophilized. The branched glucan which has gluconic acid bound to the reducing end-modified having molecular weight of 120 kDa was obtained in yield of 1.7 g.

*Aquifex aeolicus* VF5-derived α-glucan phosphorylase (18 Units/ml) prepared in Production Example 1 was allowed to act on each reaction liquid containing 1.6 mg/mL of the aforementioned reducing end-modified branched glucan, 10 mM galactose-1-phosphate or N-acetylglucosamine-1-phosphate, and 100 mM sodium acetate buffer (pH 5.5) at 50°C for 20 hours to bind a galactose residue or an N-acetylglucosamine residue to a non-reducing end of a branched glucan which has gluconic acid bound to a reducing end. The binding ratio of galactose or N-acetylglucosamine was obtained by the same method as that of Example 3 and was 33% and 32%, respectively. In this manner, a branched glucan which is non-reducing end-modified with a galactose residue or an N-acetylglucosamine residue, in which a reducing end was modified, was obtained.

(Example 9: Production of branched glucan which has galactose and glucosamine bound thereto)

*Aquifexaeolicus* VF5-derived α-glucan phosphorylase (50 U) obtained in Production Example 1 was allowed to act on 5 ml of a reaction liquid containing 110 mg of the branched glucan obtained in Production Example 2, 10 mM galactose-1-phosphate, and 100 mM sodium acetate buffer (pH 5.5) at 50°C for 5 hours.

An amount of galactose transferred to the branched glucan is equal to an amount of inorganic phosphate produced in the reaction liquid. Then, the concentration of inorganic phosphate in the reaction liquid was quantitated, and from the resulting concentration of inorganic phosphate, an amount of galactose transferred to the branched glucan was obtained. Assuming that galactose was equally bound to the branched glucan in the reaction liquid, an amount of the branched glucan which has a galactose residue bound thereto was obtained. 111.7 mg of a galactose-bound branched glucan was produced. It is noted that the binding ratio of a galactose residue to a non-reducing end was 20.8%.

From a reaction liquid containing this branched glucan which has a galactose residue bound thereto, unreacted galactose-1-phophate and inorganic phosphate in the reaction liquid were removed using a PD-10 column (manufactured by GE Healthcare) according to the instructions of the manufacturer. *Aquifex aeolicus* VF5-derived α-glucan phosphorylase (50 U) obtained in Production Example 1 was allowed to act on 5 ml of a reaction liquid containing 110 mg of the resulting branched glucan which has a galactose residue bound to a non-reducing end, 10 mM glucosamine-1-phosphate, and 100 mM sodium acetate buffer (pH 5.5) at 50°C for 5 hours. An amount of glucosamine transferred to the branched glucan which has a galactose residue bound thereto was obtained by the same method as that of an amount of galactose transferred to the aforementioned branched glucan. 112.7 mg of the branched glucan which has galactose and a glucosamine residue bound thereto was produced. It is noted that the binding ratio of a glucosamine residue to a non-reducing end was 33.8%.

(Example 10: Evaluation of glucoamylase degradability of branched glucan which has various saccharide residues bound thereto)

After a reaction liquid containing 0.5% of the branched glucan which has a saccharide residue bound thereto produced in Example 5, Example 6, or Reference Example 7, 20 mM sodium acetate buffer (pH 5.5), and glucoamylase (17 U/ml; EC3.2.1.3) was incubated at 37°C for 18 hours, an amount of glucose in the reaction liquid was quantitated by a glucose oxidase method using Glucose CII-Test Wako (manufactured by Wako Pure Chemical Industries, Ltd.). A ratio of the glucan portion which had been degraded with glucoamylase relative to the whole branched glucan was obtained as a glucoamylase degradation ratio (%), and a ratio of the glucan portion which had not been degraded with glucoamylase relative to the whole branched glucan was obtained as a glucoamylase resistance (%) according to the following equations. The results are shown in Table 3.

$$\text{Glucoamylase degradation ratio} = (\text{amount of glucose in solution after reaction (mg)}/\text{amount of glucan in solution before reaction (mg)}) \times 100$$

Glucoamylase resistance = 1-glucoamylase degradation ratio

[Table 3]

Table 3: Amylase resistance of branched glucan which has various saccharide residues bound thereto (%)

| | Branched glucan of Production Example 2 | Branched glucan which has N-acetylglucosamine residue bound thereto of Example 5 | Branched glucan which has galactose residue bound thereto of Example 6 | Branched glucan which has glucosamine residue bound thereto of Reference Example 7 |
|---|---|---|---|---|
| Binding ratio to non-reducing end (%) | 0 | 46.8 | 38.5 | 66.8 |
| Glucoamylase degradation ratio (%) | 100 | 26.2 | 69.6 | 96.4 |
| Glucoamylase resistance (%) | 0 | 73.8 | 30.4 | 3.6 |

The branched glucan which hasan N-acetylglucosamine residue bound thereto and the branched glucan which has a galactose residue bound thereto exhibited resistance to glucoamylase. However, almost all of the branched glucan which has a glucosamine residue bound thereto was degraded with glucoamylase, and did not exhibit glucoamylase resistance. As described above, it was found that glucoamylase resistance can be imparted when galactose or N-acetylglucosamine is bound to a non-reducing end.

The glucan of the present invention exhibits a characteristic nature that it has resistance to glucoamylase alone. However, since a variety of amylases are present in a body, the glucan is thought to be a safe substance excellent in biodegradability, which is finally metabolized in a body and degraded to a size capable of being excreted to the outside of a body even if the glucan has resistance to glucoamylase.

(Example 11: Purification and structure confirmation of branched glucan which has N-acetylglucosamine residue bound to non-reducing end)

By the same method as that of Example 5, a branched glucan which has an N-acetylglucosamine residue bound to a non-reducing end was produced. First, *Aquifex aeolicus* VF5-derived $\alpha$-glucan phosphorylase (50 U) obtained in Production Example 1 was allowed to act on 2.5 ml of a reaction liquid containing 28 mg of the branched glucan obtained in Production Example 2, 15 mM N-acetylglucosamine-1-phosphate, and 100 mM sodium acetate buffer (pH 5.5) at 50°C for 24 hours. When a ratio of non-reducing end modification was analyzed similarly to Example 5, an amount of N-acetylglucosamine transferred to the branched glucan (ratio of non-reducing end modification) was 62.9%. After the resulting reaction liquid was adjusted to a pH of 3 with a 5 N HCl solution, a precipitate containing a denatured protein was removed by centrifugation. The supernatant was collected and neutralized with a 5 N NaOH solution. Then, using PD-10 column, small molecules were removed and, at the same time, the solution was desalted, this was further filtered with a 0.2 $\mu$m filter, and the resulting filtrate was lyophilized, thereby, a powder of a non-reducing end-modified branched glucan which has an N-acetylglucosamine residue bound to a non-reducing end was obtained. Hereinafter, a branched glucan which has an N-acetylglucosamine residue bound to a non-reducing end is also referred to as B-GlcNAc.

As described below, by comparison with the branched glucan produced in Production Example 2 (unmodified branched glucan; B), the structure of a non-reducing end-modified branched glucan (B-GlcNAc) was confirmed.

A powder of the purified non-reducing end-modified branched glucan (B-GlcNAc) obtained as described above was dissolved in 10 mM acetate buffer (pH 5.5) to be 0.2% by weight, various amylases of conditions 1 to 4 of Table 4 were added thereto, and the mixture was incubated at an appropriate temperature, thereby, the glucan was digested. Thereafter, an amount of glucose in the reaction liquid was measured. Since the amount of enzyme shown in Table 4 is an excessive amount, all of bonds that can be cut by the enzyme or combination of enzymes are cut. A glucose oxidase method was used for quantitating glucose.

In addition, the reaction liquids after enzymatic digestion under respective conditions 2 to 4 were analyzed with a HPAEC-PAD apparatus (pumping system: DX300, detector: PAD-2, analysis column: CarboPacPA100) manufactured by DIONEX. Elusion was performed under the conditions of a flow rate: 1 ml/min, a NaOH concentration: 150 mM, a sodium acetate concentration: 0 min-50 mM, 1 min-50 mM, 27 min-350 mM (Gradient curve No. 4), 35 min-850 mM (Gradient curve No. 7), 37 min-850 mM. The results are shown in Fig. 1. In addition, a schematic view of a structure of a product obtained after enzymatic treatment, presumed from properties of these enzymatic reactions, is shown in Fig. 3. Fig. 1 shows the results of HPAEC-PAD analysis after digestion of a non-reducing end-modified branched glucan (B-GlcNAc) or an unmodified branched glucan (B) with various amylases. Respective samples were as follows: Fig. 1-1: G1 to G6; Fig. 1-2: isoamylase degradation product of branched glucan (B) of Production Example 2; Fig. 1-3: product from degradation of branched glucan (B) of Production Example 2 with isoamylase and glucoamylase; Fig. 1-4: product from degradation of branched glucan (B) of Production Example 2 with isoamylase, glucoamylase and α-amylase; Fig. 1-5: isoamylase degradation product of branched glucan which has N-acetylglucosamine residue bound to non-reducing end; Fig. 1-6: product from degradation of branched glucan which has N-acetylglucosamine residue bound to non-reducing end with isoamylase and glucoamylase; Fig.1-7: product from degradation of branched glucan which has N-acetylglucosamine residue bound to non-reducing end with isoamylase, glucoamylase and α-amylase.

Regarding an amount of glucose, calculating the production amount of glucose produced by a reaction under Condition 4 of an unmodified branched glucan (B) as 100%, the glucose production amounts (%) under respective conditions are shown in Table 4. These results will be explained below.

Under Condition 1, glucoamylase which cuts α-1,4 and α-1,6 bonds of a glucan in a glucose unit from a non-reducing end side was allowed to act. For this reason, in the unmodified branched glucan of Production Example 2 constructed only of glucose residues, the amount of glucose was 100%, and all of this unmodified branched glucan was degraded into glucose. However, in B-GlcNAc in which an N-acetylglucosamine residue was bound to a non-reducing end of a glucan, the amount of glucose was 31.2%, and about 68% of bonds in this non-reducing end-modified branched glucan could not be degraded. This ratio was a value near the binding ratio (62.9%) of N-acetylglucosamine to a non-reducing end, which was quantitated from the phosphoric acid concentration shown in Example 5. As a result, it is presumed that an end-modified branched glucan (B-GlcNAc) has a structure on which glucoamylase having the property of cutting an α-1,4 glucosidic bond from a non-reducing end side cannot act and, therefore, an N-acetylglucosamine residue is bound to a non-reducing end part.

Under Condition 2, isoamylase which cuts an α-1,6 bond was allowed to act. Under Condition 2, regarding either of the branched glucan of Production Example 2 and the end-modified branched glucan of Example 5, the amount of glucose was 0.0%.

Further, under Condition 3, isoamylase and glucoamylase were allowed to act in combination. Under Condition 3, an α-1,6 bonds were cut with isoamylase, but regarding either of the branched glucan of Production Example 2 and the end-modified branched glucan of Example 5, the amount of glucose was not changed from that for Condition 1, and was 100% and 31.2%, respectively.

Under Condition 4, isoamylase, glucoamylase, and α-amylase have been allowed to act in combination. α-Amylase has the property of randomly cutting an α-1,4 bond of a glucan. Under Condition 4, all of the unmodified branched glucan of Production Example 2 was degraded into glucose, but only 83.7% of bonds in B-GlcNAc in which an N-acetylglucosamine residue bound to a non-reducing end were degraded. Therefore, in B-GlcNAc, a part of a structure which cannot be degraded with various amylases capable of degrading the unmodified branched glucan of Production Example 2 accounts for 16.3% of the total, and it is presumed that a product having this structure is an oligosaccharide which has an N-acetylglucosamine residue bound to a non-reducing end. (solid star mark in Fig. 1-7) . It is noted that the elution position having solid star mark by HPAEC-PAD in Fig. 1-7 was consistent with the elution position of an authentic sample of 4-0-α-N-acetylglucosaminylmaltose in which N-acetylglucosamine residue is bound via α-1,4 bond to the non-reducing end of maltose.

[Table 4]

Table 4 : Amount of glucose in reaction liquid after digestion of B-GlcNAc or B with various amylases

| Enzymatic digestion | | Amount of glucose (%) | |
|---|---|---|---|
| Condition | Kind of enzyme (addition concentration) | End-modified branched glucan (B-GlcNAc) | Unmodified branched glucan (B) of Production Example 2 |
| Condition 1 | Glucoamylase (5U/ml) | 31.2 | 100.0 |
| Condition 2 | Isoamylase (5U/ml) | 0.0 | 0.0 |
| Condition 3 | Isoamylase (5U/ml)<br>Glucoamylase (5U/ml) | 31.2 | 100.0 |
| Condition 4 | Isoamylase (5U/ml)<br>Glucoamylase (5U/ml) α-Amylase(5U/ml) | 83.7 | 100.0 |

(Example 12: Purification and structure confirmation of branched glucan which has galactose residue bound to non-reducing end)

*Aquifexaeolicus* VF5-derived α-glucan phosphorylase (50 U) obtained in Production Example 1 was allowed to act on 2.5 ml of a reaction liquid containing 28 mg of the branched glucan obtained in Production Example 2, 15 mM galactose-1-phosphate, and 100 mM sodium acetate buffer (pH 5.5) at 50°C for 24 hours. An amount of galactose transferred to the branched glucan was 62.2%. By purifying the resulting reaction liquid by the same method as that of Example 11, a powder of the branched glucan which has a galactose residue bound to a non-reducing end was obtained. Hereinafter, the branched glucan which has a galactose residue bound to a non-reducing end is also referred to as B-Gal.

As described below, by comparison with the branched glucan produced in Production Example 2 (unmodified branched glucan; B), the structure of the modified branched glucan which has a galactose residue bound to a non-reducing end (B-Gal) was confirmed.

A powder of the purified non-reducing end branched glucan (B-Gal) obtained as described above was dissolved in 10 mM acetate buffer (pH 5.5) to be 0.2% by weight, various amylases of Conditions 1 to 4 of Table 5 were added thereto, and the mixture was incubated at an appropriate temperature, thereby, the glucan was digested. Thereafter, an amount of glucose in the reaction liquid was measured. Since the enzyme amount shown in Table 5 is an excessive amount, bonds cuttable with the enzyme or combination of enzymes are all cut. A glucose oxidase method was used for quantitating glucose.

In addition, the reaction liquids after enzymatic digestion under respective conditions 2 to 4 were analyzed with HPAEC-PAD by the method of Example 11. The results are shown in Fig. 2. In addition, a schematic view of a structure of a product obtained after enzymatic treatment, which was presumed from the properties of these enzymatic reactions, is shown in Fig. 3. Fig. 2 shows the results of HPAEC-PAD analysis after digestion of the non-reducing end-modified branched glucan (B-Gal) or the unmodified branched glucan (B) with various amylases. Respective samples were as follows: Fig. 2-1: G1 to G6; Fig. 2-2: isoamylase degradation product of branched glucan (B) of Production Example 2; Fig. 2-3: product of degradation of branched glucan (B) of Production Example 2 with isoamylase and glucoamylase; Fig. 2-4: product of degradation of branched glucan (B) of Production Example 2 with isoamylase, glucoamylase and α-amylase; Fig. 2-5: isoamylase degradation product of branched glucan which has galactose residue bound to non-reducing end; Fig. 2-6: isoamylase and glucoamylase degradation product of branched glucan which has galactose residue bound to non-reducing end; Fig. 2-7: isoamylase, glucoamylase and α-amylase degradation product of branched glucan which has galactose residue bound to non-reducing end.

The production amount of glucose produced in a reaction under Condition 4 of the unmodified branched glucan (B) as 100%, the glucose production amounts (%) under respective conditions are calculated and shown in Table 5. These results will be explained below.

Under Condition 1, glucoamylase which cuts α-1,4 and α-1,6 bonds of a glucan in a glucose unit from a non-reducing end side was allowed to act. For this reason, the amount of glucose for the unmodified branched glucan of Production Example 2 constructed only of glucose residues was 100%, and this unmodified branched glucan was all degraded into glucose. However, in B-Gal in which a galactose residue is bound to a non-reducing end of a glucan, the amount of glucose was 30.2%, and about 70% of bonds in this non-reducing end-modified branched glucan, could not be degraded. This ratio was a value near the binding ratio of galactose to a non-reducing end (62.2%), which was quantitated from the phosphoric acid concentration. As a result, it is presumed that the end-modified branched glucan produced in Example 5 has a structure which cannot be acted by glucoamylase having the property of cutting an α-1,4 glucoside bond from a non-reducing end side and, therefore, a galactose residue is bound to a non-reducing end part.

Under Condition 2, isoamylase which cuts an α-1,6 bond was allowed to act. Under Condition 2, concerning the branched glucan of Production Example 2, maltooligosaccharides were produced (Fig. 2-2). When isoamylase was allowed to act on the branched glucan which has galactose bound thereto, oligosaccharides in which a non-reducing end is a glucose residue (solid circles in Fig. 2-5, eluted at the same elution position as that of Fig. 2-2, respectively) and oligosaccharides which has galactose bound to a non-reducing end (solid stars in Fig. 2-5) were produced.

Under Condition 3, when isoamylase and glucoamylase were allowed to act on the non-reducing end-modified glucans produced in Example 5, the peaks with solid circle in Fig. 2-5 were degraded and glucose (G1) was produced, but there was no change in the peaks for oligosaccharides which has galactose bound to a non-reducing end (solid stars in Fig. 2-5). The amount of glucose under Condition 3 of B-Gal in which a galactose residue bound to a non-reducing end was 30.3%.

Under Condition 4, isoamylase, glucoamylase, and α-amylase have been allowed to act in combination. α-Amylase has the property of randomly cutting an α-1,4 bond of a glucan. Under Condition 4, the unmodified branched glucan of Production Example 2 was all degraded into glucose, but only 90.9% of bonds in B-Gal in which a galactose residue bound to a non-reducing end were degraded. Therefore, in B-Gal, a part of a structure which cannot be degraded with various amylases capable of degrading the unmodified branched glucan of Production Example 2 accounts for 9.1% of the total, and it is presumed that this product is an oligosaccharide which has a galactose residue bound to a

non-reducing end. (solid star mark in Fig. 2-7) . It is noted that the elution position having solid star mark by HPAEC-PAD in Fig. 2-7 was consistent with the elution position of an authentic sample of 4-0-α-galactosylmaltose in which galactose residue is bound via α-1,4 bond to the non-reducing end of maltose.

[Table 5]

Table 5: Amount of glucose in reaction liquid after digestion of B-Gal or B with various amylases

| Enzymatic digestion | | Amount of glucose (%) | |
|---|---|---|---|
| Condition | Kind of enzyme (addition concentration) | End-modified branched glucan (B-Gal) | Branched glucan (B) of Production Example 2 |
| Condition 1 | Glucoamylase (5U/ml) | 30 .2 | 100.0 |
| Condition 2 | Isoamylase (5U/ml) | 0.0 | 0.0 |
| Condition 3 | Isoamylase (5U/ml) Glucoamylase (5U/ml) | 30 .3 | 100.0 |
| Condition 4 | Isoamylase (5U/ml) Glucoamylase (5U/ml) α-Amylase (5U/ml) | 90.9 | 100.0 |

(Example 13: Biodegradability of branched glucan which has N-acetylglucosamine residue bound thereto (1))

A powder of the branched glucan which has an N-acetylglucosamine residue bound thereto purified in Example 11 was dissolved by adding into 20 mM acetate buffer (pH 5.5) to be 0.5% by weight, to this solution was added 23 U/ml of α-amylase, and the mixture was incubated at 37°C, thereby, the α-amylase was allowed to act, and reduction in the weight average molecular weight of the product was investigated over time.

For the measurement of weight average molecular weight of the product, the method of measuring the weight average molecular weight of branched glucan shown in Production Example 2 was used. The results are shown in Table 6 and Fig. 4.

The weight average molecular weight of the branched glucan which hasan N-acetylglucosamine residue bound thereto was reduced by the action of α-amylase, and the weight average molecular weight became 1/2 or less in the reaction time of 10 minutes, and about 1/5 in the reaction time of 30 minutes. At the reaction time of 60 minutes, the branched glucan which has an N-acetylglucosamine residue bound thereto was degraded to a molecule having a molecular weight of 30000 or less. Amylase in blood is α-amylase. Therefore, the non-reducing end-modified branched glucan of the present invention is thought to be degraded with the amylase in blood to a molecule having a molecular weight of 30000 or less. Since molecules having a molecular weight of 30000 or less are thought to be excreted into urine, it is thought that the non-reducing end-modified branched glucan of the present invention is biodegradable.

[Table 6]

Table 6: Change in weight average molecular weight over time of product when branched glucan which hasN-acetylglucosamine residue bound thereto is degraded with α-amylase

| Reaction time (min) | Weight average molecular weight (x $10^3$) |
|---|---|
| 0 | 157.9 |
| 10 | 75.8 |
| 20 | 58.4 |
| 30 | 37.8 |
| 60 | 28.4 |

(Example 14: Biodegradability of acetylated branched glucan which has N-acetylglucosamine residue bound thereto (2))

A powdery branched glucan was produced by acetylating the branched glucan of Production Example 2, and an acetylated branched glucan which has an N-acetylglucosamine residue bound thereto was produced by the same method as that of Example 11. Acetylation of the branched glucan was performed by mixing the branched glucan dissolved in dimethyl sulfoxide (DMSO), DMSO, a 4% aqueous $Na_2CO_3$ solution, and 5 M vinyl acetate prepared in DMSO in a composition of Table 7, and allowed to react at 25°C for 1 hour with stirring. The resulting reaction liquid was diluted 2-fold with ultrapure water, desalting and solvent removal were performed using a gel filtration column (PD-10), and purified acetylated branched glucans were collected with ultrapure water as an aqueous solution. To 300 μl of the collectedaqueous solution of purified acetylated branched glucans was added 200 μl of a 5 N aqueous sodium hydroxide solution, and this was heated at 55°C for 30 minutes, thereby, the acetylated branched glucans were deacetylated. To this reaction liquid was added 300 μl of a 1 N Tris buffer (pH 7) and, further, 200 μl of 5 N hydrochloric acid was added to neutralize the solution. When an amount of glucose in the solution after neutralization was quantitated by a phenol sulfuric acid method, free acetic acid in the solution after neutralization was quantitated with a free acetic acid quantitating kit, and an acetylation degree was obtained, it was confirmed that the acetylation degree of the resulting acetylated branched glucans were 0.1 to 1.3. As described above, three kinds of aqueous solutions containing acetylated glucans were obtained. Each aqueous acetylated glucan solution was lyophilized to obtain each about 300 mg of a powder of the acetylated glucans.

[Table 7]

Table 7: Summary of reaction conditions

| Acetylation degree | 0 | 0.1 | 0.5 | 1.3 |
|---|---|---|---|---|
| 1.87% Branched glucan (B) (ml) | 16.00 | 16.00 | 16.00 | 16.00 |
| DMSO (ml) | 3.00 | 2.86 | 2.78 | 2.56 |
| 4% $Na_2CO_3$ (ml) | 1.00 | 1.00 | 1.00 | 1.00 |
| 5 M vinyl acetate (ml) | 0 | 0.14 | 0.22 | 0.44 |

*Aquifex aeolicus-derived* α-glucan phosphorylase (50 U/ml) prepared in Production Example 1 was added to a reaction liquid containing 2.5% of the resulting acetylated branched glucan powder, 10 mM N-acetylglucosamine-1-phosphate, and 100 mM acetate buffer (pH 5.5) and allowed to react at 50°C for 18 hours. After the resulting reaction liquid containing a branched glucan which has an N-acetylglucosamine residue bound thereto was adjusted to a pH of 3 with a 5 N HCl solution, a precipitate containing a denatured protein was removed by centrifugation. The supernatant was collected and neutralized with a 5 N NaOH solution. Then, using a gel filtration column (PD-10), small molecules were removed, and at the same time, the solution was desalted , this was further filtered with a 0.2 μm filter, and the resulting filtrate was lyophilized, thereby, a powder of acetylated branched glucans produced by the method of Example 14 was obtained.

23 U/ml α-amylase was added to the resulting reaction liquid containing 0.5% by weight of the acetylated branched glucan which hasan N-acetylglucosamine residue bound thereto and 20 mM acetate buffer (pH 5.5), and this was incubated at an appropriate temperature, thereby, α-amylase was allowed to act on the acetylated branched glucans, and reduction in the weight average molecular weight of the products were investigated over time.

Measurement of the molecular weight was performed similarly to Example 13. The results are shown in Table 8 and Fig. 5. From this result, it was shown that the branched glucan which hasan N-acetylglucosamine residue bound thereto can be controlled for degradation by acetylation of the branched glucan moiety.

[Table 8]

Table 8: Change over time in weight average molecular weight when non-reducing end N-acetylglucosamine residue-bound branched glucans having different acetylation degrees are degraded with α-amylase

| | Weight average molecular weight of product (x $10^3$) | | | |
|---|---|---|---|---|
| Acetylation degree | 0 | 0.1 | 0.5 | 0.9 |
| Reaction time (min) | | | | |
| 0 | 157.9 | 158.0 | 172.3 | 168.9 |
| 10 | 75.8 | 94.8 | 144.7 | 164.5 |
| 20 | 41.1 | 67.9 | 120.6 | 164.7 |
| 30 | 36.3 | 55.3 | 110.3 | 161.3 |
| 60 | 28.4 | 39.5 | 93.6 | 161.1 |

(Example 15: Biodegradability of branched glucan which has galactose residue bound thereto (1))

The powder of the branched glucan which has a galactose residue bound thereto purified in Example 12 was dissolved by adding to 20 mM acetate buffer (pH 5.5) to be 0.5% by weight, to this solution was added 23 U/ml of α-amylase, and this was incubated at 37 °C to act α-amylase, and reduction in the weight average molecular weight of the product was investigated over time.

For the measurement of weight average molecular weight of a product, the method of measuring the weight average molecular weight of branched glucan shown in Production Example 2 was used. The results are shown in Table 9 and Fig. 6.

The weight average molecular weight of the branched glucan which has a galactose residue bound thereto was reduced by the action of α-amylase, and the weight average molecular weight became 1/3 or less in the reaction time of 10 minutes, and about 1/5 in the reaction time of 20 minutes. After the reaction time of 60 minutes, the branched glucan which has a galactose residue bound thereto was rapidly degraded to a molecule having the molecular weight of 30000 or less. Blood amylase is α-amylase. Therefore, the non-reducing end-modified branched glucan of the present invention is thought to be degraded with blood amylase to a molecule having the molecular weight of 30000 or less. Since molecules having a molecular weight of 30000 or less are thought to be excreted into urine, it is thought that the non-reducing end-modified branched glucan of the present invention is biodegradable.

[Table 9]

Table 9: Change in weight average molecular weight over time of product when branched glucan which has galactose residue bound thereto is degraded with α-amylase

| Reaction time (min) | Weight average molecular weight (x $10^3$) |
|---|---|
| 0 | 145.2 |
| 10 | 42.1 |
| 20 | 29.0 |
| 30 | 27.6 |
| 60 | 23.2 |

(Example 16: Biodegradability of acetylated branched glucan which has galactose residue bound thereto (2))

Acetylated branched glucans in which a galactose residue is bound to a non-reducing end of the acetylated branched glucans used in Example 14 were prepared, and control of amylase degradability was studied.

*Aquifex aeolicus*-derived α-glucan phosphorylase (50 U/ml) prepared by Production Example 1 was added to a reaction liquid containing 2.5% of the resulting acetylated branched glucan, 10 mM galactose-1-phosphate, and 100 mM acetate buffer (pH 5.5), and this was allowed to react at 50°C for 18 hours. After the resulting reaction liquid containing the branched glucans which have a galactose residue bound thereto was adjusted to a pH of 3 with a 5 N HCl solution, a precipitate containing a denatured protein was removed by centrifugation. The supernatant was collected and neutralized with a 5 N NaOH solution. Then, using PD-10 column, small molecules were removed and, at the same time, the solution was desalted and, further, the solution was filtered with a 0.2 μm filter, thereafter, the resulting filtrate was lyophilized, thereby, a powder in which a galactose residue is bound to a non-reducing end of the acetylated branched glucan produced by the method of Example 14 was obtained.

23 U/ml of α-amylase was added to a reaction liquid containing 0.5% by weight of the resulting branched glucan which has a galactose residue bound thereto and 20 mM acetate buffer (pH 5.5), the mixture was incubated at an appropriate temperature, thereby, α-amylase was allowed to act on the acetylated branched glucans, and reduction in the weight average molecular weight of the product was investigated over time.

For the measurement of weight average molecular weight of a product, the method of measuring the weight average molecular weight of branched glucan shown in Production Example 2 was used. The results are shown in Table 10 and Fig. 7. From this result, it was shown that the branched glucan which has a galactose residue bound thereto can be controlled for degradation by acetylation of the branched glucan moiety.

[Table 10]

Table 10 : Change over time in weight average molecular weight when non-reducing end galactose residue-bound branched glucans having different acetylation degrees are degraded with α-amylase

| | Weight average molecular weight of product (x $10^3$) | | | |
|---|---|---|---|---|
| Acetylation degree | DS0 | DS0.1 | DS0.5 | DS0.9 |
| Reaction time (min) | | | | |
| 0 | 145.2 | 146.1 | 152.5 | 155.4 |

(continued)

| | Weight average molecular weight of product (x $10^3$) | | | |
|---|---|---|---|---|
| Acetylation degree | DS0 | DS0.1 | DS0.5 | DS0.9 |
| Reaction time (min) | | | | |
| 10 | 42.1 | 87.7 | 128.1 | 151.4 |
| 20 | 29.0 | 62.8 | 106.8 | 151.5 |
| 30 | 27.6 | 51.1 | 97.6 | 148.4 |
| 60 | 23.2 | 36.5 | 82.8 | 148.3 |

(Example 17: Biodegradability of branched glucan which has an N-acetylglucosamine residue bound thereto (3))

In vivo biodegradability of a branched glucan which has an N-acetylglucosamine residue bound thereto was investigated. In brief, as an evaluation sample, the branched glucan produced in Production Example 2 and the acetylated branched glucan prepared by the method shown in Example 14 were fluorescently labeled, respectively, and glucan amounts in blood and urine were investigated by a GPC-fluorescent labeling method.

For fluorescent labeling of the glucan, the branched glucan and the acetylated branched glucan having an acetylation degree of 0.5 were prepared in a 50 mg/ml DMSO solution, respectively. 2 ml of each glucan solution in DMSO was put in a test tube and stirred at 90°C. To this was added a 50 mg/ml fluorescein isothiocyanate (FITC) solution in DMSO in an amount of 56 μl and 112 μl, respectively. One drop of pyridine and one drop of di-n-butyltin dilaurate were added dropwise, and the mixture was stirred at 90°C for 2 hours. After completion of the reaction, to the reaction liquid was added 20 ml of ethanol, the solution was centrifuged at 3500 rpm for 5 minutes, and a precipitate was collected. Further, ethanol was added to perform washing, the precipitate was dissolved in 1 ml of ultrapure water, 1 ml of the solution was applied on a gel filtration column (PD-10), 1.5 ml of ultrapure water was passed thereon. Thereafter, 2 ml was collected with ultrapure water to perform purification. The resulting samples were lyophilized. For a part of the resulting samples, UV absorption at 490 nm was measured. Uranine was used as a standard substance to obtain the FITC introduction amount of a FITC-labeled branched glucan (F-B) and acetylated branched glucan (F-AcB) according to the following equation, it was confirmed that the introduction ratio was 0.86% and 0.82%, respectively.

FITC introduction ratio (W/W%)=((fluorescein concentration of FITC-labeled branched glucan) / (total saccharide amount of FITC-labeled branched glucan)) x 100

From the resulting two kinds of fluorescein isothiocyanated branched glucans, further F-B-GlcNAc (binding ratio 64. 6%) and F-AcB-GlcNAc (binding ratio 51.7%) in which N-acetylglucosamine is bound to a non-reducing end of a glucan, were prepared by the method of Example 3. The resultant product was administered in an amount of 25 mg/kg into tail veins of 9 week old male SD rats (6 animals), and the concentration of the FITC-labeled body in blood after administration was investigated using a high performance liquid chromatography (HPLC) system (column: manufactured by Tosoh Corporation, TSK-gel G4000 PWXL, flow rate 1.0 ml/min, eluate 200 mM NaCl 20 mM Tris-HCl buffer (pH 7.8)) equipped with a fluorescent detector (10RF 10AXL manufactured by Shimadzu Corporation). Each 300 μl of blood was collected at each time point from 5 minutes to 48 hours after administration shown in Table 11, and 100 μl of the supernatant was collected after centrifugation at 10, 000 rpm for 1 minute. After addition of 10 μl of 30 wt% trifluoroacetic acid, centrifugation at 10,000 rpm was performed for 5 minutes. To the 60 μl of collected supernatant, 60 μl of 200 mM Tris-HCl buffer (pH 7.8) was added. If necessary, the samples were diluted. Thereafter, it this was filtered with a 0.45 μm filter, and 100 μl of the filtrate was poured into the HPLC system.

The results are shown in Table 11 and Fig. 8. There was exhibited a tendency that, in blood, the branched glucan which has N-acetylglucosamine bound thereto is rapidly degraded and disappears. The acetylated branched glucan which has N-acetylglucosamine bound thereto exhibited a tendency that reduction in the molecular weight is very slow and that the glucan is slowly degraded and disappears. It was found that degradation of the branched glucan which has N-acetylglucosamine bound thereto can be controlled by acetylation.

[Table 11]

Table 11

| Time | Residual ratio of Dose glucan in blood (% of | |
|---|---|---|
| (min) | F-AcB-GlcNAc | F-B-GlcNAc |
| 5 | 100.00 | 12.59 |
| 15 | 73.64 | 8.25 |

(continued)

| Time | Residual ratio of Dose glucan in blood (% of | |
|---|---|---|
| (min) | F-AcB-GlcNAc | F-B-GlcNAc |
| 30 | 48.50 | 4.88 |
| 60 | 39.47 | 2.39 |
| 120 | 16.99 | 0.45 |
| 360 | 0.95 | 0.00 |
| 2880 | 0.00 | 0.00 |

(Example 18: Biodegradability of branched glucan which has galactose residue bound thereto (3))

In vivo biodegradability of a branched glucan which has a galactose residue bound thereto was investigated.

The FITC-labeled branched glucan (F-B) and acetylated branched glucan (F-AcB) prepared in Example 17 were further treated with the method of Example 4 to prepare F-B-Gal (binding ratio 64.3%) and F-AcB-Gal (binding ratio 51.4%) in which a galactose residue is bound to a non-reducing end of the glucan. The resultant was administered in an amount of 25 mg/kg into tail veins of 9 week old male SD rats (6 animals), and the concentration of the FITC-labeled body in blood after administration was measured by the method of Example 17. The results are shown in Table 12 and Fig. 9. There was exhibited a tendency that, in blood, the branched glucan which has a galactose bound thereto (F-B-Gal) is rapidly degraded and disappears. The acetylated branched glucan which has a galactose residue bound thereto (F-AcB-Gal) exhibited a tendency that reduction in the molecular weight is very slow, and the glucan is slowly degraded and disappears. It was understood that degradation of the branched glucan which has a galactose residue bound thereto can be controlled by acetylation.

[Table 12]

Table 12

| Time | Residual ratio of glucan in blood (% of Dose) | |
|---|---|---|
| (min) | F-AcB-Gal | F-B-Gal |
| 5 | 100.00 | 12.59 |
| 15 | 79.66 | 8.25 |
| 30 | 43.31 | 2.84 |
| 60 | 35.16 | 0.79 |
| 120 | 19.16 | 0.26 |
| 360 | 0.53 | 0.01 |
| 2880 | 0.00 | 0.00 |

(Example 19: Evaluation of targeting of branched glucan which has galactose residue bound to non-reducing end)

Whether a branched glucan which has a galactose residue bound to a non-reducing end is taken into liver in a larger amount than a branched glucan which has no galactose residue bound thereto or not was investigated.

The acetylated branched glucan (F-AcB140) produced in Example 14 and the acetylated branched glucan in which a galactose residue is bound to a non-reducing end (F-AcB140 (Gal)) produced in Example 15 were administered into a tail vein of a 7 week old male ICR mouse (around 30 g), and orbital blood was collected 2 minutes after administration, and whole-blood collection and evisceration were performed 10 minutes, 1 hour, 2 hours, and 6 hours after administration. Serum, and liver homogenate extraction liquid were analyzed by the GPC fluorescent detecting method as in Example 17. The results are shown in Table 13 and Fig. 10.

Regarding the serum, 300 μl of blood was centrifuged at 10000 rpm for 1 minute to collect serum, 10 μl of 30% trifluoroacetic acid was mixed therein and, further, the mixture was centrifuged at 10000 rpm for 1 minute to obtain 60 μl of the supernatant. 200 mM Tris-HCl buffer (pH 7.8) was added in an amount of 60 μl. Regarding liver, PBS in a 4-fold amount was added to the liver and this was homogenized, and then the homogenate was centrifuged at 10000 rpm for 5 minutes to obtain 200 μl of the supernatant. 20 μl of 30% trifluoroacetic acid was mixed therein and, further, the mixture was centrifuged at 10000 rpm for 5 minutes to obtain 150 μl of the supernatant, and an equal amount of 200 mM Tris-HCl buffer (pH 7.8) was added to prepare it.

For the branched glucan which has a galactose residue bound thereto, the concentration in blood was significantly decreased and, on the other hand, the concentration in liver was significantly increased. From these results, application to liver targeting was suggested.

[Table 13]

Table 13

| Time (min) | Average concentration in blood (% of Dose) | | Average concentration in liver (% of Dose) | |
|---|---|---|---|---|
| | F-AcB140 | F-AcB140 (Gal) | F-AcB140 | F-AcB140 (Gal) |
| 2 | 113.3 | 110.0 | | |
| 10 | 89.4 | 67.5 | 6.2 | 7.4 |
| 60 | 41.2 | 13.6 | 19.7 | 27.1 |
| 120 | 14.8 | 1.7 | 27.0 | 30.4 |
| 360 | 0.3 | 0.0 | 30.3 | 28.8 |

(Example 20: Dendritic cell stimulating activity of various non-reducing end-modified glucans)

After protein removal, desalting, and small molecule removal of the branched glucan of Production Example 2 were performed, filtration with a 0.2 μm filter was performed, and then lyophilization was performed. 50 U of AqGP of Production Method 1 was added to 5 ml of a reaction liquid containing 110 mg of the resulting branched glucan powder, 1, 3, 10, or 15 mM glucose-1-phosphate derivative (N-acetylglucosamine-1-P (GlcNAc-1-P), galactose-1-P (Gal-1-P), mannose-1-P (Man-1-P), glucosamine-1-P (GlcN-1-P), or glucuronic acid-1-P (GlcA-1-P)) and 100 mM sodium acetate buffer (pH 5.5) and this was allowed to react at 50°C for 24 hours. The reaction liquid was adjusted to a pH of 3 with a 5 N HCl solution, and precipitates containing denatured proteins were removed by centrifugation. The supernatant was collected and neutralized with a 5 N NaOH solution. Then, small molecule removal and desalting of it were performed with a PD-10 column, it was filtrated with a 0.2 μm filter, and then it was lyophilized.

Then, a dendritic cell was stimulated. Details were as follows : For preparing a dendritic cell, bone marrow cells were separated from femur of a C3H/HeJ mouse (7 week old, female, Japan SLC, Inc.), suspended in 10 ml of a RPMI1640 (Sigma) medium containing 10% FBS (bovine fetal serum, Invitrogen), 50 ng/ml rm GM-CSF (PEPROTECH), 25 ng/ml IL-4 (BD Biosciences), and 50 μg/ml gentamycin (Sigma) so as to be 5 x $10^6$ cells/ml, and cultured with a petri dish in $CO_2$ incubator at 37 °C for 7 days. Each 2.5 ml of the RPMI medium was added on Day 2 and Day 4 from the start of culturing. After culturing for 7 days, the medium was removed, a PBS buffer was put in the petri dish, and suspending cells were removed. Further, ice-cooled PBS Buffer containing 5 mMEDTA was flushed on the petri dish bottom, and adhesive immature dendritic cells were peeled with the water stream. The resulting immature dendritic cell suspension was centrifuged at 4°C and 1,200 rpm for 5 minutes to collect a precipitate. The resulting dendritic cell was suspended in a RPMI medium containing 10% FBS (bovine fetal serum, Invitrogen) and 50 μg/ml gentamycin (Sigma) so as to be 5 x $10^5$ cell/ml. The dendritic cell suspension (180 μl) was put in a 96-well plate, and incubated in a $CO_2$ incubator at 37°C for 1 hour to adhere cells to the plate. INF-γ (10 ng/ml) and various non-reducing end-modified glucans (1 mg/ml) having the binding ratio to a non-reducing end described in Table 14 prepared above were added to this cell, and incubated in a $CO_2$ incubator at 37°C for 48 hours, thereby, cell stimulation was performed. After cell stimulation, the cell supernatant was collected by centrifugation, the production amount of IL-6 in the supernatant was measured using the OptEIA ELISA kit (BD Biosciences) by the ELISA method, and IL-6 produced by dendritic cell stimulation was calculated.

The results are shown in Table 14 and Fig. 11.

[Table 14]

| Bound saccharide residue | Average binding ratio to end (%) | Average number of binding to end | IL-6 production amount (pg/ml) |
|---|---|---|---|
| **Gal** | 0 | 0 | 59.8 |
| | 3.6 | 2 | 61.1 |
| | 9.6 | 6 | 70.9 |
| | 23.8 | 14 | 90.0 |
| | 43.8 | 26 | 140.5 |
| | 59.3 | 36 | 184.5 |
| GlcNAc | 0 | 0 | 59.8 |
| | 3.4 | 2 | 60.1 |
| | 12.5 | 8 | 77.8 |
| | 20.5 | 12 | 101.9 |
| | 55.5 | 33 | 203.3 |
| Man | 0.0 | 0 | 59.8 |
| | 3.3 | 2 | 60.8 |
| | 12.1 | 7 | 73.8 |
| | 21.0 | 13 | 88.6 |
| | 66.9 | 40 | 122.1 |
| GlcA | 0.0 | 0 | 59.8 |
| | 6.9 | 4 | 72.9 |
| | 20.4 | 12 | 106.2 |
| | 38.6 | 23 | 143.3 |
| | 85.3 | 51 | 153.7 |
| GlcN | 0.0 | 0 | 59.8 |
| | 13.0 | 8 | 60.5 |
| | 20.0 | 12 | 58.2 |
| | 61.5 | 37 | 61.8 |
| | 77.7 | 40 | 61.1 |
| Average binding ratio to end (%) : Average binding ratio of each saccharide residue of non-reducing end-modified branched glucan<br>Average number of binding to end: {Branching number of each non-reducing end-modified branched glucan x average binding ratio to end} / 100<br>Gal = Galactose residue<br>GlcNAc = N-acetylglucosamine residue<br>Man = Mannose residue<br>GlcA = Glucuronic acid residue<br>GlcN = Glucosamine residue | | | |

(Example 21: Production of various non-reducing end-modified glucans)

Non-reducing end-modified branched glucans were produced in combination of binding saccharides shown in Conditions 1 to 15 of Table 15.

Regarding Conditions 1 to 6, 50 U of AqGP of Production Method 1 was added to 5 ml of a reaction liquid

containing 110 mg of the branched glucan powder (which was obtained after protein removal, desalting, and small molecule removal of the branched glucan of Production Example 2, filtration of it with a 0.2 μm filter, and then lyophilization of it), 15 mM glucose-1-phosphate derivative (N-acetylgulcosamine-1-P (GlcNAc-1-P), galactose-1-P (Gal-1-P), mannose-1-P (Man-1-P), glucosamine-1-P (GlcN-1-P), or glucuronic acid-1-P (GlcA-1-P)) and a 100 mM sodium acetate buffer (pH 5.5) and allowed to act at 50°C for 24 hours. The results of quantitation of inorganic phosphate after the reaction were calculated as amounts of binding to a non-reducing end and shown in the table. The reaction liquid was adjusted to a pH of 3 with a 5 N HCl solution, and a precipitate containing a denatured protein was removed by centrifugation. The supernatant was collected and neutralized with a 5 N NaOH solution. Then, a small molecule removal and desalting of it were performed with a PD-10 column and, further, it was filtered with a 0.2 μm filter. Regarding Conditions 7 to 14, 5 ml of a reaction liquid containing 110 mg of the branched glucan powder of Production Example 2, and 10 mM N-acetylglucosamine-1-P or galactose-1-P was prepared similarly to Conditions 1 to 6. Thereafter, it was subjected to small molecule removal and desalting, and 2 ml of a reaction liquid containing 1 ml of the branched glucan in which a non-reducing end was modified with one kind of saccharide and 10 mM glucuronic acid-1-P, glucosamine-1-P, galactose-1-P, or mannose-1-P was prepared similarly to Conditions 1 to 6. Thereafter, it was subjected to small molecule removal and desalting. Regarding Conditions 16 to 22, 10 ml of a reaction liquid containing 220 mg of the branched glucan powder of Production Example 2, and 10mM N-acetylglucosamine-1-Porgalactose-1-P was prepared similarly to Conditions 1 to 6. Thereafter, it was subjected to small molecule removal and desalting to obtain the branched glucan in which a non-reducing end was modified with one kind of saccharide. 2 ml of a reaction liquid containing 1 ml of the branched glucan in which a non-reducing end was modified with one kind of saccharide, and two kinds of 10 mM glucuronic acid-1-P, glucosamine-1-P, galactose-1-P or mannose-1-P was prepared similarly to Conditions 1 to 6. Thereafter, it was subjected to small molecule removal and desalting. Regarding Condition 23, 5 ml of a reaction liquid containing 110 mg of the branched glucan powder of Production Example 2, 10 mM mannose-1-P, glucuronic acid-1-P, and glucosamine-1-P was prepared similarly to Conditions 1 to 6. Regarding Conditions 1 to 23, all samples were allowed to an enzymatic reaction, and thereafter subjected to degeneration of GP enzyme protein, small molecule removal and desalting, filtered with a 0.2 μm filter, and lyophilized. The resulting various branched glucans were used in Examples 22 to 27.

[Table 15]

Table 15

| Condition | Bound saccharide residue | Amount of binding of various saccharides to non-reducing end | | | | | | |
|---|---|---|---|---|---|---|---|---|
| | | GlcA (%) | GlcN (%) | GlcNAc (%) | Gal (%) | Man (%) | Total (%) | (Number) |
| 1 | None | | | | | | 0.0 | 0 |
| 2 | GlcA | 76.0 | | | | | 76.0 | 46 |
| 3 | GlcN | | 61.5 | | | | 61.5 | 37 |
| 4 | GlcNAc | | | 60.9 | | | 60.9 | 37 |
| 5 | Gal | | | | 43.8 | | 43.8 | 26 |
| 6 | Man | | | | | 62.8 | 62.8 | 38 |
| 7 | GlcNAc/GlcA | 11.2 | | 55.5 | | | 66.7 | 40 |
| 9 | GlcNAC/Gal | | | 19.4 | 43.8 | | 63.2 | 38 |
| 10 | GlcNAc/Man | | | 55.5 | | 39.3 | 94.8 | 57 |
| 11 | Gal/GlcA | 15.3 | | | 43.8 | | 59.1 | 36 |
| 13 | Gal/Man | | | | 43.8 | 38.4 | 82.2 | 49 |
| 14 | Man/GlcA | 28.5 | | | | 62.8 | 91.3 | 55 |
| 17 | GlcNAc/GlcA/Gal | | | | | | 44.1 | 27 |
| 18 | GlcNAc/GlcA/Man | | | | | | 39.8 | 24 |
| 21 | GlcNAc/Gal/Man | | | | | | 51.3 | 31 |

(continued)

| Condition | Bound saccharide residue | Amount of binding of various saccharides to non-reducing end | | | | | | |
|---|---|---|---|---|---|---|---|---|
| | | GlcA (%) | GlcN (%) | GlcNAc (%) | Gal (%) | Man (%) | Total (%) | (Number) |
| 23 | Gal/GlcA/Man | | | | | | 45.9 | 28 |
| GlcA = Glucuronic acid residue<br>GlcN = Glucosamine residue<br>GlcNAc = N-acetylglucosamine residue<br>Gal = Galactose residue<br>Man = Mannose residue | | | | | | | | |

(Example 22: Dendritic cell stimulating activity of branched glucan which has one of various saccharides bound to non-reducing end)

Various branched glucans (1 mg/ml cell liquid) produced in Example 21 were added to the C3H/HeJ mouse (female, 7 week old)-derived dendritic cells prepared in Example 20, and these were allowed to react in a $CO_2$ incubator at 37°C for 48 hours. The supernatant of the reaction liquid was collected, and IL-6 produced by cell stimulation was measured by the ELISA method. The results are shown in Table 16 and Fig. 12.

Regarding all of branched glucans which has glucuronic acid, N-acetylglucosamine, galactose, or mannose bound to a non-reducing end, the IL-6 production amount was increased as compared with those for the branched glucan of Production Example 2. On the other hand, regarding the branched glucan which has a glucosamine bound thereto, the production amount of IL-6 was decreased than those for the branched glucan of Production Example 2, and there was exhibited a tendency that production of IL-6 is suppressed.

[Table 16]

Table 16: Production condition

| Production condition (Example 21) | Cell stimulating substance (bound saccharide) | IL-6 production amount |
|---|---|---|
| 1 | Glc | 87.3 |
| 2 | GlcA | 143.3 |
| 3 | GlcN | 61.8 |
| 4 | GlcNAc | 139.9 |
| 5 | Gal | 114.6 |
| 6 | Man | 122.1 |
| | DW | 4.2 |
| | Pam3 200ng/ml | 247.1 |
| | Pam3 10μg/ml | 318.0 |

(Example 23: Dendritic cell stimulating activity of branched glucans in which N-acetylglucosamine and various other saccharides are bound to non-reducing end)

Various branched glucans (1 mg/ml cell liquid) prepared in Example 19 were added to a dendritic cell prepared by the same method as that of Example 20, and they were reacted in a $CO_2$ incubator at 37°C for 48 hours. The supernatant of the reaction liquid was collected, and IL-6 produced by cell stimulation was measured by the ELISA method. The results are shown in Table 17 and Fig. 13.

Regarding the branched glucan which has N-acetylglucosamine bound to a non-reducing end, the IL-6 production amount was increased as compared with those for the branched glucan of Production Example 2. Using N-acetylglucosamine and galactose or mannose in combination, the production amount of IL-6 was further increased. The branched glucan which has N-acetylglucosamine bound to a non-reducing end was recognized to have the activity of stimulating a dendritic cell and, further, the synergistic effect of the cell stimulating activity was recognized by additionally binding galactose or mannose simultaneously with N-acetylglucosamine.

[Table 17]

Table 17

| Production condition (Example 21) | Cell stimulating substance (bound saccharide) | IL-6 production amount |
|---|---|---|
| 1 | Glc | 87.3 |
| 4 | GlcNAc | 139.9 |
| 7 | GlcNAc/GlcA | 195.9 |
| 10 | GlcNAc/Man | 310.3 |
| 9 | Gal/GlcNAc | 317.1 |
| | DW | 4.2 |
| | Pam3 200ng/ml | 247.1 |
| | Pam3 10μg/ml | 318.0 |

(Example 24: Dendritic cell stimulating activity of branched glucans which have galactose and other various saccharides bound to non-reducing end)

Similarly to Example 21, the IL-6 production amount of branched glucans, in which bindings of galactose and other saccharides were combined, was investigated. The results are shown in Table 18 and Fig. 14. Regarding the branched glucan which has galactose bound thereto, the IL-6 production amount was larger as compared with those for the branched glucan of Production Example 2, and the production amount of IL-6 was further increased by combining galactose, and glucuronic acid, N-acetylglucosamine or maltose. The branched glucan which has galactose bound to a non-reducing end was recognized to have the activity of stimulating a dendritic cell and, further, the synergistic effect of the dendritic cell stimulating activity was recognized by additionally binding glucuronic acid, N-acetylglucosamine, or mannose in addition to galactose.

[Table 18]

Table 18

| Production condition (Example 21) | Cell stimulating substance (bound saccharide) | IL-6 production amount |
|---|---|---|
| 1 | Glc | 87.3 |
| 5 | Gal | 140.5 |
| 11 | Gal/GlcA | 179.9 |
| 9 | Gal/GlcNAc | 317.1 |
| 13 | Gal/Man | 377.4 |
| | DW | 4.2 |
| | Pam3 200ng/ml | 247.1 |
| | Pam3 10μg/ml | 318.0 |

(Example 25: Macrophage stimulating activity of branched glucans in which N-acetylglucosamine and other various saccharides are bound to non-reducing end)

Using C3H/HeJ mouse (female, 7 week old) intraperitoneal cells, the cells were added to various branched glucans prepared in Example 19 (1 mg/ml cell liquid) to allow them to react for 48 hours . The supernatant of the reaction liquid was collected, and IL-6 produced by cell stimulation was measured by the ELISA method. The results are shown in Table 19 and Fig. 15.

Regarding the branched glucan which has N-acetylglucosamine bound to a non-reducing end, the IL-6 production amount was larger as compared with those for the branched glucan of Production Example 2, and the production amount of IL-6 was further increased by combining N-acetylglucosamine and galactose or mannose. The branched glucan which has an N-acetylglucosamine bound to a non-reducing end was recognized to have the activity of stimulating a macrophage and, further, the synergistic effect of the cell stimulating activity was recognized by additionally binding

galactose or mannose to N-acetylglucosamine.

[Table 19]

Table 19

| Production condition (Example 21) | Cell stimulating substance (bound saccharide) | IL-6 production amount |
|---|---|---|
| 1 | Glc | 154.0 |
| 4 | GlcNAc | 296.0 |
| 7 | GlcNAc/GlcA | 264.0 |
| 10 | GlcNAc/Man | 444.3 |
| 9 | Gal/GlcNAc | 422.7 |
| | DW | 0.0 |
| | Pam3 200ng/ml | 161.3 |

(Example 26: Macrophage stimulating activity of branched glucans in which galactose and other various saccharides are bound to non-reducing end)

Similar to Example 23, the IL-6 production amount with branched glucans, in which bindings of galactose and other saccharides were combined, was investigated. The results are shown in Table 20 and Fig. 16. In the branched glucan which has a galactose bound thereto, the IL-6 production amount was larger as compared with the branched glucan of further increased by combining galactose, and glucuronic acid, N-acetylglucosamine or maltose. The branched glucan which has a galactose bound to a non-reducing end was recognized to have the activity of stimulating a macrophage and, further, the synergistic effect of the cell stimulating activity was recognized by additionally binding glucuronic acid, N-acetylglucosamine, or mannose to galactose.

[Table 20]

Table 20

| Production condition (Example 21) | Cell stimulating substance (binding saccharide) | IL-6 production amount |
|---|---|---|
| 1 | Glc | 154.0 |
| 5 | Gal | 257.7 |
| 11 | Gal/GlcA | 374.8 |
| 9 | Gal/GlcNAc | 422.7 |
| 13 | Gal/Man | 455.5 |
| | DW | 0.0 |
| | Pam3 200ng/ml | 161.3 |

As described above, the present invention has been exemplified using preferable embodiments of the present invention, but the present invention should not be construed so as to be limited to these embodiments. It is understood that the scope of the present invention should be construed only by claims. It is understood that those skilled in the art can carry out an equivalent scope based on the description of the present invention and common technical knowledge, from the description of specific preferable embodiments of the present invention.

Industrial Applicability

It is thought that the glucan in which at least one residue selected from an N-acetylglucosamine residue and a galactose residue is bound via $\alpha$-1, 4 bond to a non-reducing end (non-reducing end-modified glucan) of the present invention, a hydroxyl group-modified product thereof and a reducing end-modified product thereof can be accumulated into a particular tissue when administered into blood. For this reason, they are useful in tissue targeting of a medically effective ingredient. Since the non-reducing end-modified glucan, a hydroxyl group-modified product thereof, a non-reducing end-modified product thereof and a reducing end-modified product thereof of the present invention have the activity of stimulating an antigen-presenting cell such as a dendritic cell or a macrophage to produce IL-6 (antigen-presenting cell stimulating activity), they are useful for stimulating an antigen-presenting cell, and further, useful as a vaccine adjuvant. Since the non-reducing end-modified glucan, a hydroxylgroup-modified product thereof, a non-reducing end-modified product thereof and a reducing end-modified product thereof of the present invention can have an extended blood half life longer than that of an unmodified glucan, and are finally completely degraded in a living body and excreted, they are extremely highly safe and, for this reason, are useful as a modifying material for a medically effective ingredient, a clinical diagnostic, an imaging agent and a nanoparticulate carrier for DDS. The non-reducing end-modified glucan, a hydroxyl group-modified product thereof and a reducing end-modified product thereof of the present invention can be widely utilized in foods, cosmetics, and medicaments.

Sequence Listing free text

SEQ ID NO: 1: Base sequence of the *Aquifex aeolicus* VF5-derived α-glucan phosphorylase.
SEQ ID NO: 2 : Amino acid sequence of the *Aquifex aeolicus* VF5-derived α-glucan phosphorylase.
SEQ ID NO: 3: Base sequence of the potato-derived α-glucan phosphorylase.
SEQ ID NO: 4: Amino acid sequence of the potato-derived α-glucan phosphorylase.
SEQ ID NO: 5: Base sequence encoding the amino acid sequence of the *Thermococcus zilligii* AN1-derived α-glucan phosphorylase.
SEQ ID NO: 6: Amino acid sequence of the *Thermococcus zilligii* AN1-derived α-glucan phosphorylase.

## Claims

1. A branched glucan,
wherein the branched glucan has a plurality of non-reducing ends, and
at least one residue selected from an N-acetylglucosamine residue and a galactose residue is bound via an α-1,4-bond to each of two or more non-reducing ends of the branched α-1,4-glucan,
but neither an N-acetylglucosamine residue nor a galactose residue is present at the position other than the non-reducing ends of the branched α-1,4-glucan,
wherein none of the non-reducing ends of the branched α-1,4-glucan is bound to an uronic acid residue, and
the degree of polymerization of the branched α-1,4-glucan is 15 or more and $4 \times 10^5$ or less.

2. The branched glucan according to claim 1, wherein the branched α-1,4-glucan is selected from the group consisting of a branched maltooligosaccharide, starch, amylopectin, glycogen, dextrin, enzymatically synthesized branched glucan and highly branched cyclic glucan.

3. A hydroxyl group-modified product of the branched glucan according to claim 1, wherein the modification on the hydroxyl group is a modification on some or all of alcoholic hydroxyl groups of the glucan, and the modification on the hydroxyl group is independently selected from the group consisting of hydroxyalkylation, alkylation, acetylation, carboxymethylation, sulfation and phosphorylation.

4. A reducing end-modified product of the branched glucan according to claim 1 or a hydroxyl group-modified product thereof.

5. A non-reducing end-modified product of the branched glucan according to claim 1 or a hydroxyl group-modified product thereof, or a reducing end-modified product thereof, which is further modified by linking a monosaccharide residue other than an N-acetylglucosamine residue and a galactose residue via an α-1,4-bond to at least one non-reducing end of the plurality of non-reducing ends of the branched α-1,4 glucan.

6. The non-reducing end-modified product of the branched glucan or a hydroxyl group-modified product thereof, or a reducing end-modified product thereof according to claim 5, wherein the monosaccharide residue is one kind or two or more kinds selected from a glucosamine residue, a mannose residue, and a xylose residue.

7. The non-reducing end-modified product of the branched glucan or a hydroxyl group-modified product thereof, or a reducing end-modified product thereof according to claim 5, wherein the monosaccharide residue is one kind or two kinds selected from a mannose residue, and a xylose residue.

8. A method for producing a branched glucan in which at least one residue selected from an N-acetylglucosamine residue and a galactose residue is bound to each of two or more non-reducing ends, **characterized by** allowing an α-glucan phosphorylase to act on an aqueous solution comprising a branched α-1,4-glucan having two or more non-reducing ends and N-acetylglucosamine-1-phosphate or galactose-1-phosphate, wherein the degree of polymerization of the branched α-1,4-glucan is 15 or more and $4 \times 10^5$ or less, wherein none of the non-reducing ends of the branched α-1,4-glucan is bound to an uronic acid residue.

9. The method according to claim 8, wherein the α-glucan phosphorylase has 95% or more sequence identity with the amino acid sequence of α-glucan phosphorylase derived from *Aquifex aeolicus* VF5, and has activity of transferring N-acetylglucosamine residue or galactose residue to a non-reducing end of a glucan to form an α-1,4-bond.

10. A medicament comprising:

    the branched glucan according to claim 1, a hydroxyl group-modified product thereof, or a reducing end-modified product thereof, and
    a medically effective ingredient.

11. A medicament comprising:

a non-reducing end-modified product of the branched glucan according to claim 1 or a hydroxyl group-modified product thereof, or a reducing end-modified product thereof, which is further modified by linking a monosaccharide residue other than an N-acetylglucosamine residue and a galactose residue via an α-1,4-bond to at least one non-reducing end of the plurality of non-reducing ends of the branched α-1,4 glucan, wherein the monosaccharide residue is one kind or two kinds selected from a glucosamine residue, a mannose residue, and a xylose residue; and
a medically effective ingredient.

**12.** The medicament according to claim 10 or 11, wherein the medically effective ingredient is selected from the group consisting of a low-molecular weight organic compound, a protein, a peptide, an antibody, an antibody fragment, a receptor, a receptor fragment, a DNA, an RNA, a siRNA, an miRNA and an RNA aptamer.

**13.** The medicament according to claim 10 or 11, wherein the medically effective ingredient is an antigen protein or a peptide.

**14.** A composition for clinical diagnosis comprising the branched glucan according to claim 1, a hydroxyl group-modified product thereof, or a reducing end-modified product thereof.

**15.** A composition for clinical diagnosis comprising a non-reducing end-modified product of the branched glucan according to claim 1 or a hydroxyl group-modified product thereof, or a reducing end-modified product thereof, which is further modified by linking a monosaccharide residue other than an N-acetylglucosamine residue and a galactose residue via an α-1,4-bond to at least one non-reducing end of the plurality of non-reducing ends of the branched α-1,4 glucan, wherein the monosaccharide residue is one kind or two kinds selected from a glucosamine residue, a mannose residue, and a xylose residue.

## Patentansprüche

**1.** Verzweigtes Glucan,
wobei das verzweigte Glucan eine Mehrzahl von nicht reduzierenden Enden besitzt, und
zumindest ein Rest ausgewählt aus einem N-Acetylglucosaminrest und einem Galactoserest über eine α-1,4-Bindung an jedes von zwei oder mehreren nicht reduzierenden Enden des verzweigten α-1,4-Glucans gebunden ist, jedoch weder ein N-Acetylglucosaminrest noch ein Galactoserest an einer von den nicht reduzierenden Enden des α-1,4-Glucans verschiedenen Position vorhanden ist,
wobei keines der nicht reduzierenden Enden des verzweigten α-1,4-Glucans an einen Uronsäurerest gebunden ist, und
der Polymerisationsgrad des verzweigten α-1,4-Glucans 15 oder mehr und 4 x $10^5$ oder weniger beträgt.

**2.** Verzweigtes Glucan nach Anspruch 1, wobei das verzweigte α-1,4-Glucan ausgewählt ist aus der Gruppe bestehend aus einem verzweigtem Maltooligosaccharid, Stärke, Amylopectin, Glycogen, Dextrin, enzymatisch synthetisiertem verzweigten Glucan und hochverzweigtem cyclischen Glucan.

**3.** An einer Hydroxylgruppe modifiziertes Produkt des verzweigten Glucans nach Anspruch 1, wobei die Modifizierung an der Hydroxylgruppe eine Modifizierung an einigen oder allen alkoholischen Hydroxylgruppen des Glucans ist und die Modifizierung an der Hydroxylgruppe unabhängig ausgewählt ist aus der Gruppe bestehend aus Hydroxyalkylierung, Alkylierung, Acetylierung, Carboxymethylierung, Sulfatierung und Phosphorylierung.

**4.** An einem reduzierenden Ende modifiziertes Produkt des verzweigten Glucans nach Anspruch 1 oder an einer Hydroxylgruppe modifiziertes Produkt davon.

**5.** An einem nicht reduzierenden Ende modifiziertes Produkt des verzweigten Glucans nach Anspruch 1 oder an einer Hydroxylgruppe modifiziertes Produkt davon oder an einem reduzierendem Ende modifiziertes Produkt davon, welches ferner durch Vernetzen eines von einem N-Acetylglucosaminrest und einem Galactoserest verschiedenen Monosaccharidrests über eine α-1,4-Bindung an mindestens ein nicht reduzierendes Ende der Mehrzahl von nicht reduzierenden Enden des verzweigten α-1,4-Glucan, modifiziert worden ist.

**6.** An einem nicht reduzierenden Ende modifiziertes Produkt des verzweigten Glucans oder an einer Hydroxylgruppe modifiziertes Produkt davon oder an einem reduzierenden Ende modifiziertes Produkt davon nach Anspruch 5,

wobei der Monosaccharidrest eine Art oder zwei oder mehrere Arten ausgewählt aus einem Glucosaminrest, einem Mannoserest und einem Xyloserest ist.

7. An einem nicht reduzierenden Ende modifiziertes Produkt des verzweigten Glucans oder an einer Hydroxylgruppe modifiziertes Produkt davon oder an einem reduzierenden Ende modifiziertes Produkt davon nach Anspruch 5, wobei der Monosaccharidrest eine Art oder zwei Arten ausgewählt aus einem Mannoserest und einem Xyloserest ist.

8. Verfahren zur Herstellung eines verzweigten Glucans, bei dem zumindest ein Rest ausgewählt aus einem N-Acetylglucosaminrest und einem Galactoserest an jedes von zwei oder mehreren nicht reduzierenden Enden gebunden ist, **dadurch gekennzeichnet, dass** man eine α-Glucanphosphorylase auf eine wässrige Lösung, die ein verzweigtes α-1,4-Glucan mit zwei oder mehreren nicht reduzierende Enden und N-Acetylglucosamin-1-phosphat oder Galactose-1-phosphat umfasst, einwirken lässt, wobei der Polymerisationsgrad des verzweigten α-1,4-Glucans 15 oder mehr und $4 \times 10^5$ oder weniger beträgt, wobei keines der nicht reduzierenden Enden des verzweigten α-1,4-Glucans an einen Uronsäurerest gebunden wird.

9. Verfahren nach Anspruch 8, bei dem die α-Glucan-phosphorylase 95% oder mehr Sequenzidentität mit der Aminosäuresequenz der von *Aquifex aeolicus* VF5 abgeleiteten α-Glucanphosphorylase aufweist, und die eine Aktivität zur Übertragung des N-Acetylglucosaminrests oder des Galactoserests auf ein nicht reduzierendes Ende eines Glucans aufweist, wobei eine α-1,4-Bindung gebildet wird.

10. Medikament, umfassend:

    das verzweigte Glucan nach Anspruch 1, ein an einer Hydroxylgruppe modifiziertes Produkt davon oder ein an einem reduzierenden Ende modifiziertes Produkt davon, und
    einen medizinisch wirksamen Bestandteil.

11. Medikament, umfassend:

    ein an einem nicht reduzierenden Ende modifiziertes Produkt des verzweigten Glucans nach Anspruch 1 oder ein an einer Hydroxylgruppe modifiziertes Produkt davon oder ein an einem reduzierenden Ende modifiziertes Produkt davon, das ferner durch Vernetzen eines von einem N-Acetylglucosaminrest und einem Galactoserest verschiedenen Monosaccharidrests über eine α-1,4-Bindung an mindestens ein nicht reduzierendes Ende der Mehrzahl von nicht reduzierenden Enden des verzweigten α-1,4-Glucans verändert worden ist, wobei der Monosaccharidrest eine Art oder zwei Arten ausgewählt aus einem Glucosaminrest, einem Mannoserest und einem Xyloserest ist; und
    einen medizinisch wirksamen Bestandteil.

12. Medikament nach Anspruch 10 oder 11, wobei der medizinisch wirksame Bestandteil ausgewählt ist aus der Gruppe bestehend aus einer niedermolekularen organischen Verbindung, einem Protein, einem Peptid, einem Antikörper, einem Antikörperfragment, einem Rezeptor, einem Rezeptorfragment, einer DNA, einer RNA, einer siRna, einer miRNA und einem RNA-Aptamer.

13. Medikament nach Anspruch 10 oder 11, wobei der medizinisch wirksame Bestandteil ein Antigenprotein oder -peptid ist.

14. Zusammensetzung für die klinische Diagnose, umfassend das verzweigte Glucan nach Anspruch 1, ein an einer Hydroxylgruppe modifiziertes Produkt davon oder ein an einem reduzierenden Ende modifiziertes Produkt davon.

15. Zusammensetzung für die klinische Diagnose, umfassend ein an einem nicht reduzierendem Ende modifiziertes Produkt des verzweigten Glucans nach Anspruch 1 oder ein an einer Hydroxylgruppe modifiziertes Produkt davon oder ein an einem reduzierendem Ende modifiziertes Produkt davon, welches ferner durch Vernetzen eines von einem N-Acetylglucosaminrest und einem Galactoserest verschiedenen Monosaccharidrests über eine α-1,4-Bindung an mindestens ein nicht reduzierendes Ende der Mehrzahl von nicht reduzierenden Enden des verzweigten α-1,4-Glucans verändert worden ist, wobei der Monosaccharidrest eine Art oder zwei Arten ausgewählt aus einem Glucosaminrest, einem Mannoserest und einem Xyloserest ist.

## Revendications

1. Glucane ramifié,

- lequel glucane ramifié comporte plusieurs extrémités non-réductrices,
- dans lequel au moins un résidu choisi parmi un résidu de N-acétyl-glucosamine et un résidu de galactose est lié par l'intermédiaire d'une liaison α-1,4 à chacune de deux extrémités non-réductrices ou plus du α-1,4-glucane ramifié,
- mais il n'y a ni résidu de N-acétyl-glucosamine ni résidu de galactose présent en une position autre que les extrémités non-réductrices du α-1,4-glucane ramifié,
- dans lequel aucune des extrémités non-réductrices du α-1,4-glucane ramifié n'est liée à un résidu d'acide uronique,
- et dans lequel le degré de polymérisation du α-1,4-glucane ramifié vaut 15 ou plus, mais $4.10^5$ ou moins.

2. Glucane ramifié conforme à la revendication 1, lequel α-1,4-glucane ramifié est choisi dans l'ensemble constitué par les suivants : malto-oligosaccharide ramifié, amidon, amylopectine, glycogène, dextrine, glucane ramifié synthétisé par voie enzymatique, et glucane cyclique hautement ramifié.

3. Produit de modification de groupes hydroxyle d'un glucane ramifié conforme à la revendication 1, dans lequel la modification de groupes hydroxyle est une modification de certains groupes hydroxyle alcooliques du glucane ou de tous ces groupes, et cette modification de groupes hydroxyle est choisie indépendamment dans l'ensemble formé par une hydroxy-alkylation, une alkylation, une acétylation, une carboxy-méthylation, une sulfurylation et une phosphorylation.

4. Produit de modification d'extrémités réductrices d'un glucane ramifié conforme à la revendication 1, ou produit de modification de groupes hydroxyle de celui-ci.

5. Produit de modification d'extrémités non-réductrices d'un glucane ramifié conforme à la revendication 1, ou produit de modification de groupes hydroxyle de celui-ci, ou produit de modification d'extrémités réductrices de celui-ci, qui est encore modifié par liaison d'un résidu monosaccharide autre qu'un résidu de N-acétyl-glucosamine ou un résidu de galactose, par l'intermédiaire d'une liaison α-1,4, à au moins l'une des extrémités non-réductrices de l'α-1,4-glucane ramifié.

6. Produit de modification d'extrémités non-réductrices d'un glucane ramifié, ou produit de modification de groupes hydroxyle de celui-ci, ou produit de modification d'extrémités réductrices de celui-ci, conforme à la revendication 5, dans lequel le résidu monosaccharide est d'un ou de deux type(s) ou plus, et choisi parmi un résidu de glucosamine, un résidu de mannose et un résidu de xylose.

7. Produit de modification d'extrémités non-réductrices d'un glucane ramifié, ou produit de modification de groupes hydroxyle de celui-ci, ou produit de modification d'extrémités réductrices de celui-ci, conforme à la revendication 5, dans lequel le résidu monosaccharide est d'un ou de deux type(s), et choisi parmi un résidu de mannose et un résidu de xylose.

8. Procédé de production d'un glucane ramifié dans lequel au moins un résidu choisi parmi un résidu de N-acétyl-glucosamine et un résidu de galactose est lié à chacune de deux extrémités non-réductrices ou plus, **caractérisé en ce qu'**on fait agir une α-glucane phosphorylase sur une solution aqueuse comprenant un α-1,4-glucane ramifié comportant deux extrémités non-réductrices ou plus et un résidu N-acétyl-glucosamine-1-phosphate ou galactose-1-phosphate, étant entendu que le degré de polymérisation du α-1,4-glucane ramifié vaut 15 ou plus, mais $4.10^5$ ou moins, et qu'aucune des extrémités non-réductrices du α-1,4-glucane ramifié n'est liée à un résidu d'acide uronique.

9. Procédé conforme à la revendication 8, dans lequel l'α-glucane phosphorylase présente une séquence identique, à un degré de 95 % ou plus, à la séquence d'acides aminés de l'α-glucane phosphorylase dérivée d'*Aquifex aeolicus* VF5, et a pour activité de transférer un résidu de N-acétyl-glucosamine ou un résidu de galactose sur une extrémité non-réductrice d'un glucane pour former une liaison α-1,4.

10. Médicament comprenant

- un glucane ramifié conforme à la revendication 1, un produit de modification de groupes hydroxyle d'un tel glucane ou un produit de modification d'extrémités non-réductrices d'un tel glucane,
- et un ingrédient à effet médical.

**11.** Médicament comprenant

- un produit de modification d'extrémités non-réductrices d'un glucane ramifié, conforme à la revendication 1, ou un produit de modification de groupes hydroxyle de celui-ci, ou un produit de modification d'extrémités réductrices de celui-ci, qui est encore modifié par liaison d'un résidu monosaccharide autre qu'un résidu de N-acétyl-glucosamine ou un résidu de galactose, par l'intermédiaire d'une liaison $\alpha$-1,4, à au moins l'une des extrémités non-réductrices de l'$\alpha$-1,4-glucane ramifié, étant entendu que ce résidu monosaccharide est d'un ou de deux type(s), et choisi parmi un résidu de glucosamine, un résidu de mannose et un résidu de xylose,
- et un ingrédient à effet médical.

**12.** Médicament conforme à la revendication 10 ou 11, dans lequel l'ingrédient à effet médical est choisi dans l'ensemble constitué par un composé organique à faible poids moléculaire, une protéine, un peptide, un anticorps, un fragment d'anticorps, un récepteur, un fragment de récepteur, un ADN, un ARN, un petit ARN interférent, un micro-ARN et un ARN aptamère.

**13.** Médicament conforme à la revendication 10 ou 11, dans lequel l'ingrédient à effet médical est une protéine antigénique ou un peptide.

**14.** Composition pour diagnostic clinique, comprenant un glucane ramifié conforme à la revendication 1, ou un produit de modification de groupes hydroxyle de celui-ci, ou un produit de modification d'extrémités réductrices de celui-ci.

**15.** Composition pour diagnostic clinique, comprenant un produit de modification d'extrémités non-réductrices d'un glucane ramifié conforme à la revendication 1, ou un produit de modification de groupes hydroxyle de celui-ci, ou un produit de modification d'extrémités réductrices de celui-ci, qui est encore modifié par liaison d'un résidu monosaccharide autre qu'un résidu de N-acétyl-glucosamine ou un résidu de galactose, par l'intermédiaire d'une liaison $\alpha$-1,4, à au moins l'une des extrémités non-réductrices de l'$\alpha$-1,4-glucane ramifié, étant entendu que ce résidu monosaccharide est d'un ou de deux type(s), et choisi parmi un résidu de glucosamine, un résidu de mannose et un résidu de xylose.

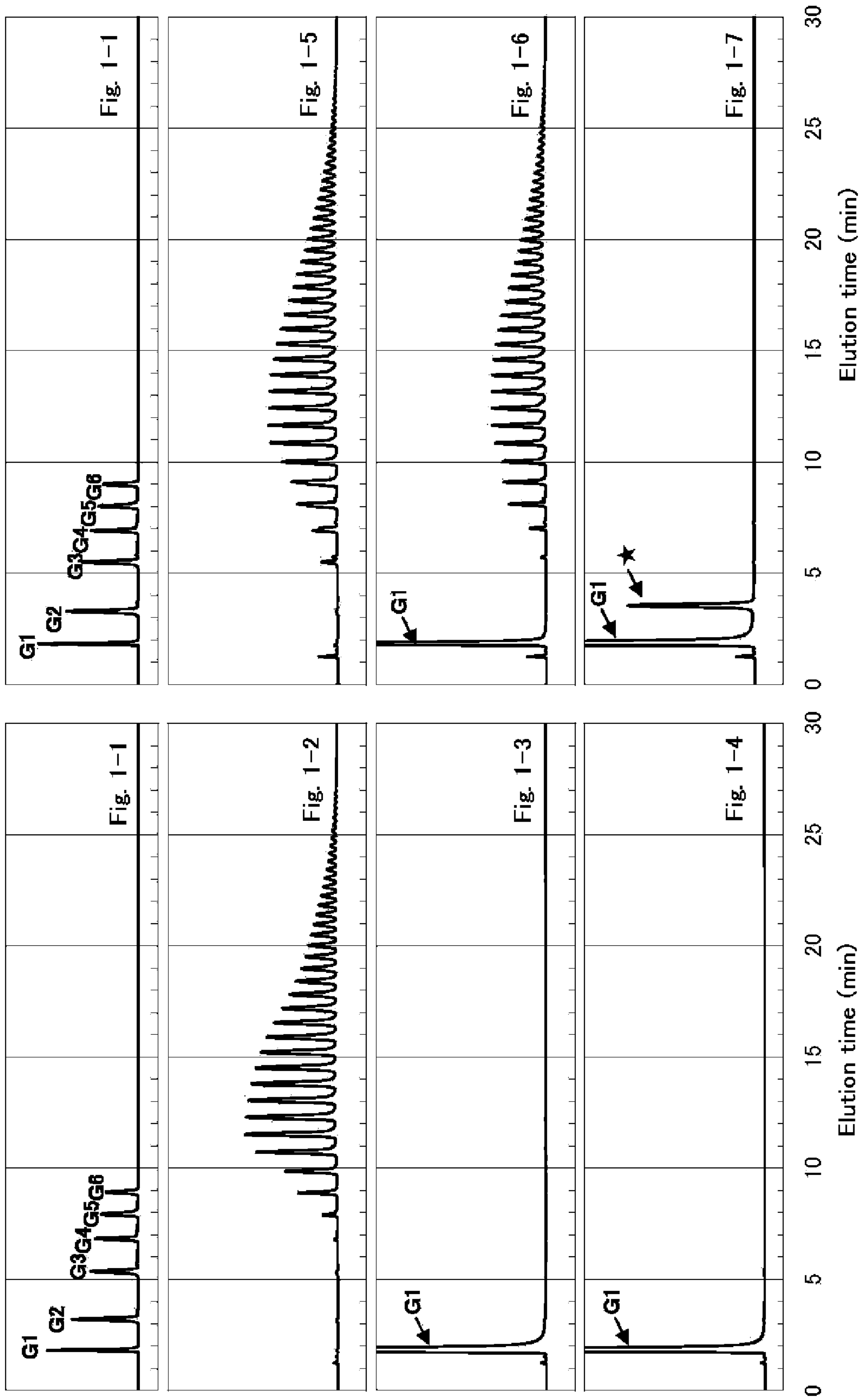
Fig. 1-1
Fig. 1-5
Fig. 1-6
Fig. 1-7
Fig. 1-1
Fig. 1-2
Fig. 1-3
Fig. 1-4
G1
G2
G3
G4
G5
G6
★
Elution time (min)
0
5
10
15
20
25
30

Fig.1

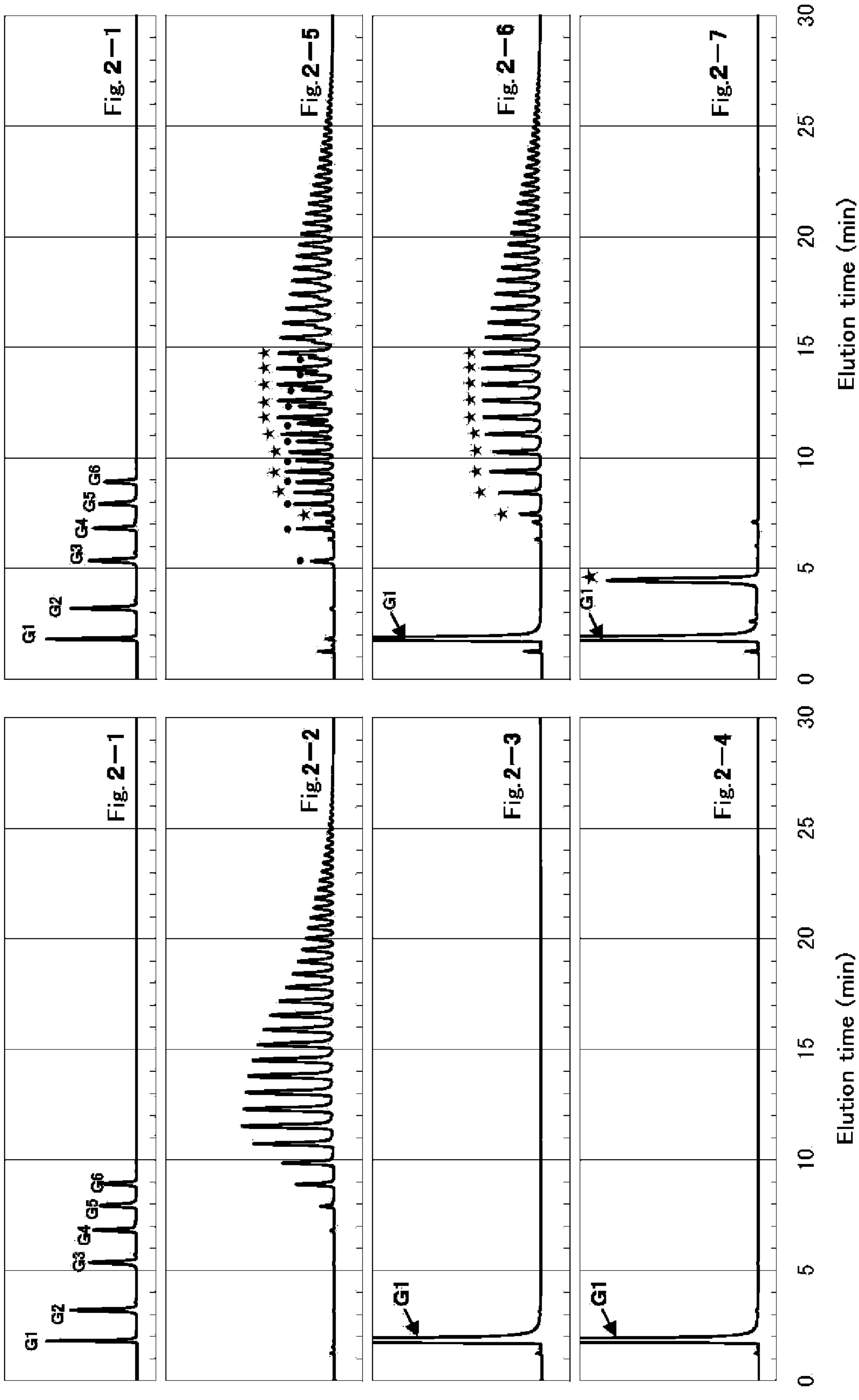
Fig. 2—1
G1
G2
G3
G4
G5
G6
Fig.2—2
Fig.2—3
Fig.2—4
Fig.2—5
Fig.2—6
Fig.2—7
0
5
10
15
20
25
30
Elution time (min)

*Fig.2*

Fig. 3-1

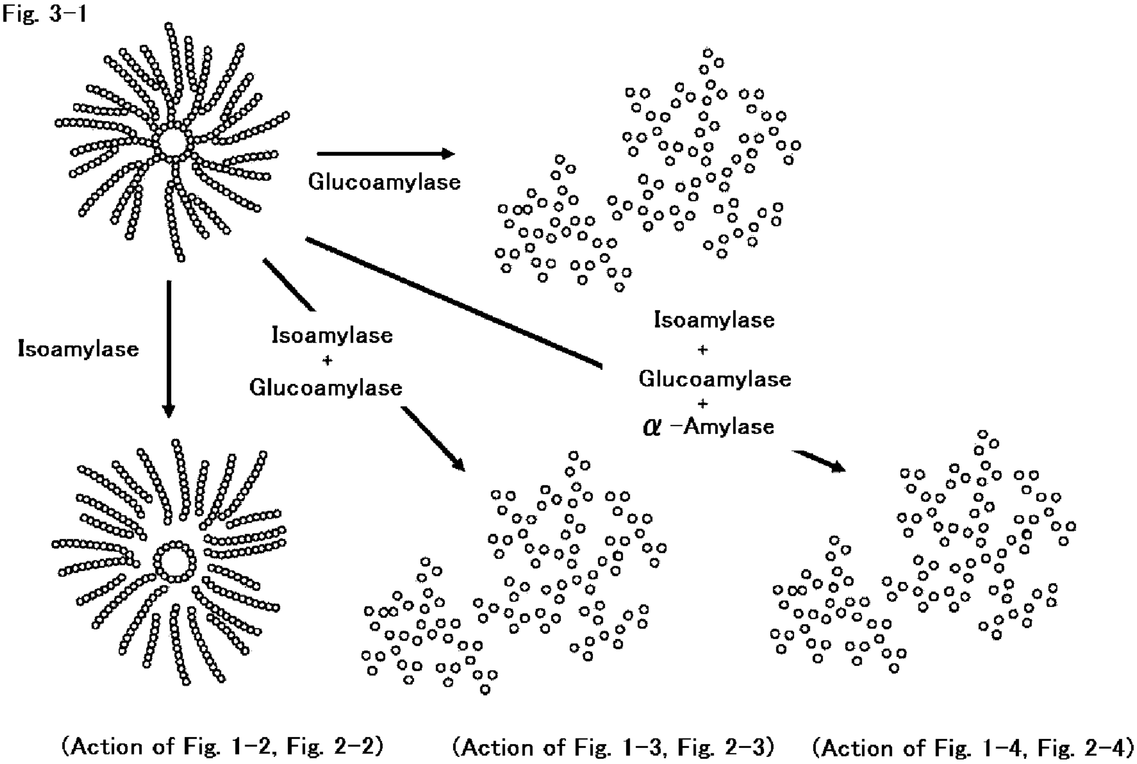


Fig. 3-2

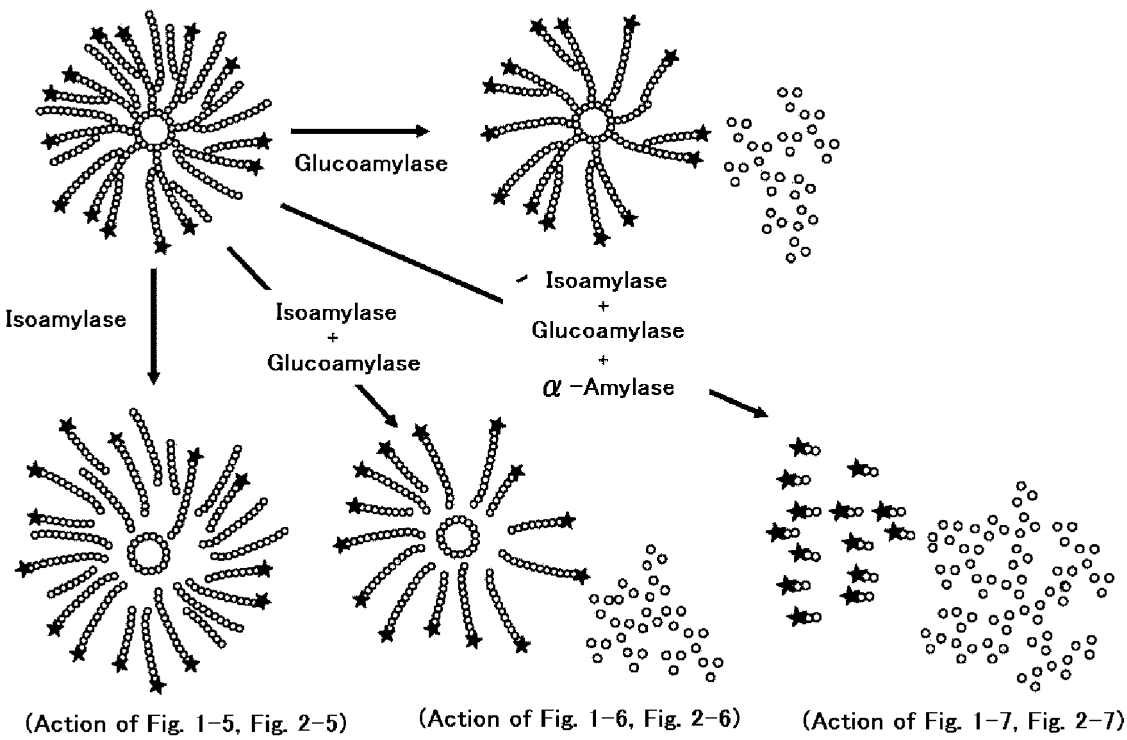


*Fig.3*

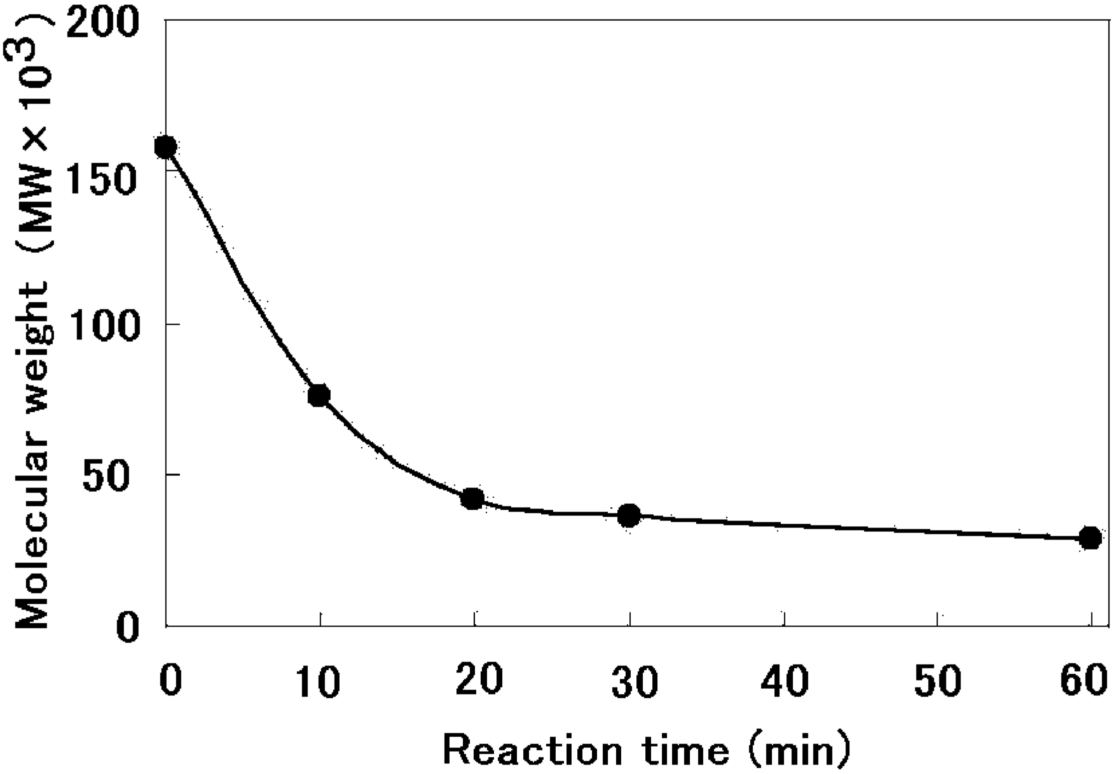
Molecular weight (MW × 10^3)
200
150
100
50
0
0
10
20
30
40
50
60
Reaction time (min)

*Fig. 4*

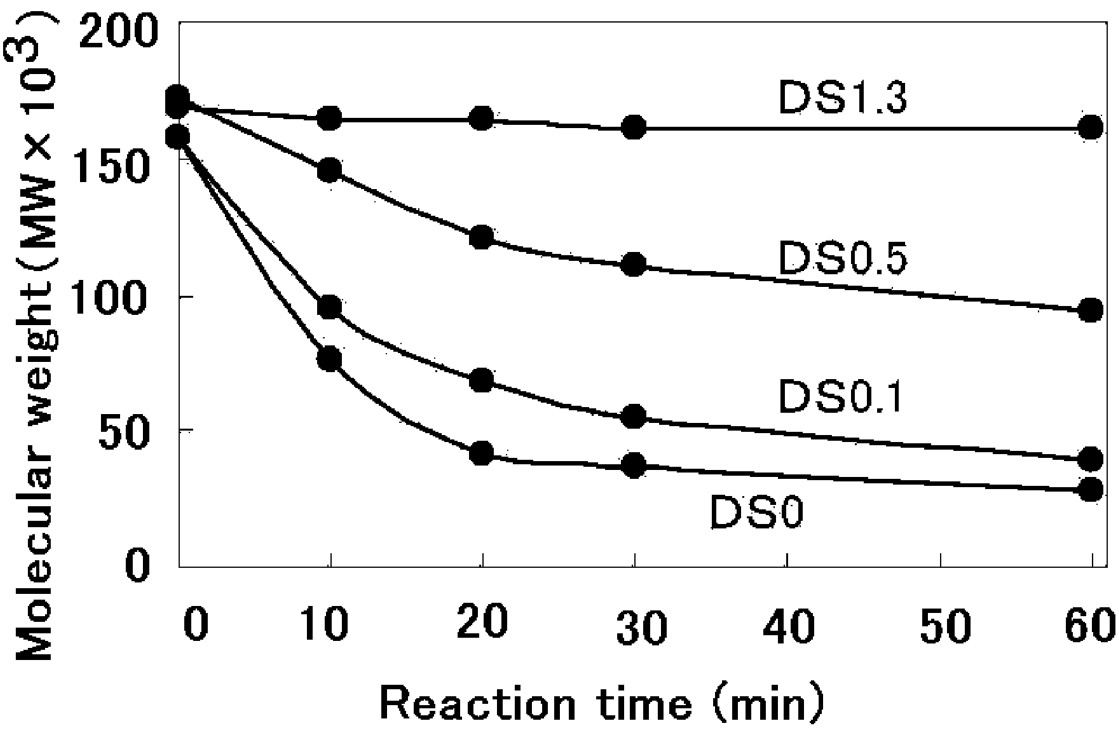
Molecular weight (MW × 10^3)
200
150
100
50
0
DS1.3
DS0.5
DS0.1
DS0
0
10
20
30
40
50
60
Reaction time (min)

*Fig. 5*

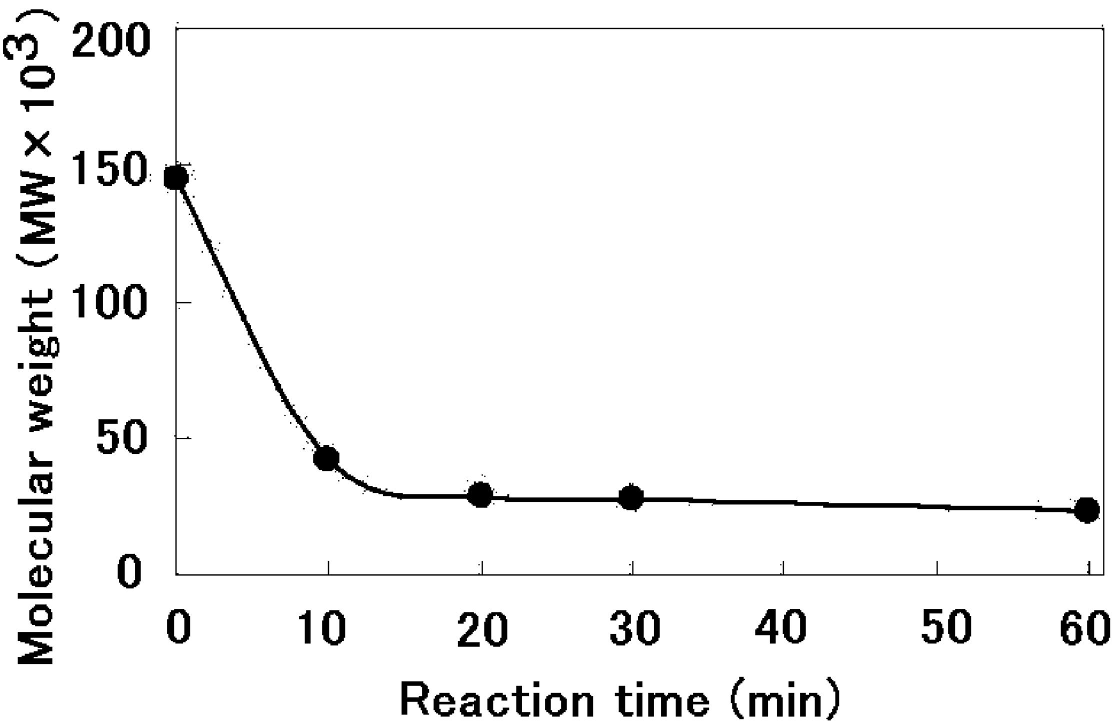
Molecular weight (MW×10³)
200
150
100
50
0
0
10
20
30
40
50
60
Reaction time (min)

*Fig. 6*

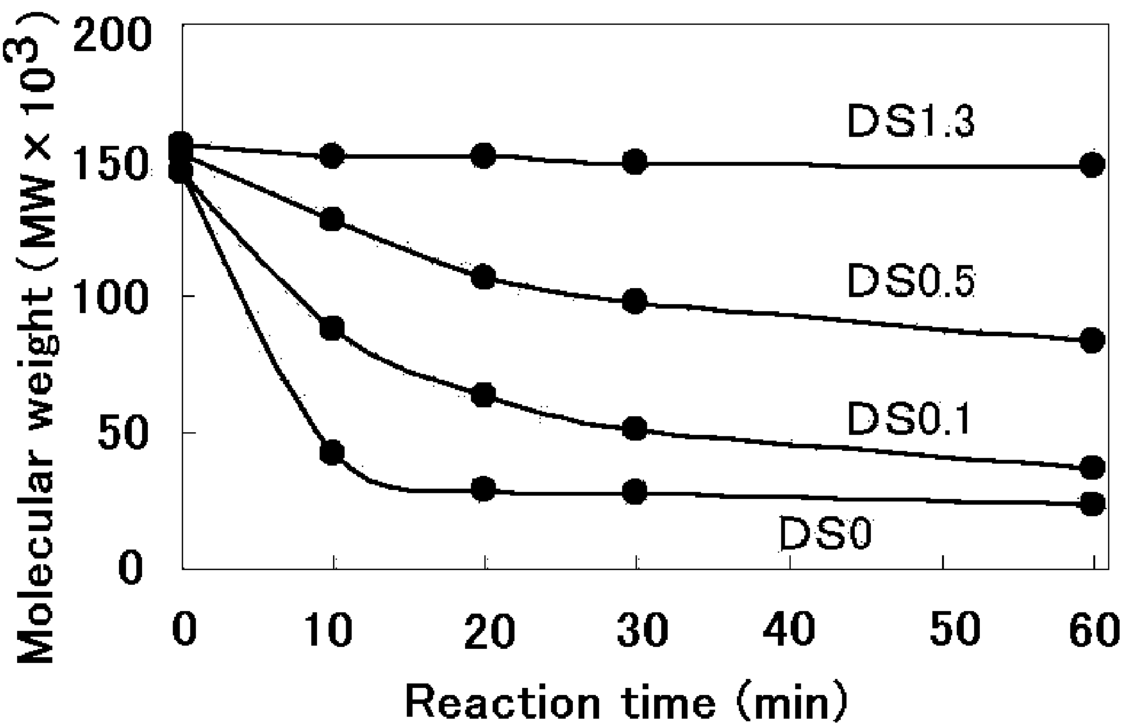
Molecular weight (MW×10³)
200
150
100
50
0
DS1.3
DS0.5
DS0.1
DS0
0
10
20
30
40
50
60
Reaction time (min)

*Fig. 7*

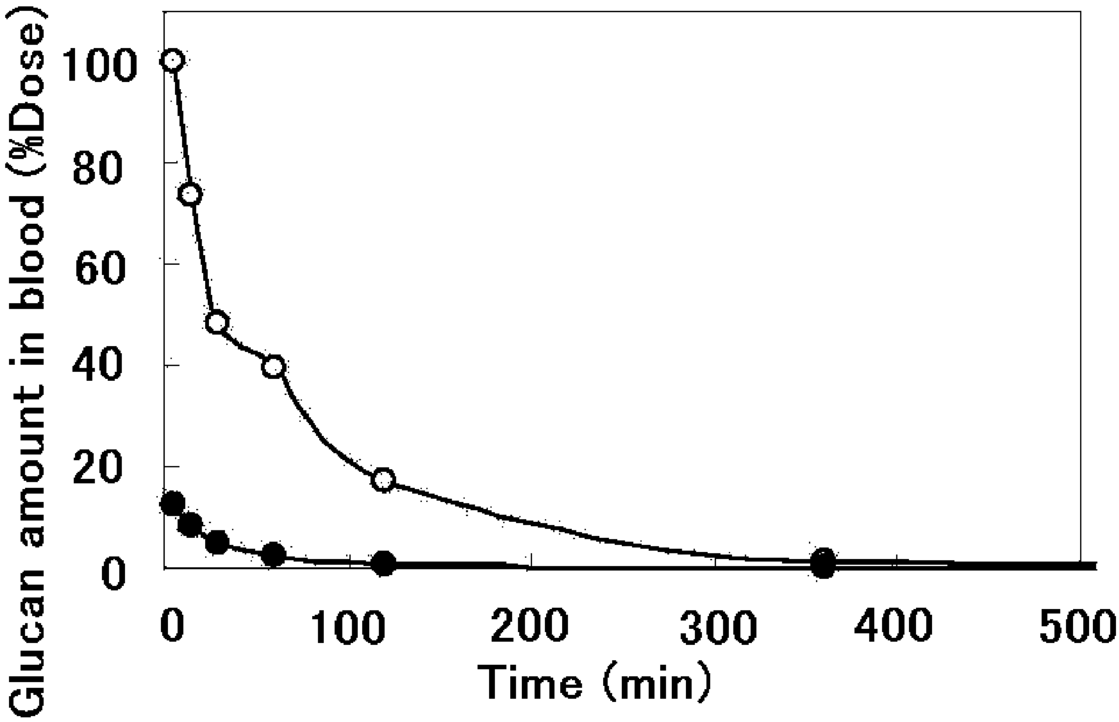
Glucan amount in blood (%Dose)
100
80
60
40
20
0
0
100
200
300
400
500
Time (min)

*Fig. 8*

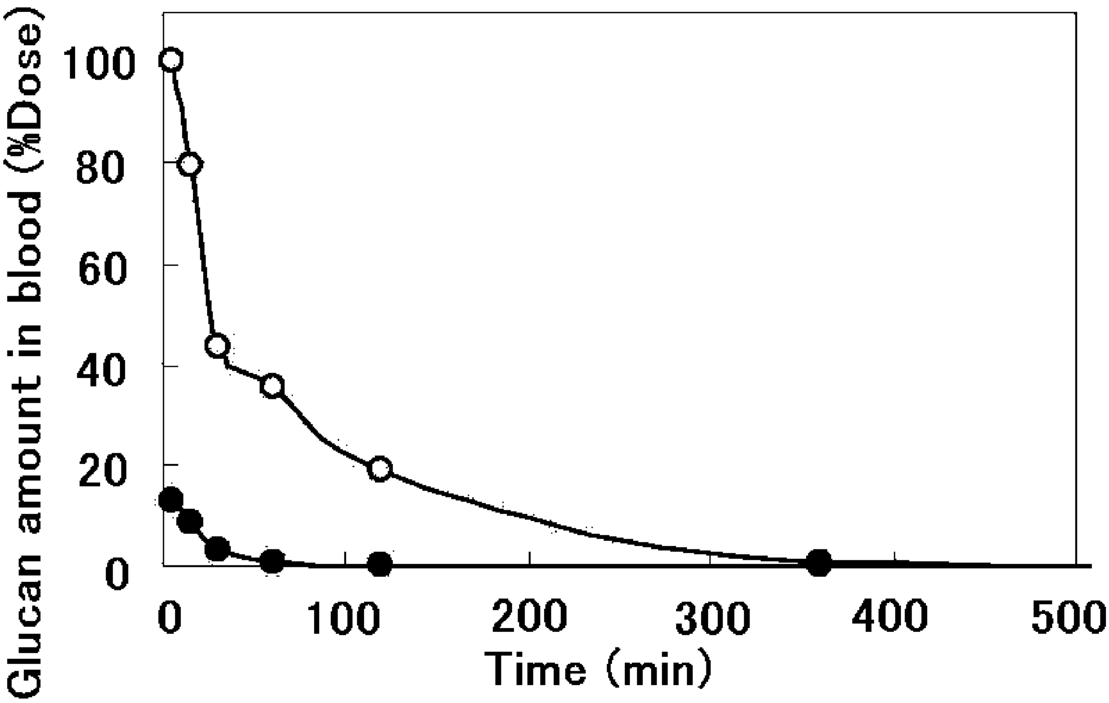
Glucan amount in blood (%Dose)
100
80
60
40
20
0
0
100
200
300
400
500
Time (min)

*Fig. 9*

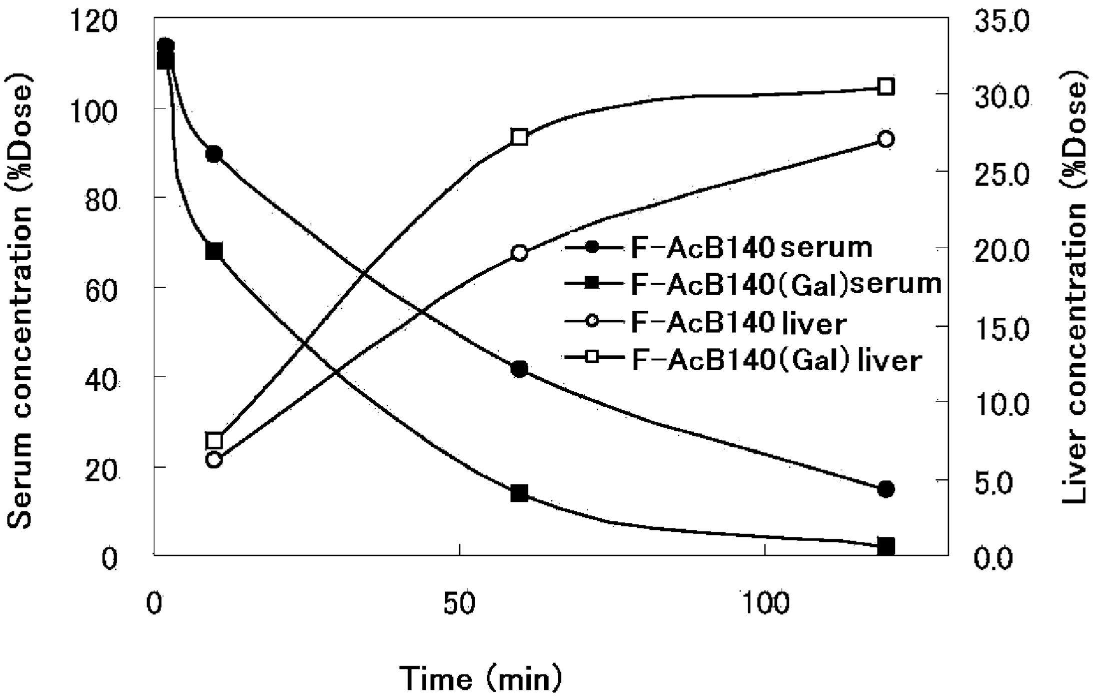
120
100
80
60
40
20
0
Serum concentration (%Dose)
35.0
30.0
25.0
20.0
15.0
10.0
5.0
0.0
Liver concentration (%Dose)
0
50
100
Time (min)
F-AcB140 serum
F-AcB140(Gal)serum
F-AcB140 liver
F-AcB140(Gal) liver

*Fig.10*

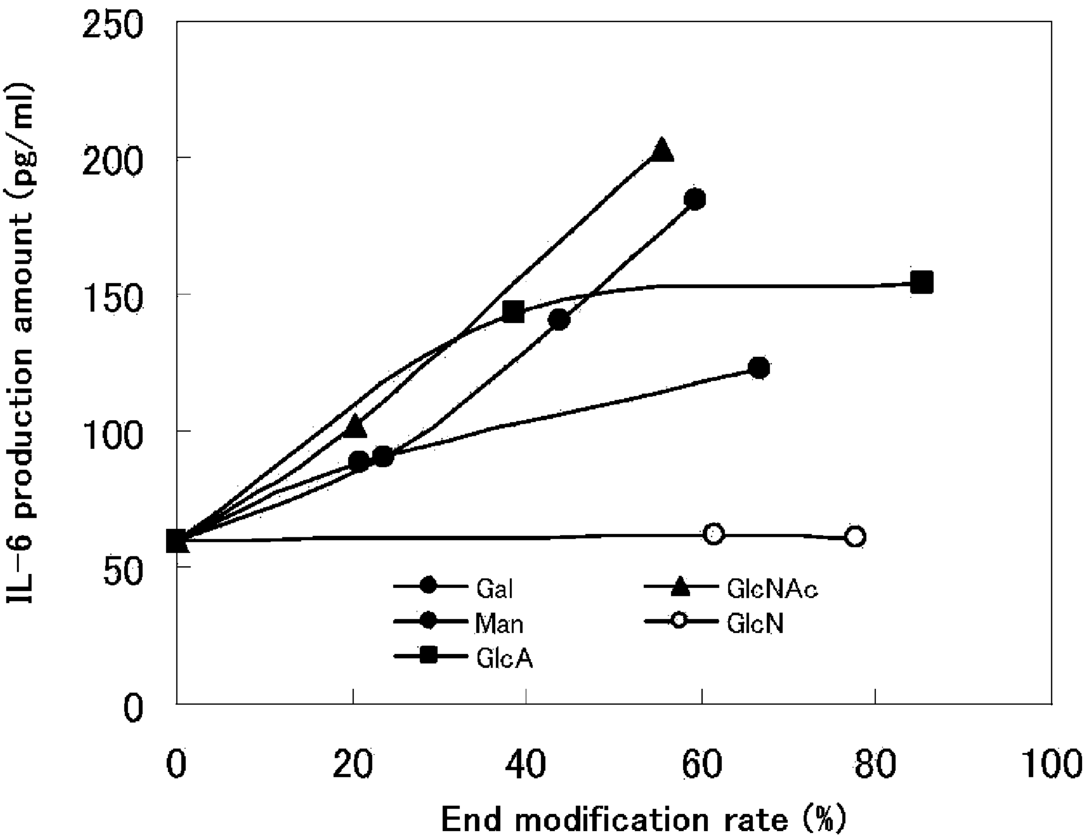
250
200
150
100
50
0
IL-6 production amount (pg/ml)
0
20
40
60
80
100
End modification rate (%)
Gal
Man
GlcA
GlcNAc
GlcN

*Fig.11*

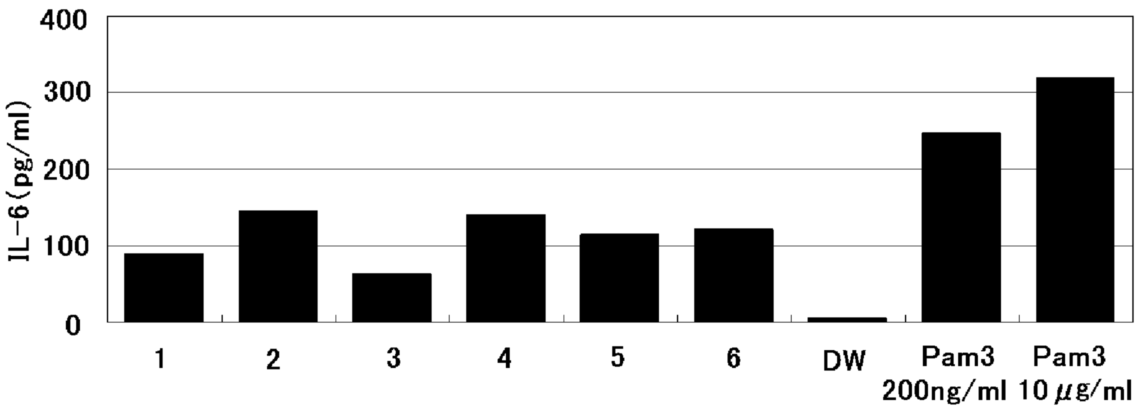
400
300
200
100
0
IL-6(pg/ml)
1
2
3
4
5
6
DW
Pam3 200ng/ml
Pam3 10 µg/ml

*Fig.12*

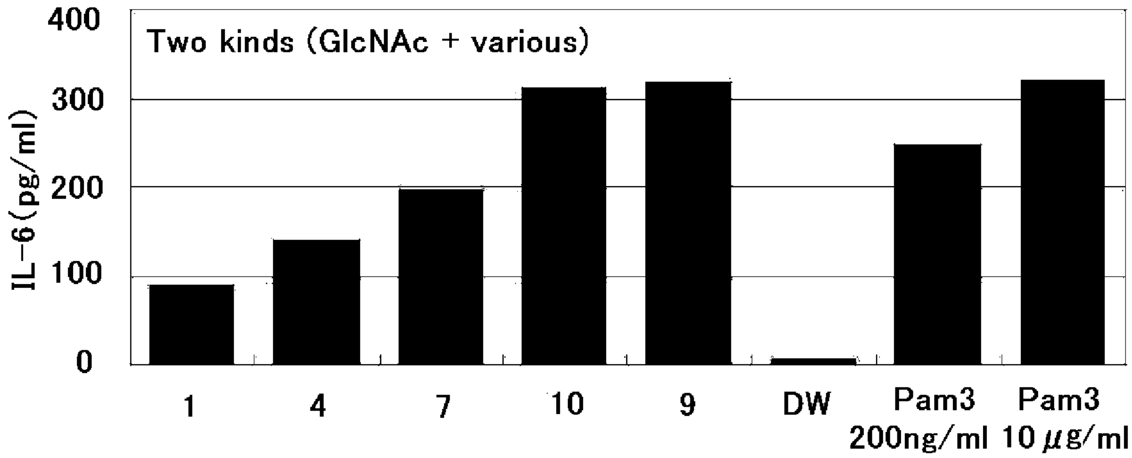
Two kinds (GlcNAc + various)
400
300
200
100
0
IL-6(pg/ml)
1
4
7
10
9
DW
Pam3 200ng/ml
Pam3 10 µg/ml

*Fig.13*

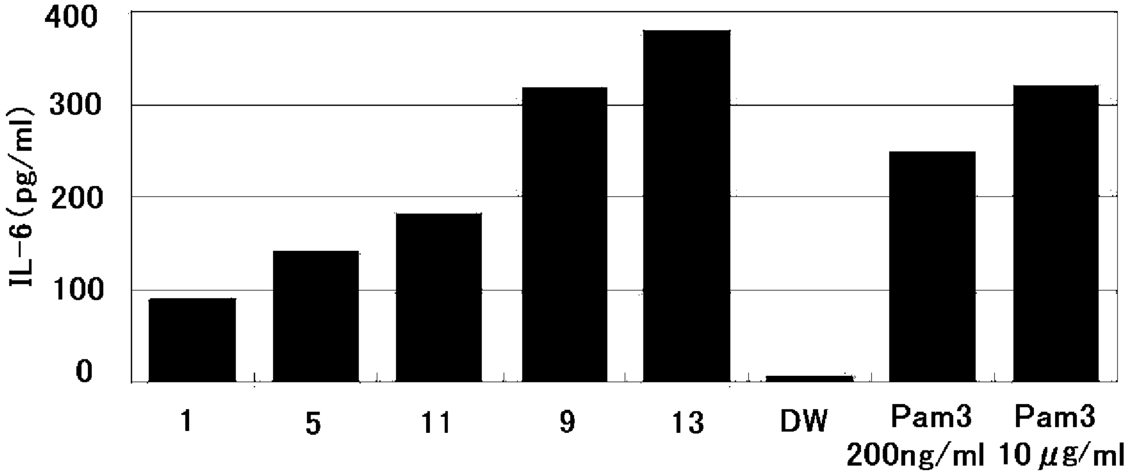
IL-6(pg/ml)
400
300
200
100
0
1
5
11
9
13
DW
Pam3
200ng/ml
Pam3
10 μg/ml

*Fig.14*

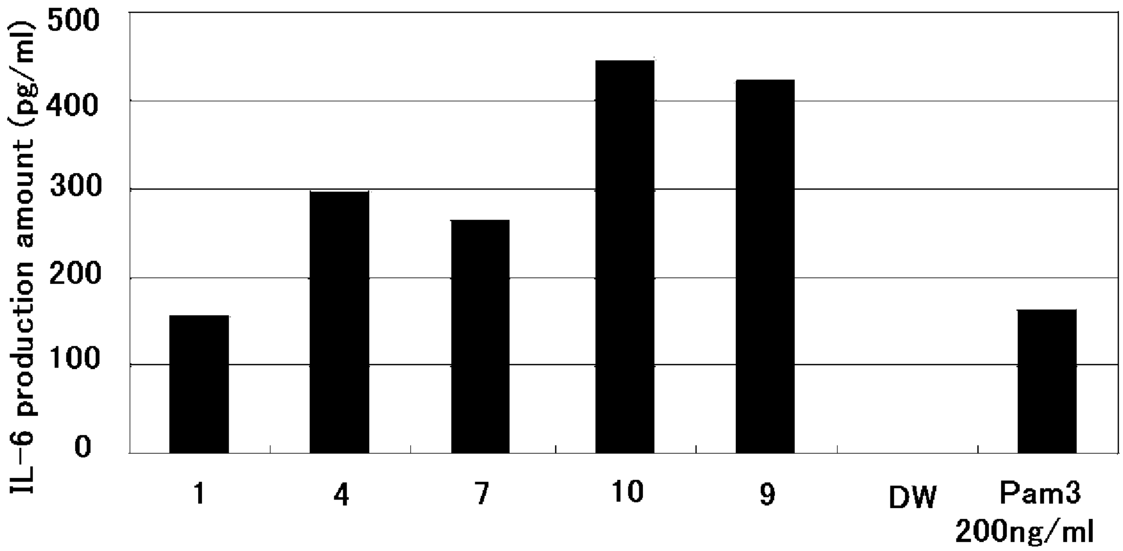
IL-6 production amount (pg/ml)
500
400
300
200
100
0
1
4
7
10
9
DW
Pam3
200ng/ml

*Fig.15*

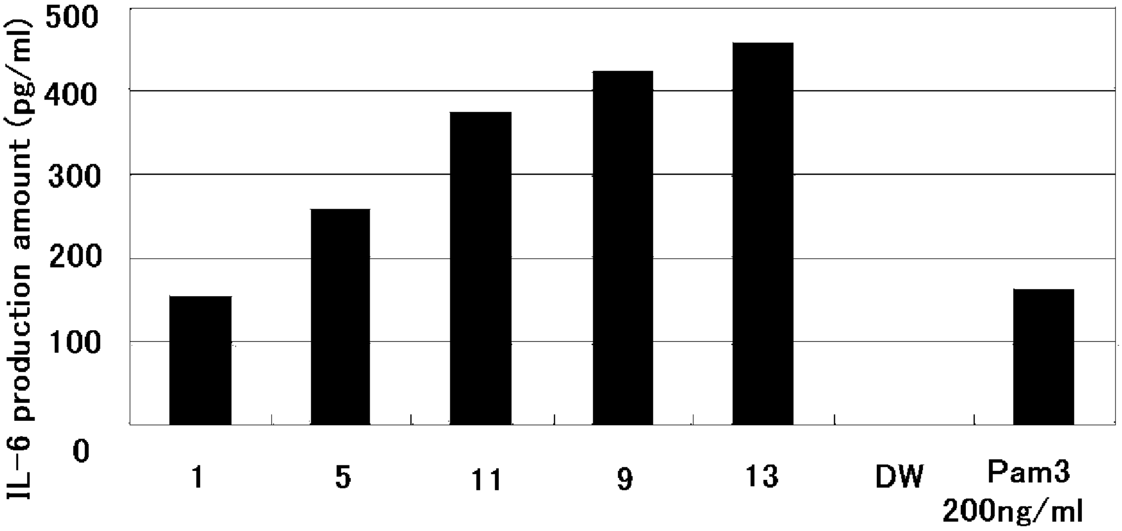
IL-6 production amount (pg/ml)
500
400
300
200
100
0
1
5
11
9
13
DW
Pam3
200ng/ml

*Fig.16*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02097107 A
- JP 8134104 A
- JP 3107358 B
- JP 2007097517 A
- JP 5276883 A
- JP 7241181 A
- WO 01073106 A
- JP 2008121693 A
- JP 2000316581 A
- JP 2004526463 W
- JP 2010249087 A


### Non-patent literature cited in the description


- Janeway's Immunobiology. Takehiko Sasazuki, Nankodo Co., Ltd,
- Janeway's Immunobiology. Garlnd Science. Taylar & Francis Group, 2008
- **GEIGER, J. D. et al.** *Cancer Res.,* 2001, vol. 61, 8513-8519
- General Statement: Kinousei DDS carrier wo mochiita seizai sekkei ni yoru soyaku (Drug Discovery by Preparation Design Using Functional DDS Carrier). **HIROAKI OKADA.** Drug delivery using functional DDS carriers. CMC Publishing Co., Ltd, 2008, 1-23
- **MASAYUKI YOKOYAMA.** Polymeric materials for drug carriers, Special Topic, DDS ni riyou sareru kobunshi kagaku (Polymer Chemistry Utilized in DDS). *Drug Delivery System,* 2008, vol. 23-6, 610-617
- **MARIA LAURA IMMORDINO et al.** *International Journal of Nanomedicine,* 2006, vol. 1 (3), 297-315
- **J. MILTON HARRIS ; ROBERT B. CHESS.** NATURE REVIEWS. *DRUG DISCOVERY,* March 2003, vol. 2, 214-221
- **NAWAJ et al.** *Carbohydr. Res.,* 2008, vol. 343, 2692-2696
- **MANU R. M. DE GROEVE et al.** *Biotechnol. Lett.,* 2009, vol. 31, 1873-1877
- **TAKEISHI, K. ; GOTOH, O.** Sequence Relationships among Various 4.5 S RNA Species. *J. Biochem.,* 1984, vol. 92, 1173-1177
- **T. J. SCHOCH et al.** *J. American Chemical Society,* 1942, vol. 64, 2957
- **HIZUKURI et al.** *Starch,* 1983, vol. 35, 348-350
- **NAKANO et al.** *Journal of Biochemistry,* 1989, vol. 106, 691-695